# EUROPÄISCHE PATENTSCHRIFT

(11) **EP 1 484 615 B1**
(45) Veröffentlichungstag und Bekanntmachung des Hinweises auf die Patenterteilung: **16.04.2008**
(21) Anmeldenummer: 04010893.8
(22) Anmeldetag: 02.06.2004
(51) Int. Cl.: G01N 33/86, C12Q 1/56

(54) **Nachweisverfahren für Fibrinogen und/oder Fibrinogen-Derivate**
Method for detecting fibrinogen and/or fibrinogen derivatives
Procédé de détection du fibrinogène et/ou de ses derivés

(30) Priorität: 04.06.2003 DE 10325173
(43) Veröffentlichungstag der Anmeldung: 08.12.2004
(60) Teilanmeldung: 07009386.9
(73) Patentinhaber: Stief, Thomas, Dr., 35415 Pohlheim (DE)
(72) Erfinder: Stief, Thomas, Dr., 35415 Pohlheim (DE)
(74) Vertreter: Ackermann, Joachim

(56) Entgegenhaltungen:
- EP-A- 0 137 269
- EP-A- 0 699 909
- DE-A- 4 133 946
- US-A- 5 292 664
- COLLER B S ET AL: "Effects of vancomycin on platelets, plasma proteins and hepatitis B surface antigen" THROMB.DIATHES.HAEMORRH. 1975, Bd. 34, Nr. 1, 1975, Seiten 83-93, XP009048680

## Beschreibung

Die vorliegende Erfindung betrifft Nachweisverfahren für Fibrinogen und/oder Fibrinogen-Derivate, die auf einer Trübungsmessung basieren.

Die Bestimmung der Aktivität und/oder der Konzentration von Fibrinogen und/oder von Fibrinogen-Derivaten im Blut und/oder Blutplasma ist von hoher klinischer Bedeutung. Unter Fibrinogen-Derivaten werden erfindungsgemäß Derivate des Fibrinogens verstanden, wie sie insbesondere unter Einwirkung von Enzymen, insbesondere Thrombin und/oder Plasmin, oder von Oxidantien auf Fibrinogen entstehen. Beispielsweise sind Fibrin und/oder Fibrin- oder Fibrinogen-Spaltprodukte oder oxidiertes Fibrinogen Derivate des Fibrinogens.

Die nach dem Stand der Technik bekannten Tests zum Nachweis von Fibrinogen und/oder von Fibrinogen-Derivaten sind relativ komplex und/oder die Testbedingungen sind nicht physiologisch, beispielsweise durch den Zusatz von hohen Konzentrationen an Thrombin (EP-B-0137269, DE-A-4133946, US-B-6448 024, DE 41 33 946, US 5.292,664), so dass die Testergebnisse nicht zuverlässig die Fibrinogen-Aktivität und/oder die Konzentration an Fibrinogen-Derivaten, wie sie im Blut des Patienten vorliegt, wiedergeben.

Als nächstliegender Stand der Technik sind anzusehen der funktionelle Nachweis von Fibrinogen nach der modifizierten Clauss-Methode (DE-A-4133946) und die sogenannte PT-Fbgen-Methode (aus der Prothrombinzeit abgeleitetes Fibrinogen, beispielsweise Thromborel-S ^{®}, Firma DadeBehring, Marburg, Deutschland).

Bei dem Verfahren nach Clauss wird die Plasmaprobe ca. 10fach verdünnt, Thrombin zugesetzt und die Zeit des Gerinnungseintritts (=Gerinnungszeit) gemessen. Diese Gerinnungszeit korreliert hierbei indirekt mit der Konzentration an aktivem Fibrinogen der Probe. In einem modifizierten Clauss-Ansatz gemäß DE-A-4133946 wird 100 µl Probe mit 200 µl 50 IU/ml bovinem Thrombin, 0. 15 g/l Polymerisationsinhibitor Gly-Pro-Arg-Pro-Ala-amid, 1.5 g/l CaCl₂, 15 µg/ml Polybren^{®}, 0.8 g/l Polyethylenglycol 6000, 6.4 g/l NaCl, 50 mM Tris, 1 % bovinem Albumin versetzt und die Zeit des Gerinnungseintritts (= Gerinnungszeit) turbidimetrisch erfasst.

Bei der sogenannten Prothrombin-Zeit-abgeleiteten Fibrinogen-Konzentration (PT-Fbgen) wird aus der Trübung des im Test entstandenen Gerinnsels auf den Fbgen-Gehalt der Probe rückgeschlossen (Thromborel-S^{®}, DadeBehring). Problematisch ist hier die ungewisse Thrombin-Aktivität, die von Patient zu Patient unterschiedlich ist, welche in der PT die Gerinnung des Fibrinogens auslöst, weswegen die klinische Verlässlichkeit dieses Testes angezweifelt wird (Mackie et al., Thromb. Haemost., Band 87 (2002), S. 997-1005; s. Beispiel 1.1.3).

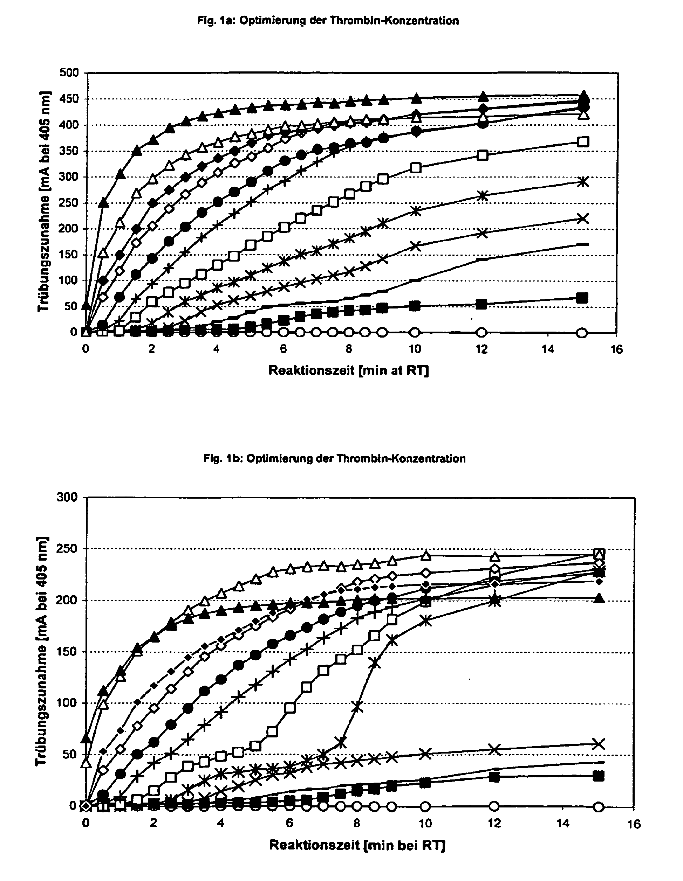

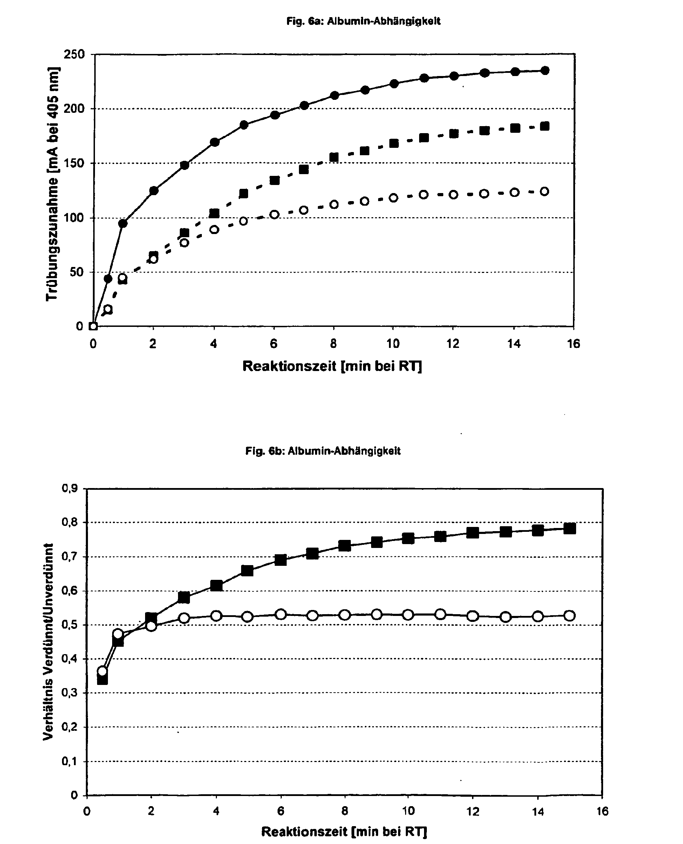

Sowohl 10fach-Verdünnung der Probe nach Clauss als auch Einsatz eines Polymerisationsinhibitors bei der modifizierten Clauss-Methode und/oder extrem hohe Thrombin-Konzentrationen (EP-B-0137269, DE-A-4133946, US-B-6448 024) sind unphysiologisch. Bei allen diesen Tests wird darüber hinaus nicht berücksichtigt, dass das Fibrinogen bei unphysiologischer Matrix - Matrix ist definiert als die Flüssigkeit mit all ihren Komponenten, in welcher die zu untersuchende Substanz eingebettet ist (d.h. die Umgebung des Analyten) - und/oder unphysiologisch hohen Thrombin-Aktivitäten entsprechend unphysiologisch reagieren kann, wie erfindungsgemäß belegt (s. Abb. 1a-d, 6a,b).

Aufgabe der vorliegenden Erfindung es daher, ein Verfahren zur Bestimmung der Fibrinogen-Aktivität bzw. der Fibrinogen-Konzentration zur Verfügung zu stellen, welches möglichst einfach anzuwenden ist, möglichst genau die wirklichen Aktivitäten und/oder Konzentrationen angibt und möglichst schnell durchzuführen ist und dabei nicht des Zusatzes unphysiologisch hoher Konzentrationen an Thrombin bedarf.

Gelöst wird diese Aufgabe durch turbidimetrische matrixunabhängige Verfahren, also durch Verfahren bei denen die Fibrinogen-Aktivität und/oder die Fibrinogen- und/oder Fibrinogen-Derivate-Konzentration durch den Trübungsanstieg der zu untersuchenden Lösung bestimmt wird. Diese Verfahren werden im folgenden Fibrinogen Functional Turbidimetric Assay (FIFTA) und Fibrinogen Antigenic Turbidimetric Assay (FIATA) genannt, wobei das letztere Verfahren nicht Gegenstand der vorliegenden Erfindung ist.

FIFTA steht für ein Verfahren zum Nachweis von Fibrinogen und/oder Fibrinogen-Derivaten und insbesondere zum Nachweis von Fibrinogen-Aktivität, das folgende Schritte umfasst:
a) zu einer auf Fibrinogen und/oder Fibrinogen-Derivate zu untersuchenden Lösung wird Thrombin und/oder Albumin in gelöster Form hinzugegeben, so dass sich finale Konzentrationen von weniger als 2 IU/ml Thrombin, vorzugsweise weniger als 1,5 oder 1 IU/ml und/oder weniger als 150 mlU oder 100 mlU pro 50 µl Probe, vor allem weniger als 0,8 oder 0,5 IU/ml und/oder weniger als 80 oder 50 mlU Thrombin pro 50 µl Probe, und/oder 1,7 - 10 % (w/v), vorzugsweise mehr als 2,5 % (w/v), vor allem mehr als 4 % (w/v) Albumin, einstellen,
b) der Trübungsanstieg der so erhaltenen Lösung wird bestimmt.

Der FIFTA imitiert die physiologische Umwandlung von Fibrinogen zu Fibrin, wie sie im Plasma stattfindet. Der FIFTA kann relativ einfach, sogar bei Raumtemperatur (RT), durchgeführt werden. Apparativ genügt ein einfaches Photometer, insbesondere ein Mikrotiterplatten-Photometer, spezielle Koagulometer oder Gerinnungs-Detektions-Systeme - d.h. Systeme, die Gerinnungszeiten messen, sind nicht erforderlich.

In einer bevorzugten Ausführungsform zeichnet sich das Verfahren dadurch aus, dass es ohne Zusatz eines Polymerisationsinhibitors durchgeführt werden kann.

Bei der zu untersuchenden Lösung handelt es sich vorzugsweise um Blut oder Blutplasma, das vorzugsweise mit Citrat, EDTA, Heparin und/oder einer Guanidinoverbindung wie beispielsweise Arginin versetzt wurde. Die Lösung kann beispielsweise in einem Volumen zwischen 0,1 und 100 µl, vorzugsweise zwischen 5 und 50 µl, insbesondere in einem Volumen von 25 - 50 µl, eingesetzt werden.

Albumin und Thrombin liegen vorzugsweise gemeinsam in einer Flüssigkeit gelöst vor, bevor sie zu der zu untersuchenden Lösung hinzugegeben werden. Das Volumen-Verhältnis zwischen zu untersuchender Lösung und zur Durchführung des Assays hinzuzugebender Thrombin- und/oder Albumin-haltiger Lösung beträgt dann vorzugsweise zwischen 1:1 und 1:5, besonders bevorzugt etwa 1:2.

Die Thrombin- und/oder Albumin-haltige Lösung (FIFTA-Reagens) kann entsprechend beispielsweise in einem Volumen zwischen 0,2 und 200 µl, vorzugsweise in einem Volumen zwischen 10 und 100 µl, vor allem in einem Volumen von etwa 50-100 µl eingesetzt werden.

Die optimale Thrombin-Konzentration im FIFTA-Reagens liegt zwischen 50 und 1000 mlU/ml (Zusatz von 5-100mIU Thrombin pro 50 µl Probe), bevorzugt zwischen 100 und 500 mIU/ml, besonders bevorzugt zwischen 200 und 400 mlU/ml, insbesondere bei etwa 300 mlU/ml, vor allem wenn es im Verhältnis 2:1 zu der zu untersuchenden Lösung eingesetzt wird. Thrombin-Konzentrationen im FIFTA-Reagenz von 200-450 mlU/ml führen zu einer ΔA/5 min (RT) von ca. 170-250 mA bei Normalplasma-Pool (100 % der Norm funktionelles Fibrinogen = 2.8 g/l aktives Fibrinogen) und 250-350 mA/5 min (RT) für Patientenplasma-Pool mit 144 % der Norm funktionelles Fibrinogen, wenn das Thrombin-Reagenz im Verhältnis 2:1 zu der zu untersuchenden Lösung eingesetzt wird. Alternativ kann Fibrinogen auch durch ein anderes Protein und/oder Proteingemisch, wie beispielsweise Reptilase oder Staphylokoagulase, zu Fibrin umgesetzt werden. Gegebenenfalls kann das FIFTA-Reagenz Calcium-lonen enthalten, insbesondere 10-15 mM bei einem Verhältnis FIFTA-Reagenz : Probe = 2:1.

Die optimale Albumin-Konzentration im FIFTA-Reagens beträgt 2 bis 20 %, insbesondere 4 bis 8 %, vorzugsweise etwa 6 %, vor allem wenn es im Verhältnis 2:1 zu der zu untersuchenden Lösung eingesetzt wird. Verwendung findet hierbei besonders bevorzugt Rinderserum-Albumin (BSA) oder Humanalbumin. Alternativ können auch Albumin-Ersatzstoffe, die dem Fachmann bekannt sind, beispielsweise Proteine oder Proteindegradationsprodukte wie Gelatine, eingesetzt werden. Der Zusatz von Albumin verhindert eine Verfälschung des Messergebnisses durch hypoproteinämische Plasmen, die zu einer apparenten Erhöhung des Fibrinogen-Wertes führen würden (Abb. 6a,b).

Vorzugsweise wird dem FIFTA-Reagens auch Polybren^{®} zugesetzt. Die optimale Polybren-Konzentration im FIFTA-Reagens liegt zwischen 0 und 30000 µg/ml, bevorzugt zwischen 0 und 1000 µg/ml, besonders bevorzugt zwischen 200 und 800 µg/ml, insbesondere bei etwa 400 µg/ml, vor allem wenn das FIFTA-Reagens im Verhältnis 2:1 zu der zu untersuchenden Lösung eingesetzt wird, und/oder Zusatz von 0-100 µg Polybren pro 50 µl Probe, besonders bevorzugt 20-80 µg Polybren pro 50 µl Probe, insbesondere bei etwa 40 µg Polybren pro 50 µl Probe.
Bei Untersuchung von Heparin-Plasmen (Plasmen mit bis zu 100 IU/ml Heparin) kann gegebenenfalls die Polybren-Konzentration 1000-30000 µg/ml im FIFTA-Reagenz betragen und/oder die zuzusetzende Polybren-Menge 100-3000 µg pro 50 µl Probe sein.
Alternativ kann ein Heparin-Neutralisator wie beispielsweise Polybren^{®} oder Heparinase zuerst zugesetzt werden und dann erst die Thrombin-Reaktion gestartet werden. Hierzu können Heparin-Plasmen zuerst mit 25 µl 4-200 mg/ml, bevorzugt 10-100 mg/ml, insbesondere ca. 50 mg/ml Polybren^{®} in 40 mM Citrat, pH 7.4, pro 50 µl Plasma gemischt werden, d.h. Zusatz von 100-5000 µg, bevorzugt 250-2500 µg, insbesondere ca. 1250 µg Polybren pro 50 µl Probe.

Durch den Einsatz von 400 µg/ml Polybren werden bis zu 20 IU/ml Heparin im Plasma neutralisiert. Normale therapeutische Heparin-Konzentrationen im Blut betragen bis zu 0,2 IU/ml. Bei kardiochirurgischen Patienten etwa kann die Heparin-Konzentration jedoch auf bis zu ca. 10 IU/ml erhöht sein. Eine Fibrinogen-Bestimmung ist jedoch gerade bei diesen Patienten sehr wichtig, da gerade bei diesen Patienten die Gefahr einer pathologischen disseminierten intravaskulären Gerinnung sehr groß ist. Die hohe Heparin-Konzentration würde jedoch die Fibrinogen-Bestimmung empfindlich stören, so dass zum Erhalt eines verlässlichen Test-Ergebnisses die Zugabe von Polybren zur Neutralisierung des Heparins erforderlich ist. Beispielsweise würde, falls das FIFTA-Reagens kein Polybren enthielte, plasmatische Heparin-Konzentrationen ≥ 0.63 IU/ml vollständig das zugesetzte Thrombin im in Tab. 1 beschriebenen Testsystem inhibieren. Die inhibitorische Konzentration 50 % betrüge dann 0.1 IU/ml Heparin.

Die zuvor genannten Komponenten des FIFTA-Reagens sind vorzugsweise gelöst in einem Puffer, besonders bevorzugt in Phosphat-gepuffertem NaCl (PBS-Lösung; 10 mM Na₂HPO₄, 138 mM NaCl, 2.7 mM KCI, pH 7.4).

Der FIFTA wird vorzugsweise bei einer Temperatur zwischen 0°C und 42°C, besonders bevorzugt bei etwa 37°C oder, weil am einfachsten zu handhaben, bei Raumtemperatur durchgeführt. Die Reaktionszeit beträgt vorzugsweise zwischen 20 Sekunden und 60 Minuten.

Die optimale Reaktionszeit für den FIFTA bei Raumtemperatur ist 1 bis 20 Minuten, besonders bevorzugt 2 bis 10 Minuten, vor allem 3 bis 7 Minuten, insbesondere etwa 5 Minuten. Die entsprechenden Zeiten für 37°C ergeben sich durch Division der zuvor angegebenen Zeiten mit dem Faktor 2,5.

Die Messung der Trübung erfolgt vorzugsweise durch spektrometrische Absorptionsmessung bei einer Wellenlänge zwischen 280 und 650 nm, besonders bevorzugt bei etwa 405 nm.

Standardisiert wird die Messung durch Vergleichsmessung mit einer Lösung bekannter Konzentration an funktionellem Fibrinogen, vorzugsweise durch Vergleichsmessung mit einem Aliquot eines Normalplasma-Pools, der 100 % der Norm entspricht (= Mittelwert (MW) des Normalbereichs = 2,8 g/I).

Beim FIFTA handelt es sich erfindungsgemäß um ein Verfahren, mit dem sowohl sehr hohe als auch sehr niedrige Fibrinogen-Aktivitäten sehr genau bestimmt werden können. So können Konzentrationen von mehr als 200 %, vorzugsweise mehr als 220 %, insbesondere mehr als 240 % der Norm, wie auch Werte unter 50 %, vorzugsweise unter 40 %, insbesondere unter 30 % der Norm mit dem FIFTA bestimmt werden. In einer bevorzugten Ausführungsform kann die untere Nachweisgrenze auf unter 10 % der Norm gesenkt werden.

Nach ca. 15 Minuten Inkubation bei Raumtemperatur erreicht der Test asymptotisch maximale Absorptionswerte (für 100 % der Norm Plasma = ca. 420 mA). Der halbmaximale Absorptionsanstieg tritt bei Raumtemperatur nach etwa 4 Minuten auf. Etwa 60 % des Trübungsmaximums wird bei Raumtemperatur nach ca. 5 Minuten erreicht. Der FIFTA ist bei optimaler Durchführung linear bis zu einer Fibrinogen-Aktivität von etwa 250 % der Norm. Die untere Nachweisgrenze bei Inkubation von 5 min bei Raumtemperatur ist 25 % der Norm. Bestimmt man den Trübungsanstieg/15 min (RT; oder ΔA/5-6 min 37°C), so sinkt die untere Nachweisgrenze auf 7 % der Norm (0,2 g/l).

Die intra- und inter-assay Variations-Koeffizient (VK) - Werte liegen unter 4 %. Der Normalbereich an funktionellem Fibrinogen gemessen durch FIFTA ist 100 % ± 20 % (Mittelwert ± eine Standardabweichung). Der FIFTA korreliert mit der modifizierten Clauss-Methode bei n=334 Patienten-Plasmen mit r=0.843.

Der FIFTA wird vorzugsweise eingesetzt für diagnostische Zwecke, insbesondere bei der pathologischen disseminierten intravaskulären Gerinnung und zur Beurteilung des Patienten-Risikos für atherothrombotische Erkrankungen wie myokardiale oder cerebrale Ischämie/Infarkt.

Gegenstand der vorliegenden Erfindung ist daher auch die Verwendung eines Diagnostikum, enthaltend Thrombin und/oder Albumin sowie gegebenenfalls Polybren. Die Komponenten können hierbei unmittelbar, wie zuvor beschrieben, bereits in gelöster Form vorliegen, oder aber in fester Form vorliegen, wobei sie dann, zur Durchführung des Verfahrens, erst in die lösliche Form überführt werden müssen. Erfindungsgemäß bevorzugt sind hierbei Konzentrationen und Lösungen wie zuvor beschrieben. Es handelt sich hierbei vorzugsweise um ein Diagnostikum zum Nachweis der pathologischen disseminierten intravaskulären Gerinnung und/oder um ein Diagnostikum zur Beurteilung des Patienten-Risikos für atherothrombische Erkrankungen wie myokardiale oder cerebrale Ischämie/Infarkt. Erfindungsgemäß für den Einsatz bevorzugt sind hierbei Konzentrationen und Lösungen wie zuvor beschrieben.

Gegenstand der vorliegenden Erfindung ist auch die Verwendung eines Testsystems bzw. Kits zum Nachweis von Fibrinogen und/oder Fibrinogen-Derivaten, dadurch gekennzeichnet, dass es eine Lösung enthaltend Thrombin und/oder Albumin und gegebenenfalls eine Lösung enthaltend Polybren umfasst, wobei Thrombin, Albumin und/oder Polybren in derselben oder in verschiedenen Lösungen enthalten sein können. Erfindungsgemäß bevorzugt sind hierbei Konzentrationen und Lösungen wie zuvor beschrieben.

Gegenstand der vorliegenden Erfindung ist desweiteren die Verwendung des Diagnostikums, des Testsystems bzw. Kits zur Bestimmung der Fibrinogen- und/oder Fibrinogen-Derivat-Konzentration und/oder -Aktivität.

Überraschenderweise wurde des weiteren gefunden, daß auch das handelsübliche Antibiotikum Vancomycin mit Fibrinogen und/oder Fibrinogen-Derivaten auf eine Weise interagiert, die in einem turbidimetrischen Assay zur Konzentrationsbestimmung verwendet werden kann. Dabei wird das lösliche Fibrinogen und/oder Fibrinogen-Derivate durch die Bindung des Vancomycins in eine unlösliche Form umgewandelt. Diese unlösliche Fibrinogen- und/oder Fibrinogen-Derivat-Form präzipitiert. Die dabei entstehende Trübung ist ein direktes Maß für die Fibrinogen- und/oder Fibrinogen-Derivat-Konzentration in der Probe. Diese Reaktion ist die Basis für einen einfachen standardisierten Antigen-Test auf Fibrinogen und/oder Fibrinogen-Derivate, den Fibrinogen Antigenic Turbidimetric Assay (FIATA).

FIATA steht somit für ein Verfahren zum Nachweis von Fibrinogen und/oder Fibrinogen-Derivaten, folgende Schritte umfassend:
a) eine zu untersuchende Lösung wird mit einer anderen Lösung enthaltend Vancomycin versetzt, vorzugsweise so, dass zwischen 0,05 und 1 µmol, besonders bevorzugt zwischen 0,1 und 0,5 µmol, vor allem zwischen 0,15 und 0,3 µmol, insbesondere etwa 0.22 µmol Vancomycin auf 25 µl Plasma kommen,
b) der Trübungsanstieg wird gemessen.

Bei der zu untersuchenden Lösung handelt es sich vorzugsweise um Blut oder Blutplasma, insbesondere um Citrat-, Heparin-, EDTA und/oder Guanidinoverbindungs-Plasma wie beispielsweise Arginin-Plasma (Beispielsweise Zusatz von 9 Teilen Blut zu 1 Teil 106 mM Citrat, 15 IU Heparin/ml Blut, 1.6 mg Kalium-EDTA/ml Blut, und/oder 50-1000 mM Arginin (pH 8,7)/ml Blut). Auch hier genügt apparativ ein einfaches Photometer, insbesondere ein Mikrotiterplatten-Photometer

Im FIATA wird vorzugsweise ein Teil Probe mit einem Volumen zwischen 0,1 und 100 µl, besonders bevorzugt mit einem Volumen zwischen 5 und 50 µl, vor allem mit einem Volumen von etwa 25 µl, mit zwei Teilen PBS-Puffer (Phosphat-gepuffertes Natriumchlorid, gegebenenfalls enthaltend Albumin, insbesondere 6% Albumin) entsprechend einem Volumen zwischen 0.2 und 200 µl, besonders bevorzugt zwischen 10 und 100 µl, vor allem mit einem Volumen von etwa 50 µl umgesetzt, um die zu messende Lösung bereitzustellen. Anstelle des PBS-Puffers kann auch eine andere Flüssigkeit (beispielsweise 0.9% NaCl oder H₂O) zum Verdünnen der Probe verwendet werden. Diese Verdünnung dient dazu, den darauffolgenden Trübungsanstieg besser erfassen zu können. Wenn das Photometer kleine Volumina sicher misst, kann auf diese Verdünnung auch verzichtet werden.

Die Vancomycin-haltige Lösung (FIATA-Reagens) wird zu der zu untersuchenden Lösung vorzugsweise in einem Volumenverhältnis von etwa 2:3, entsprechend, unter Bezug auf die oben genannten Volumina, in einem Volumen zwischen 0,2 und 200 µl, besonders bevorzugt in einem Volumen zwischen 10 und 100 µl, vor allem in einem Volumen von etwa 50 µl hinzugegeben.
Die Vancomycin-Konzentration im FIATA-Reagens wird vorzugsweise so gewählt, dass sich nach Zugabe des FIATA-Reagens zu der zu messenden Lösung eine finale Vancomycin-Konzentration von 0,4 bis 20 mM, besonders bevorzugt von zwischen 0,8 und 4 mM, vor allem von zwischen 1 und 3 mM, insbesondere von etwa 1,76 mM einstellt, d.h. insbesondere Zusatz von etwa 0.22 µmol Vancomycin/25 µl Plasma. Üblicherweise beträgt die Vancomycin-Konzentration im FIATA-Reagens entsprechend zwischen 1 und 20 mM, bevorzugt 2 und 7 mM, insbesondere etwa 4,4 mM, so dass sich nach Verdünnung die vorgenannten Konzentrationen einstellen. Gelöst ist das Vancomycin vorzugsweise in aqua dest. oder in PBS (Phosphat-gepufferte Natriumchlorid-Lösung, gegebenenfalls enthaltend Albumin, insbesondere 6% Albumin). Alternativ können auch andere dem Fachmann bekannte Puffer zur Herstellung des FIATA-Reagens verwendet werden. Die Vancomycin-haltige Lösung kann stabilisiert werden durch ein Detergens wie Triton X^{®}, besonders 0,1 % (w/v), und/oder ein Protein wie Albumin, besonders 1-7% (w/v).

Der FIATA wird vorzugsweise bei einer Temperatur zwischen 0°C und 42°C, besonders bevorzugt bei etwa 37°C oder, weil am einfachsten zu handhaben, bei Raumtemperatur durchgeführt.

Die optimale Reaktionszeit für den FIATA bei Raumtemperatur ist 2 Sekunden bis 10 Minuten, bevorzugt 5 Sekunden bis 5 Minuten, besonders bevorzugt 10 Sekunden bis 3 Minuten, vor allem 1 bis 3 Minuten. Für 37°C halbieren sich die Reaktionszeiten.

Die Messung der Trübung erfolgt vorzugsweise durch spektrometrische Absorptionsmessung bei einer Wellenlänge zwischen 280 und 650 nm, besonders bevorzugt bei einer Wellenlänge von etwa 405 nm.

Standardisiert wird die Messung durch Vergleichsmessung mit einer Lösung bekannter Konzentration an Fibrinogen, vorzugsweise durch Vergleichsmessung mit einem Aliquot eines Normalplasma-Pools, der 100 % der Norm entspricht (= Mittelwert (MW) des Normalbereichs = 2,8 g/l).

Der FIATA besitzt hierbei eine obere Linearitätsgrenze von mehr als 100 % der Norm, besonders bevorzugt mehr als 120 % der Norm, vor allem mehr als 140 % der Norm und/oder eine untere Nachweisgrenze von weniger als 20 % der Norm, besonders bevorzugt weniger als 10 % der Norm, vor allem weniger als 5 % der Norm.
Der FIATA ist in einer bevorzugten Ausführungsform annähernd linear bis zu einer Fibrinogen-Konzentration von etwa 150 % der Norm (4,2 g/l), wobei eine ΔA von 400-800 mA, insbesondere 600 mA resultiert. Die untere Nachweisgrenze ist 4 % der Norm (0.1 g/I).
Proben mit Fibrinogen-Konzentrationen > 150 % sollten verdünnt werden, vorzugsweise mit 6 % Albumin-PBS, gepooltem Serum, PBS oder 0.9 % NaCl, besonders bevorzugt mit 6 % Albumin-PBS. Alternativ kann die eingesetzte Probenmenge von 25 µl auf 5 - 15 µl, insbesondere 10-12.5 µl reduziert werden, ohne Änderung der zuzusetzenden Vancomycin-Menge.

Die intra- und inter-assay VK-Werte liegen unter 4 %. Der Normal-Bereich des FIATA ist 100 % ± 20 % (Mittelwert (MW) ± eine Standardabweichung (SD)). Bei n=321 bzw. 344 Patienten-Plasmen korrelierte der FIATA (MW=130 %; SD=52 % bzw. 43 %) mit den funktionellen Fibrinogen-Tests a) nach der modifizierten Clauss-Methode (MW=4.1 g/l; SD =1.7 g/l) mit r=0.755 und b) mit dem FIFTA (MW=124 %; SD=40 %) mit r=0.813.

Der FIATA ist geeignet nicht nur zum Nachweis von Fibrinogen, sondern auch zum Nachweis von löslichem Fibrin und/oder denaturierten Fibrinogen-Molekülen. Daher ist der FIATA auch als Screening-Test für Dysfibrinogene oder nichtfunktionelles Fibrinogen geeignet. Zum Nachweis des Gesamtgehaltes an Fibrinogen und/oder Fibrinogen-Derivaten muss das Fibrinogen hierbei nicht erst zu Fibrin umgewandelt werden, so dass auf den Zusatz von Thrombin verzichtet werden kann.

Der FIATA ist so einfach durchzuführen, dass die Bestimmung der Fibrinogen- und/oder Fibrinogen-Derivate-Konzentration innerhalb von Sekunden sogar außerhalb eines Krankenhauses durchgeführt werden kann. Daher kann der FIATA dazu beitragen, Patienten außerhalb eines Krankenhauses auf deren Risiko hinsichtlich einer myokardialen und/oder cerebralen Ischämie/Infarkt zu untersuchen.

Beschreiben wird weiterhin auch ein Diagnostikum, enthaltend Vancomycin. Das Vancomycin kann hierbei unmittelbar, wie zuvor beschrieben, bereits in gelöster Form vorliegen, oder aber in fester Form vorliegen, wobei es dann, zur Durchführung des Verfahrens, erst in die lösliche Form überführt werden muss. Bevorzugt sind hierbei Konzentrationen und Lösungen wie zuvor beschrieben. Es handelt sich hierbei vorzugsweise um ein Diagnostikum zum Nachweis der pathologischen disseminierten intravaskulären Gerinnung (DIC) und/oder um ein Diagnostikum zur Beurteilung des Patienten-Risikos für atherothrombische Erkrankungen wie myokardiale oder cerebrale Ischämie/Infarict. Für den Einsatz bevorzugt sind hierbei Konzentrationen und Lösungen wie zuvor beschrieben. Insbesondere kann die Errechnung des FIATA/FIFTA Quotienten dazu beitragen, eine Hämostaseaktivierung wie beispielsweise bei einer DIC und/oder eine Dysfibrinogenämie zu dagnostizieren: Werte ≥ 1.1, insbesondere Werte ≥ 1.3, sind hinweisend auf eine DIC und/oder eine Dysfibrinogenämie.

Offenbart wird auch ein Testsystem bzw. Kit zum Nachweis von Fibrinogen und/oder Fibrinogen-Derivaten, dadurch gekennzeichnet, dass es eine Vancomycin-haltige Lösung umfasst. Bevorzugt sind hierbei Konzentrationen und Lösungen wie zuvor beschrieben.

Optimierte Ausführungsformen für den FIATA und den FIFTA sind in den Tabellen 1 und 2 dargestellt.

**Tabelle 1: Optimierte Parameter zur Durchführung des FIFTA**

| |
|---|
| 50 µl Plasma oder Standard (beispielsweise 100 % Standard = gepooltes Normal-Plasma) 100 µl 300 mlU/ml Thrombin, 6 % Albumin-PBS, 400 µg/ml Polybrene^{®} in PBS |
| A (405 nm) |
| 5 min (RT) oder 2 min (37°C) |
| ΔA (405 nm) |

Der optimierte FIFTA wird wie folgt durchgeführt:
Zu 50 µl Citrat-Plasma (Zusatz von 9 Teilen Blut zu 1 Teil 106 mM Citrat) werden 100 µl FIFTA-Reagens hinzugegeben, das 300 mlU/ml Thrombin, 6% Albumin-PBS, 400 µg/ml Polybrene®, aufgefüllt mit PBS, enthält. Die Absorption bei 405 nm wird sofort gemessen (base value) und nach 5 Minuten, wenn die Reaktion bei Raumtemperatur durchgeführt wird. Wenn die Reaktion bei 37 °C durchgeführt wird, wird der zweite Wert nach 2 Minuten gemessen. Verwendet wird ein Microtiterplatten-Reader (Milenia, DPC, Los Angeles, USA). Die Zunahme der Absorption (ΔA) wird bestimmt und die Ergebnisse werden standardisiert gegen einen Plasmapool bekannter, vorzugsweise 100 % normaler Fibrinogen-Aktivität (Tabelle 1).

**Tabelle 2: Optimierte Parameter zur Durchführung des FIATA**

| |
|---|
| 25 µl Plasma oder Standard (beispielsweise 100 % Standard = gepooltes Normal-Plasma) 50 µl PBS |
| A (405 nm) |
| 50 µl 4,4 mM Vancomycin in PBS 2 min (RT) oder 1 min (37°C) |
| ΔA (405 nm) |

Der optimierte FIATA wird wie folgt durchgeführt:
Zu 25 µl Plasma werden 50 µl PBS zugesetzt und die Absorption (405 nm) wird gemessen (Basis-Wert). Anschließend werden 50 µl 4,4 mM Vancomycin in PBS hinzugegeben. Die Absorption bei 405 nm wird sofort gemessen (Basis-Wert) und nach 2 Minuten, wenn die Reaktion bei Raumtemperatur durchgeführt wird. Wenn die Reaktion bei 37 °C durchgeführt wird, wird der zweite Wert nach 1 Minute gemessen. Verwendet wird ein Microtiterplatten-Reader (Milenia, DPC Biermann, Krefeld, Germany). Die Zunahme der Absorption (ΔA) wird bestimmt und die Ergebnisse werden standardisiert gegen einen Plasmapool mit bekannter Konzentration an Fibrinogen-Antigen, vorzugsweise 100 % der Norm und gegen 0.9 % NaCl (Trübungs-Nullwert)(Tabelle 2).

### Beispiele

### 1. FIFTA

### 1.1. Test-Optimierung

### 1.1.1. Thrombin-Konzentration

Die Thrombin-Konzentration im Test sollte möglichst physiologisch sein; weder zuviel Thrombin noch zuwenig Thrombin sind geeignet für einen idealen funktionellen Fibrinogen-Test. 50 µl Patienten-Pool (P-Pool; 144 % der Norm = 4.04 g/l aktives Fibrinogen) und 1:2 mit 6% bovines Serum-Albumin, phosphatgepufferte physiol. NaCl (BSA-PBS) verdünnter P-Pool wurden mit 100 µl 0 - 1519 mlU/ml bovinem Thrombin 0 - 15 min (bei Raumtemperatur = RT) in Mikrotiterplatten mit Vertiefungen mit flachem Boden inkubiert und die Trübungs-Zunahme bei 405 nm wurde bestimmt.

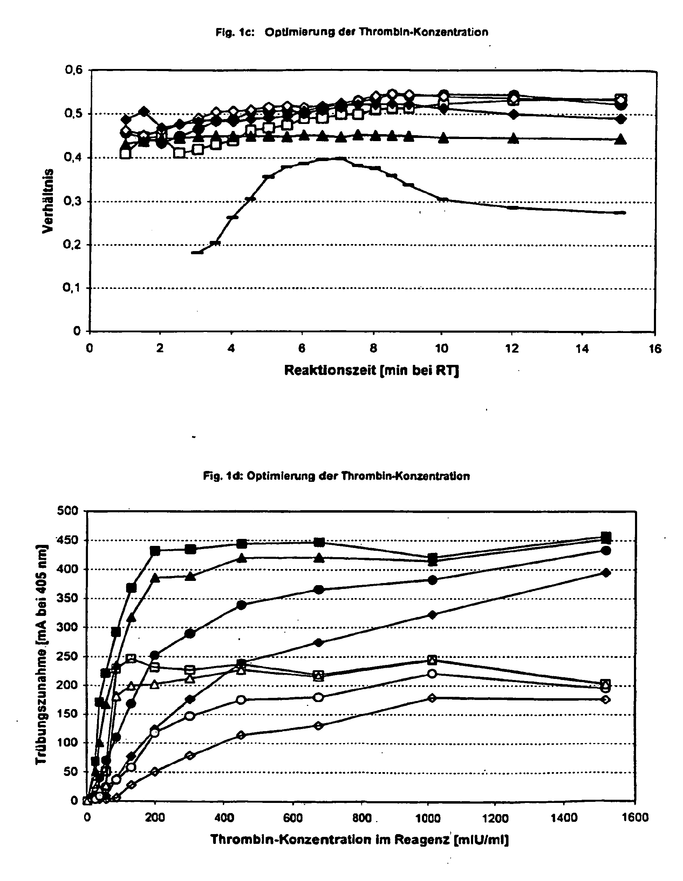

Innerhalb der ersten 5 min Reaktionszeit bei RT ist der Trübungs-Anstieg für P-Pool und für 1:2 verdünnten P-Pool nahezu linear, wenn ein Thrombin-Reagenz mit 200-450 mlU/ml Thrombin verwendet wird (Abb. 1a,b). Der korrekte Quotient von 0.5 aus den Absorptions-Anstiegen bei verdünntem P-Pool und bei P-Pool wird erreicht durch 200-450 mlU/ml Thrombin (Abb. 1c). Der maximale Trübungs-Anstieg für P-Pool ist etwa 450 mA; ca. 70 % des maximalen Trübungs-Anstiegs wird erreicht bei Verwendung von 300 mlU/ml Thrombin im Thrombin-Reagenz und 5 min Reaktionszeit bei RT (Abb. 1d). Daher ist die optimale Thrombin-Konzentration im FIFTA-Reagenz 200-450 mIU/ml, was zu einer ΔA/5 min (RT) von 250-350 mA/5 min für P-Pool führt. 300 mlU/ml wurde als die optimale Thrombin-Konzentration für das FIFTA-Reagenz ausgewählt, d.h. 200 mlU/ml finale IIa Konzentration, welche in eine ΔA/5 min von ca. 300 mA für P-Pool resultiert. Die basale Absorption des 100 % Pool-Plasmas ist etwa 150 mA, Zusatz von 100 µl 6 % BSA-PBS erhöht die Absorption um ca. 100 mA.

### 1.1.2. Polybrene^{®}-Konzentration

P-Pool supplementiert mit 0-80 IU/ml Heparin wurde im FIFTA analysiert. Das FIFTA-Reagenz enthielt 200 mlU/ml Thrombin, 6% BSA-PBS und 0, 50, 100, 200, 400, 800 oder 1600 µg/ml Polybrene® (PB; Hexadimethrinbromid).

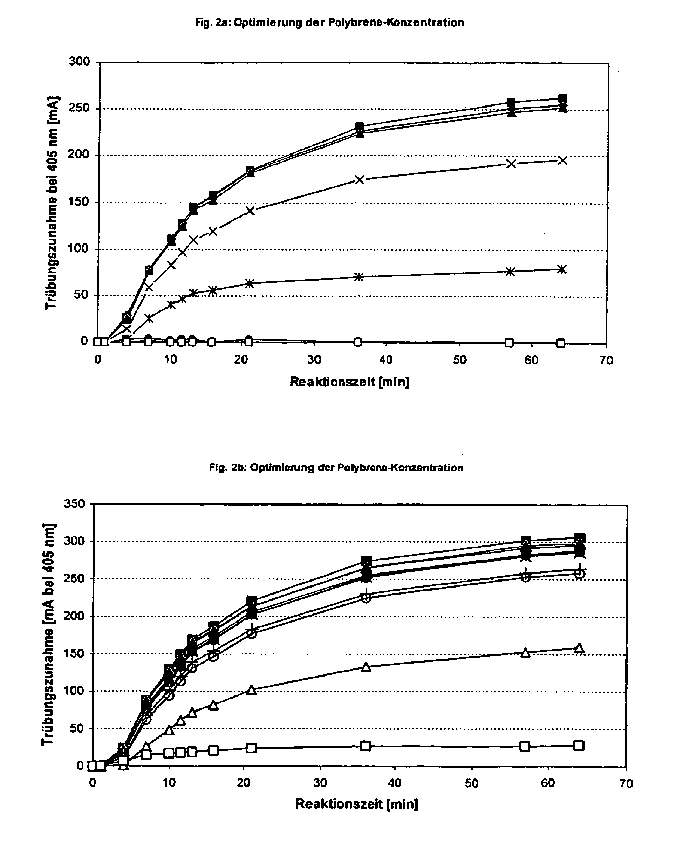

Figs.2a-g zeigen die Neutralisierung (Inhibition) von Heparin durch Polybrene^{®}. Wenn das FIFTA-Reagenz 400 µg/ml Polybrene^{®} enthält, werden plasmatische Heparin-Konzentrationen bis zu 20 IU/ml neutralisiert (inhibiert).

### Polybrene^{®}-Zusatz zu heparinisiertem Plasma

25 µl 0-75 mg/ml Polybrene^{®} in aqua dest. wurden zu 50 µl P-Pool oder zu 50 µl mit 100 IU/ml Heparin supplementiertem P-Pool zugesetzt. Dann wurden 100 µl 200 mlU/ml Thrombin in 6 % BSA-PBS, 400 µg/ml PB zugesetzt und die Absorbtions-Zunahme bei 405 nm gemessen.

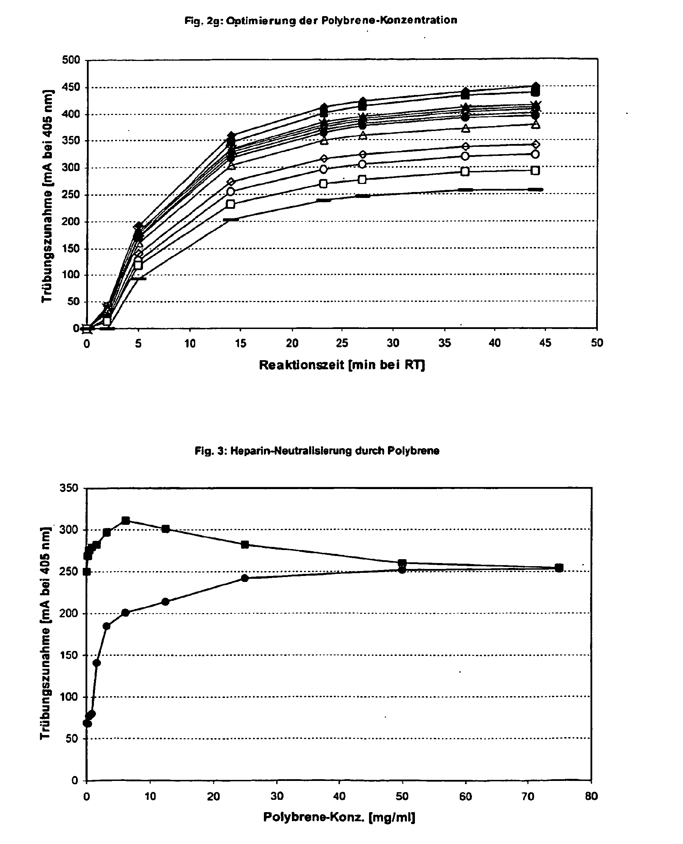

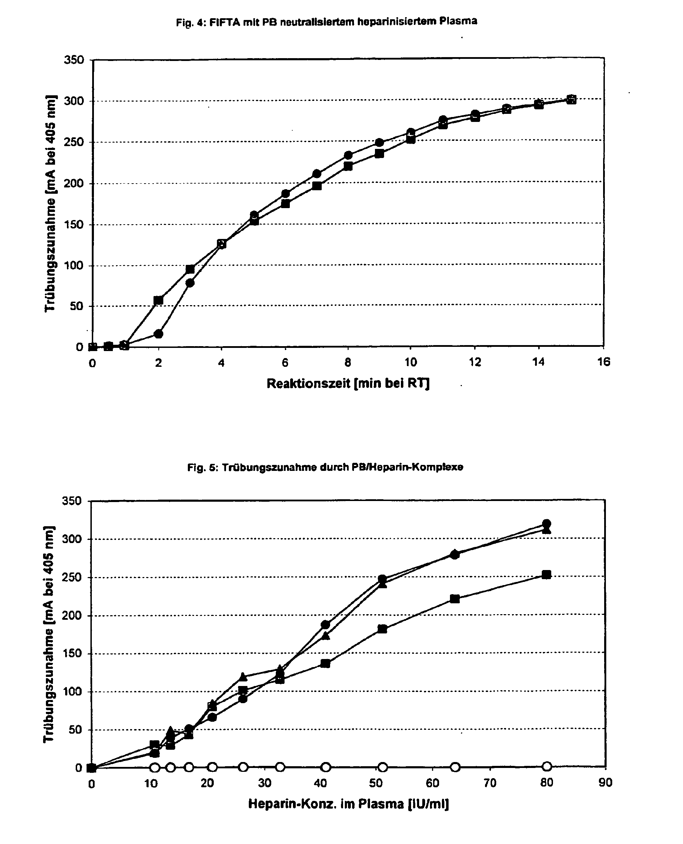

Zusatz von 25 µl ≥ 50 mg/ml Polybrene® antagonisiert völlig 100 IU/ml Heparin in 50 µl Plasma (Abb. 3). Das FIFTA-Reagenz enthält 400 µg/ml Polybrene^{®} (PB); folglich benötigt man zur Neutralisierung von 1 Teil 100 IU/ml Heparin-Plasma 1 Teil 26 mg/ml PB. PB-Heparin Komplexe in Plasma verändern nicht die Fibrinogen/Thrombin-Trübungskinetik (Abb. 4).

### Trübungs-Zunahme durch Heparin/Polybrene^{®} Komplexe

50 µl P-Pool mit 0-80 IU/ml Heparin wurden mit 100 µl FIFTA-Reagenz ohne Thrombin, welches 0, 400, 800, oder 1600 µg/ml PB enthielt inkubiert. Die Trübungs-Zunahme bei 405 nm wurde bestimmt.

Der basale Trübungs-Anstieg in Heparin-Plasma hängt ab von der Heparin-Konzentration des Plasmas und von der PB Konzentratin im FIFTA-Reagenz (Abb. 5). Der basale Trübungs-Anstieg durch 25 µl 50 mg/ml PB zugesetzt zu 50 µl P-Pool beträgt ca. 200 mA (Abb. 4).

### 1.1.3. Albumin-Konzentration

P-Pool oder P-Pool 1:2 verdünnt mit PBS oder mit 6 % BSA-PBS wurden im FIFTA analysiert, indem das 200 mlU/ml Thrombin-Reagenz ohne Albumin verwendet wurde.

Trübungs-basierte funktionelle Fibrinogen-Bestimmungen hängen ab von der Albumin-Konzentration der Probe. Der FIFTA dagegen hängt nicht ab von der Albumin-Konzentration der Probe. Ohne Albumin im FIFTA-Reagenz würde der FIFTA jedoch von der Albumin-Konzentration der Probe abhängen: wenn der P-Pool 1:2 mit PBS verdünnt wird, beträgt das gemessene Fibrinogen ca. 70 % des unverdünnten Plasmas anstatt 50 %; dies bedeutet, dass bei Patienten mit auf ca. 50 % der Norm erniedrigten Albuminkonzentrationen im Blut das turbidimetrisch gemessene funktionelle Fibrinogen ca. 40 % falsch zu hoch gemessen wird. Wenn der P-Pool 1:2 verdünnt wird mit 6 % Albumin-PBS, beträgt das gemessene Fibrinogen tatsächlich 50 % des unverdünnten P-Pools (Figs. 6a,b). Zusatz von 6 % Albumin zum FIFTA-Reagenz resultiert in dem korrekten 50 % Ergebnis bei allen verdünnten Plasmen, entweder verdünnt mit PBS oder mit 6 % Albumin.

### Vergleich der erfindungsgemäßen Fibrinogen-Testmethoden mit dem Stand der Technik

Zum Vergleich wurde gepooltes Patientenplasma (P-Pool, n=32 Einzelplasmen; normale Prothrombinzeit und aktivierte partielle Thromboplastinzeit (APTT), sowie 1:2, 1:4 und 1:8 Verdünnungen dieses P-Pools mit 0.9 % NaCl nach folgenden Methoden auf Fibrinogen getestet:
A) FIFTA (Tab. 1)
B) FIATA (Tab. 2)
C) modifizierte Clauss (DadeBehring, Bedingungen S. 2, Z. 1-6)
D) PT-Fbgen (DadeBehring, Bedingungen S. 2, Z. 8-10)

### Ergebnis:

| Methode | Plasma 1:1 | Plasma 1:2 | Plasma 1:4 | Plasma 1:8 | Plasma 1:16 |
|---|---|---|---|---|---|
| A) | 128 % | 64 % | 32 % | 16 % | 8 % |
| B) | 129% | 65% | 32% | 16% | 8% |
| C) | 3.7 g/l | 2.0 g/l | 0.6 g/l | n.m. | n.m. |
| D) | 3.5 g/l | 2.1 g/l | 1.3 g/l | 0.8 g/l | n.m. |

| | | | | | |
|---|---|---|---|---|---|
| n.m. = nicht messbar | | | | | |

Man erkennt, dass die Fibrinogen-Konzentrationen im FIFTA und FIATA korrekt wiedergegeben werden. Dagegen ist bei der mod. Clauss-Methode der im 1:2 verdünnten Plasma gemessene Fibrinogen-Wert um 8 % zu hoch und der im 1:4 verdünnten Plasma gemessene Fibrinogen-Wert um 35 % zu niedrig gegenüber dem zu erwartenden Wert. Bei der in der PT-Fbgen Methode ergibt sich für das 1:2 verdünnte Plasma ein Fibrinogen-Wert, der um 20 % zu hoch ist gegenüber dem zu erwartenden Wert, für das 1:4 verdünnte Plasma ergibt sich ein um 49 % erhöhter Fibrinogen-Wert gegenüber dem zu erwartenden Wert und für das 1:8 verdünnte Plasma ergibt sich ein um 83 % erhöhter Fibrinogen-Wert gegenüber dem zu erwartenden Wert.
Einige Patienten, insbesondere Intensivpatienten, haben plasmatische Albuminkonzentrationen von ca. 10 g/l, d.h. unter 25% der Norm. Dies kann zu stark verfälschten Fibrinogenwerten führen, wenn in solchen Plasmen nach dem Stand der Technik Fibrinogen bestimmt wird.

### 1.1.4. Reaktions-Kinetik

Der FIFTA wurde mit P-Pool und mit 6% BSA-PBS Verdünnungen von P-Pool durchgeführt. Die Trübungs-Zunahme wurde nach 0-15 min und nach 25 min, 75 min, 17 h bei RT bestimmt. Der FIFTA wurde weiterhin durchgeführt mit einem 100 % normalen Pool und einem 200 % Fibrinogen-Pool (256 % Fbgen-Pool verdünnt mit 100 % normalem gepooltem Plasma).

Der 256 % Fbgen-Pool, der 256 % Fbgen - Pool, der mit 6 % BSA-PBS zu 120 % oder zu 63 % verdünnt worden war, wurde im FIFTA mit 0-17 min Reaktionszeit (bei RT) untersucht. Die 120 % / 256 % und die 63 % / 256 % Trübungs-Quotienten wurden berechnet. Ein 300 % Fbgen-Pool, erzeugt durch Zusatz von gereinigtem aktiven Fibrinogen zu dem 256 % Fbgen-Pool, und der 1:2 verdünnte (mit 6% BSA-PBS) 300 % Fbgen-Pool wurde im FIFTA analysiert.

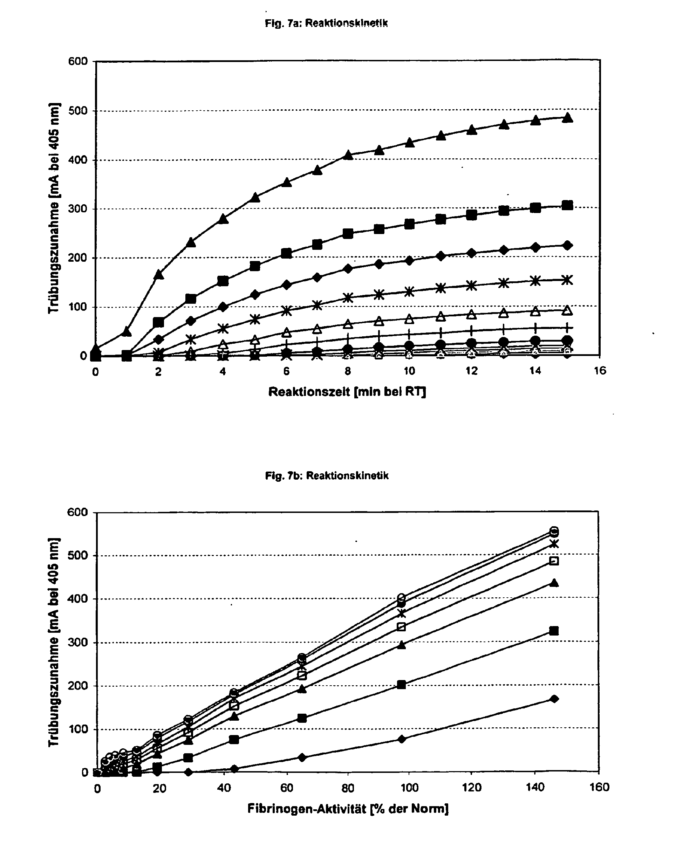

Figs. 7a,b zeigen die Reaktionskinetik des FIFTA. Bei ca. 15 min Reaktionszeit bei RT erreicht der Test asymptotisch sein Maximum. Nach ca. 5 min bei RT ist ca. 60 % des Maximums erreicht. Abb. 7b zeigt die Linearität der Kalibrationsgeraden des FIFTA. Eine lineare Beziehung zwischen Fibrinogen-Aktivität und Trübungs-Zunahme besteht bei Reaktionszeiten ≥ 5 min bei RT und aktivem Fibrinogen < 150 % der Norm.

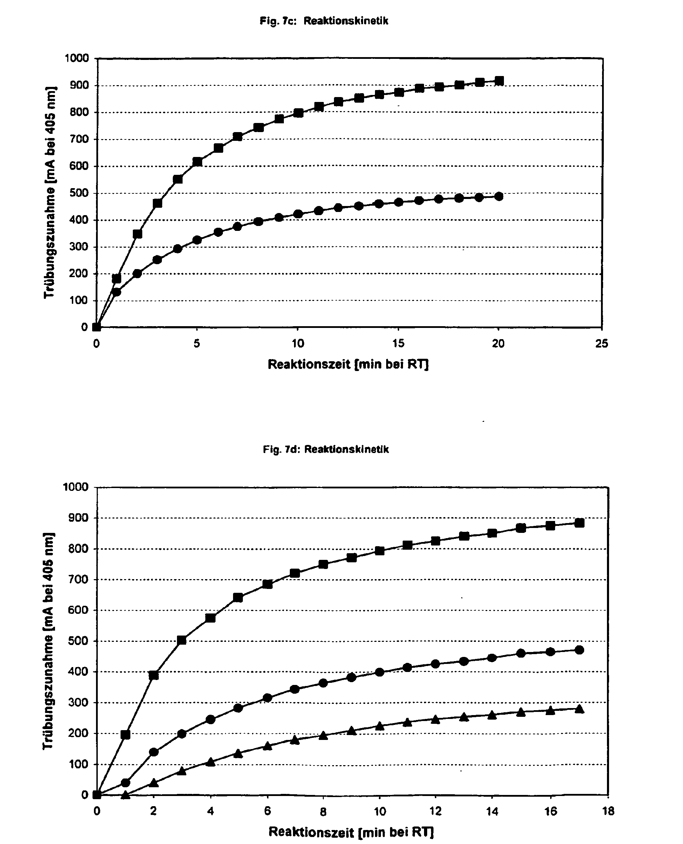

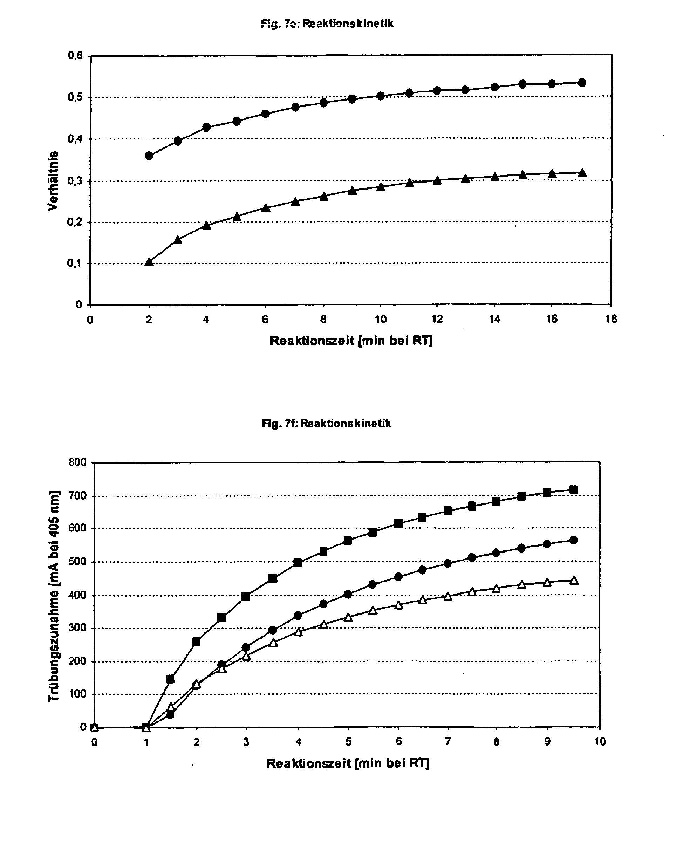

Abb. 7c zeigt die Reaktion des 200 % und des 100 % Pools. Der 100% / 200% Trübungs-Quotient ist stabil bei 0.5 bei einer Reaktionszeit > 4 min bei RT. Abb. 7d zeigt die Reaktion des 256 % Fbgen-Pool, 120 % Plasma and 63 % Plasma. Halbmaximaler Trübungs-Anstieg wird erreicht bei 4 min (RT), bei 5 min Inkubation wird 60 % des maximalen Trübungs-Anstiegs erreicht. Der 120 % / 256 % bzw. der 63 % / 256% Quotient erreicht den korrekten Wert von 0.47 bzw. 0.25 nach ca. 5 min Reaktionszeit; Reaktionszeiten > 8 min (bei RT) ergeben falsch-hohe Quotienten, was den Verlust der Linearität des FIFTA bedeutet (Abb. 7e).

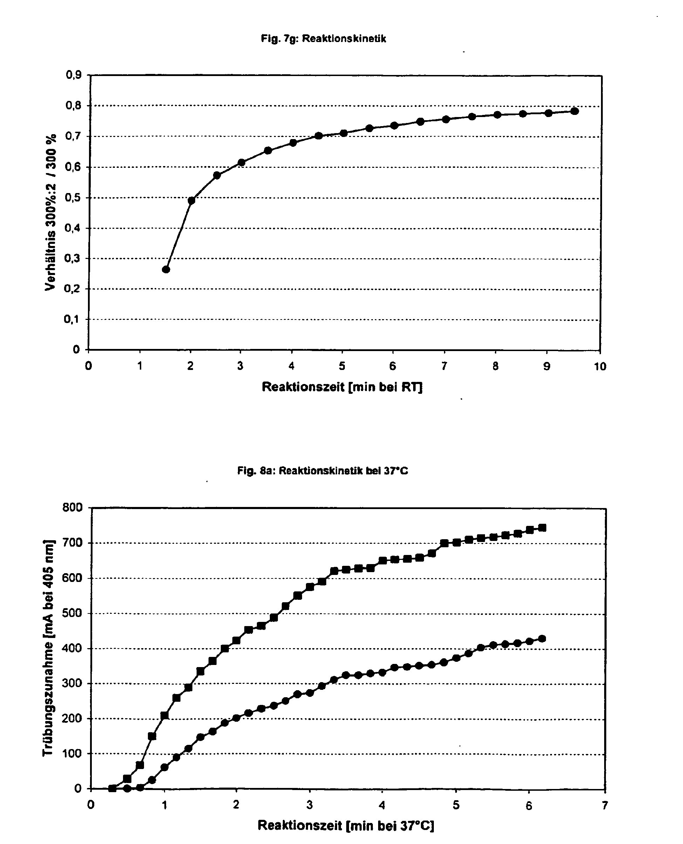

Figs. 7f,g zeigen die Reaktionen des 300 % Fbgen-Pool und des 1:2 verdünnten (mit BSA-PBS) Fbgen-Pool. Der 150% / 300% Trübungsanstiegs-Quotient erreicht den korrekten Wert von 0.5 nur bei ca. 2 min Reaktionszeit bei RT. Nach ≥ 5 min (RT) steigt der Quotient auf Werte ≥ 0.7. Dies bedeutet, daß aktive Fibrinogen-Spiegel > 250 % der Norm im FIFTA linear nur dann gemessen werden können, wenn die Reaktionszeit verkürzt wird (2 min bei RT oder 50 s bei 37°C) oder wenn die Probe zuvor 1:2 verdünnt wird.

### Reaktions-Kinetik bei 37°C

Der FIFTA wurde mit 256 % Fbgen-Pool und mit 256 % Fbgen-Pool, der 1:2 mit 6 % BSA-PBS verdünnt worden war, durchgeführt. Nach Reaktionszeiten von 30-370 Sekunden in 10 Sekunden-Intervallen und nach 20 und 60 min in einem 37°C Wasser-Bad wurde die Trübungs-Zunahme bei 405 nm bestimmt. Der 128% / 256%-Trübungs-Quotient wurde für jede Reaktionszeit berechnet.

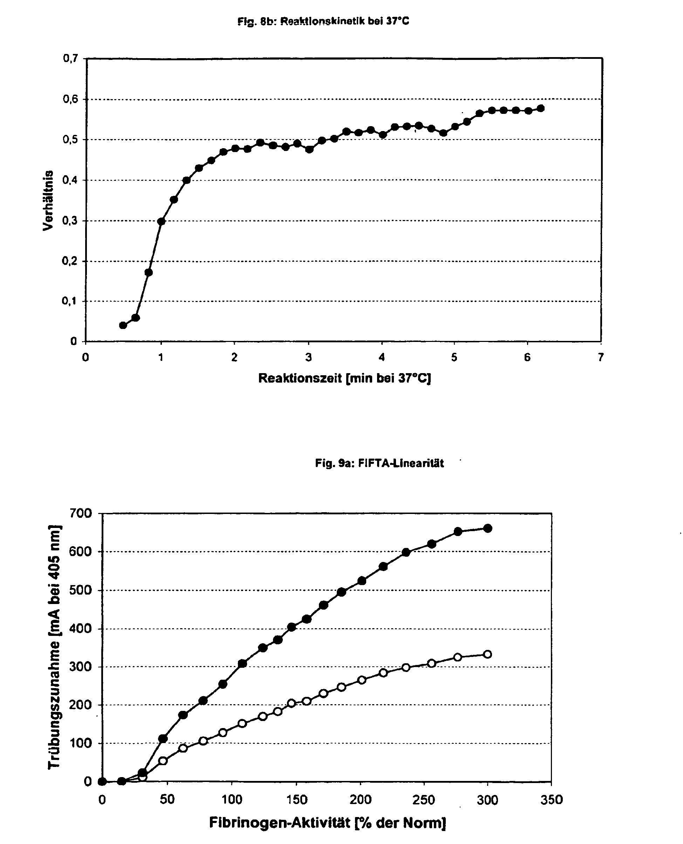

Abb. 8a zeigt den Trübungs-Anstieg in Abhängigkeit von der Reaktionszeit bei 37°C. Der halbmaximale Trübungs-Anstieg tritt ein bei einer Reaktionszeit von 100 Sekunden (37°C) für den 256 % Fbgen-Pool und bei einer Reaktionszeit von 190 Sekunden (37°C) für die 128 % Verdünnung des Fbgen-Pool. Der 128% / 256 % Absorptions-Anstiegs-Quotient erreicht den korrekten Wert von 0.5 bei einer Reaktionszeit von 2-4 min (37°C) (Abb. 8b). Daher ist die optimale FIFTA-Reaktionszeit 5 min bei RT oder 2 min bei 37°C.

### 1.1.5. Absorptions-Wellenlänge für Trübung

Der FIFTA wurde mit P-Pool und mit 256 % Fbgen-Pool durchgeführt; Reaktions-Zeit = 30 min bei RT. Die resultierende Absorption wurde bei 405 nm, 450 nm, 595 nm, und 650 nm durch einen Mikrotiter-Plattenleser bestimmt.

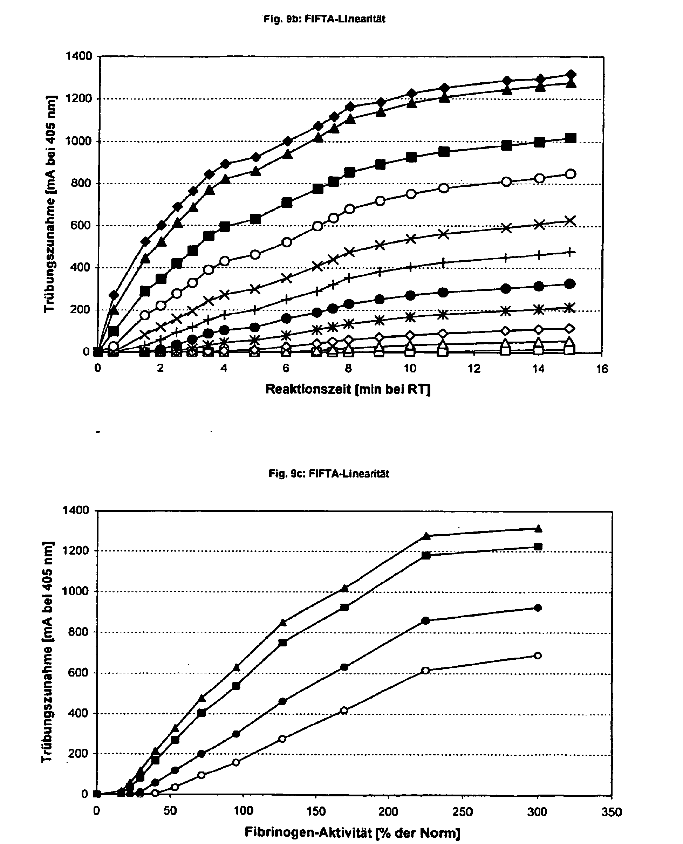

Die Trübung wird am besten gemessen bei 405 nm. Höhere Wellenlängen führen zu einem Abfall an Trübung (Abb. 9).

### 1.2. Kalibration mit gereinigtem Fibrinogen

250 mg lyophilisiertes gereinigtes Human-Fibrinogen (Haemochrom- Enzyme Res. Essen, Germany) wurden rekonstituiert mit 12.5 ml entweder 6 % BSA-PBS oder P-Pool (enthaltend 4.04 g/l Fibrinogen). Die erhaltenen supplementierten Fibrinogen-Proben wurden 1:2, 1:4, 1:8, 1:16, 1:32, 1:64, 1:128 verdünnt mit entweder 6 % BSA-PBS oder P-Pool und der FIFTA wurde durchgeführt. Diese AdditionsKalibration ergab, daß nur 46 % des gereinigten Fibrinogens funktionell aktiv ist; dadurch entstanden Proben mit 0-9.3 g/l aktivem Fibrinogen in BSA-PBS oder Proben mit 4.04-13.3 g/l in P-Pool.

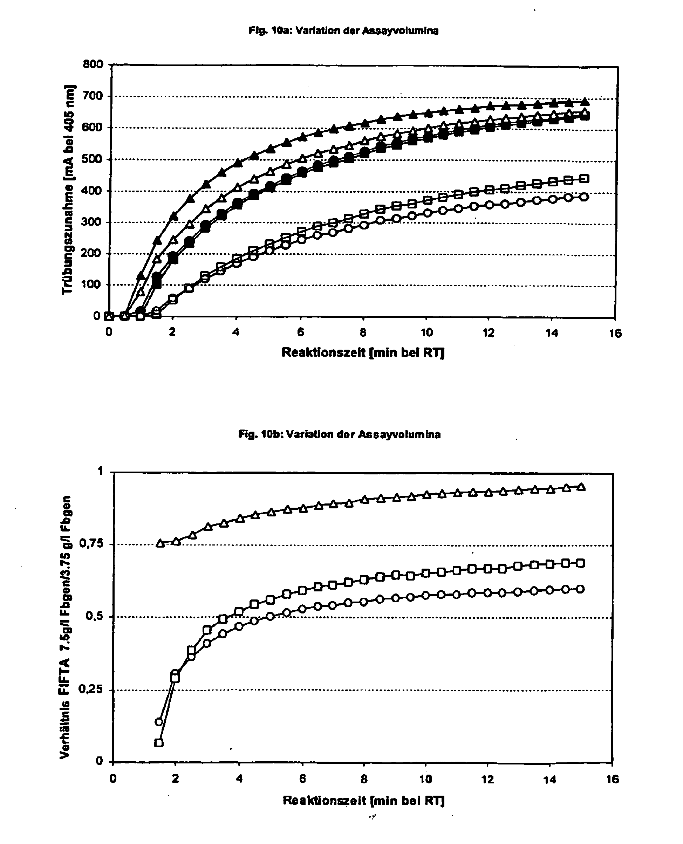

Figs.10a,b zeigen die Reaktionskinetik von gereinigtem Fibrinogen in 6% BSA-PBS im FIFTA in Abhängigkeit von der Reaktionszeit bei RT bzw. in Abhängigkeit von der Konzentration an aktivem Fibrinogen. Bei 15 min (RT) ist ca. 75 % der maximalen Trübung erreicht. Es ergibt sich kein weiterer Anstieg, wenn die Reaktionszeit von 48 min auf 158 min oder 18 h verlängert wird.

Figs.10c,d zeigen die Reaktionskinetik von P-Pool, der mit gereinigtem Fibrinogen supplementiert wurde, in Abhängigkeit von der Reaktionszeit bei RT oder in Abhängigkeit von der resultierenden aktiven Fibrinogen-Konzentration. Bei 15 min (RT) sind ca. 80 % der maximalen Trübung erreicht. Eine Verlängerung der Reaktionszeit von 48 min zu 158 min oder 18h resultiert in einen 5-10 % igen Abfall der finalen Trübung. Abb.10e zeigt den Vergleich zwischen Fibrinogen in BSA und Fibrinogen in Plasma.

### 1.3. Linearität

256 % Fbgen-Pool und 256 % Fbgen-Pool supplementiert mit gereinigtem aktivem Fibrinogen zu 277 % oder zu 300 % der Norm wurden im FIFTA und in einer FIFTA-Version mit halbierten Test-Volumina analysiert.
225 % Fbgen-Pool wurde mit aktivem Fibrinogen zu 300 % Fbgen-Pool supplementiert und mit physiol. NaCl verdünnt. Diese Plasma-Proben mit 0-300 % Fibrinogen wurden im FIFTA bestimmt.

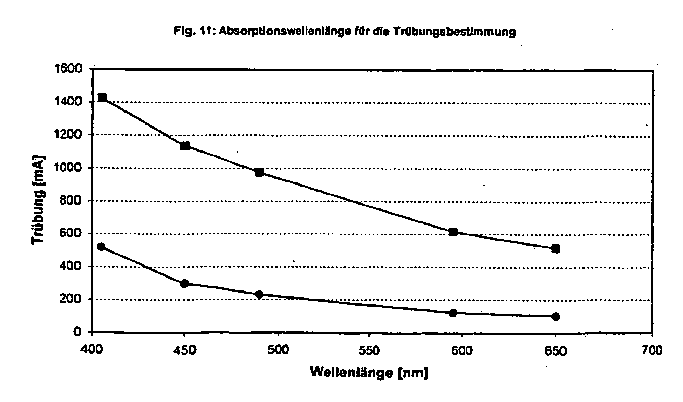

Der FIFTA ist nahezu linear im Fibrinogen-Aktivitäts-Bereich von etwa 25 - 250 % des normalen Mittelwertes (MV). 100 % der Norm Fibrinogen-Plasma resultiert in ein ΔA von ca. 250 mA (Figs. 11a-c). Der FIFTA kann auch mit 25 µl Plasma und 50 µl Reagenz durchgeführt werden, woraus eine ΔA von ca. 125 mA in der Mikrotiterplatte resultiert (Abb. 11a).

### Variation der Test-Volumina

256 % Fbgen-Pool und der 256 % Fbgen-Pool, der 1:2 mit 6 % BSA-PBS verdünnt worden war, wurden im FIFTA (50 µl Probe + 100 µl Thrombin-Reagenz) eingesetzt. 67 µl 256 % Fbgen-Pool oder 1:2 verdünnter Fbgen-Pool wurden mit 83 µl Thrombin-Reagenz inkubiert. 100 µl 256 % Fbgen-Pool oder 1:2 verdünnter Fbgen-Pool wurden mit 50 µl Thrombin-Reagenz inkubiert, welches 600 mlU/ml Thrombin enthielt; die Absorptions - Quotienten 128% / 256% wurden berechnet.

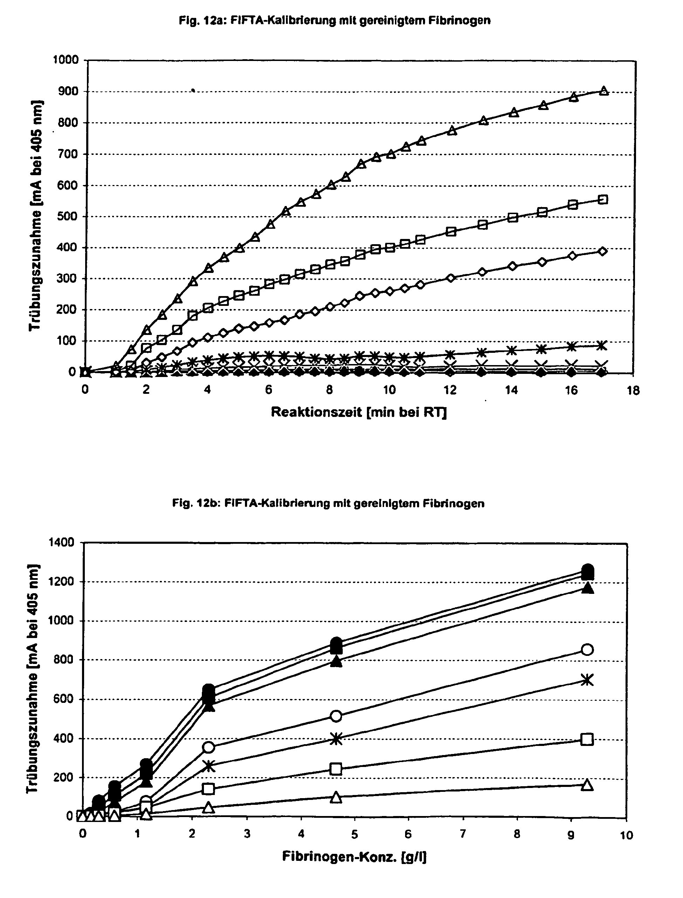

Figs. 12a,b zeigen, daß nur die 50 µl + 100 µl FIFTA Version - im Gegensatz zu einer 67 µl + 83 µl oder zu einer 100 µl + 50 µl Test-Version - in einen korrekten 128% / 256% Absorptions-Anstiegs-Quotienten von 0.5 bei 5 min RT resultiert.

### 1.4. Sensitivität

Zur Ermittlung der unteren Nachweisgrenze des FIFTA, wurden 6% BSA-PBS oder gepooltes normales Serum (0 % der Norm Fibrinogen) 10fach analysiert und die Standard-Abweichung (SD) wurde bestimmt. Die untere Nachweisgrenze wurde definiert als das 3fache der SD des 0-Wertes.

Die untere Nachweisgrenze des FIFTA ist 25 % der Norm (0.7 g/l), wenn die Reaktionszeit 5 min bei RT beträgt; Verlängerung der Reaktionszeit auf 15 min (RT) führt zu einer auf 7 % der Norm (0.2 g/l) verringerten unteren Nachweisgrenze.

### 1.5. Präzision

Der 144 % P-Pool wurde 10fach intra-assay und inter-assay analysiert. Die Mittelwerte wurden bestimmt und die Variationskoeffizienten wurden berechnet.

Für den 144 % Standard (P-Pool) wurde ein intra-assay VK von 1.8 % und ein inter-assay VK von 3.4 % bestimmt.

### 1.6. Normal-Bereich

n=39 normale Zitrat-Plasmen wurden im FIFTA analysiert, um Mittelwert (MV) und Standard-Abweichung (SD) zu erhalten, die somit den Normalbereich (MV ± 1 SD) der Fibrinogen-Funktion im Plasma ergeben. Der Test wurde kalibriert gegen gepoolte Normal-Plasmen und gegen kommerziell erhältliches tiefgefrorenes 100 % Normal-Plasma, welches 2.8 g/l aktives Fibrinogen enthielt (Haemochrom, Essen, Germany).

Der Normal-Bereich des FIFTA ist 100 % ± 20 % (MV ± 1 SD); 100 % = 2.8 g/l.

### 1.7. Korrelation mit Clauss-Methode

Bei n=334 unselektierten Patienten-Plasmen (≤ 6 h alt zum Zeitpunkt des üblichen Verwerfens der Blutproben) wurde der FIFTA durchgeführt, und die Ergebnisse wurden verglichen mit den in der Routine gemessenen Fibrinogen-Konzentrationen (modifizierte Clauss-Methode; Multifibren U®, Behring Coagulation Timer, DadeBehring, Marburg, Germany: 100 µl Probe + 200 µl 50 IU/ml bovines Thrombin, 0.15 g/l Gly-Pro-Arg-Pro-Ala-amid, 1.5 g/l CaCl₂, 15 µg/ml Polybrene®, 0.8 g/l Polyethylenglycol 6000, 6.4 g/l NaCl, 50 mM Tris, 1 % bovines Albumin; Bestimmung der Gerinnungszeit).

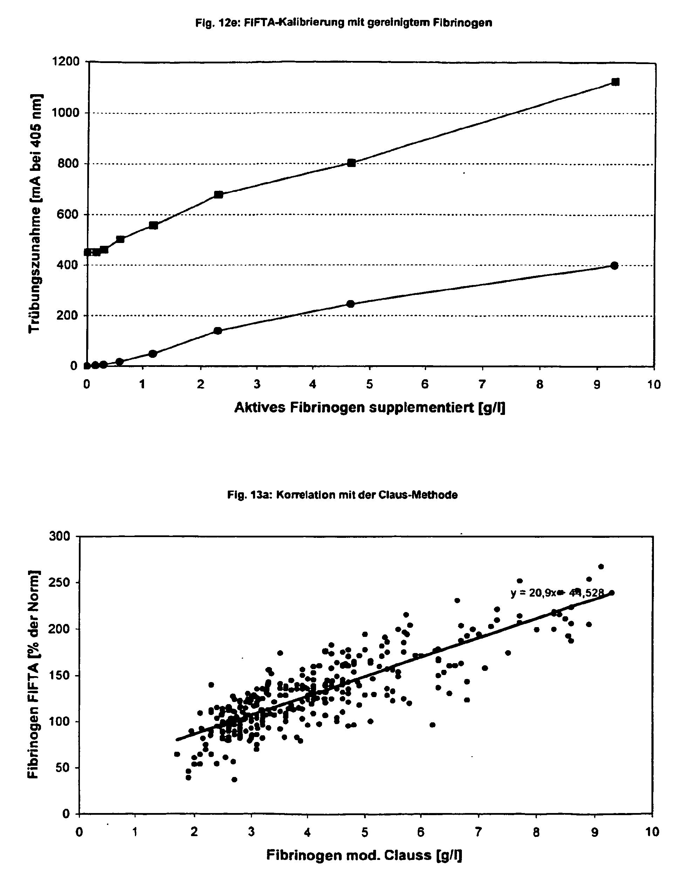

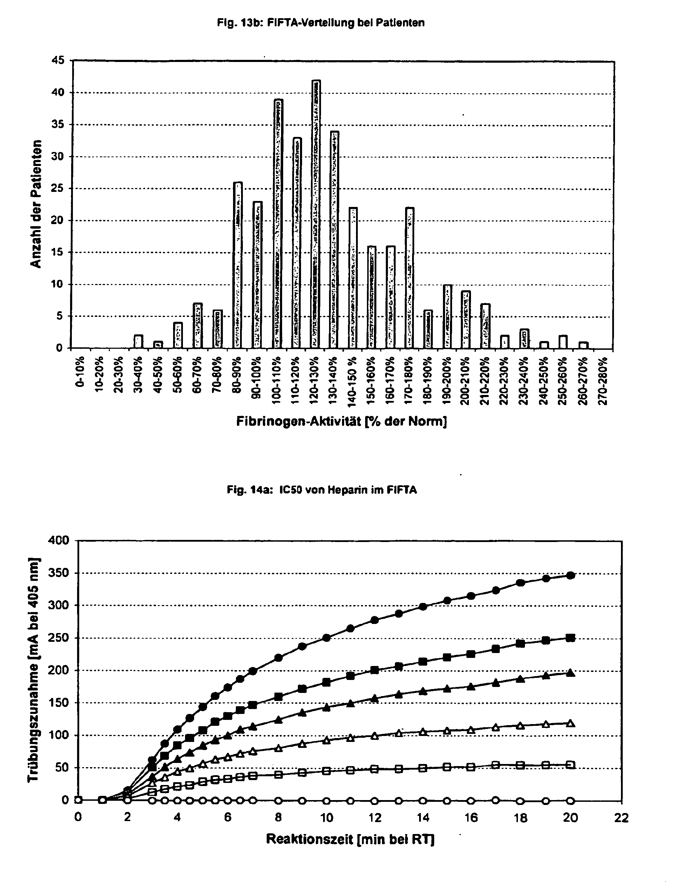

Bei n=334 unselektionierten Patienten-Plasmen wurde der FIFTA (MV = 132 %, SD = 41%) durchgeführt und verglichen mit der modifizierten Clauss-Methode (MV = 4.18 g/l, SD = 1.65 g/l); Korrelationsfaktor r= 0.843; y = 20.9 x + 44.528 (Abb. 13a). Die FIFTA Ergebnisse der Patienten verteilen sich mit einer normalen Gauss-Verteilung (Abb. 13b).

### 1.8. Inhibition der Fibrin-Bildung durch Heparin

### 1.8.1. Inhibitions-Konzentration 50% von Heparin gegen Thrombin

50 µl 100 % Pool wurden zu 10 µl 0-160 IU/ml Heparin zugesetzt. 100 µl 200 mlU/ml Thrombin in 6 % BSA-PBS (ohne Polybrene®) wurden zugefügt und der Absorptions-Anstieg bei 405 nm wurde gemessen.

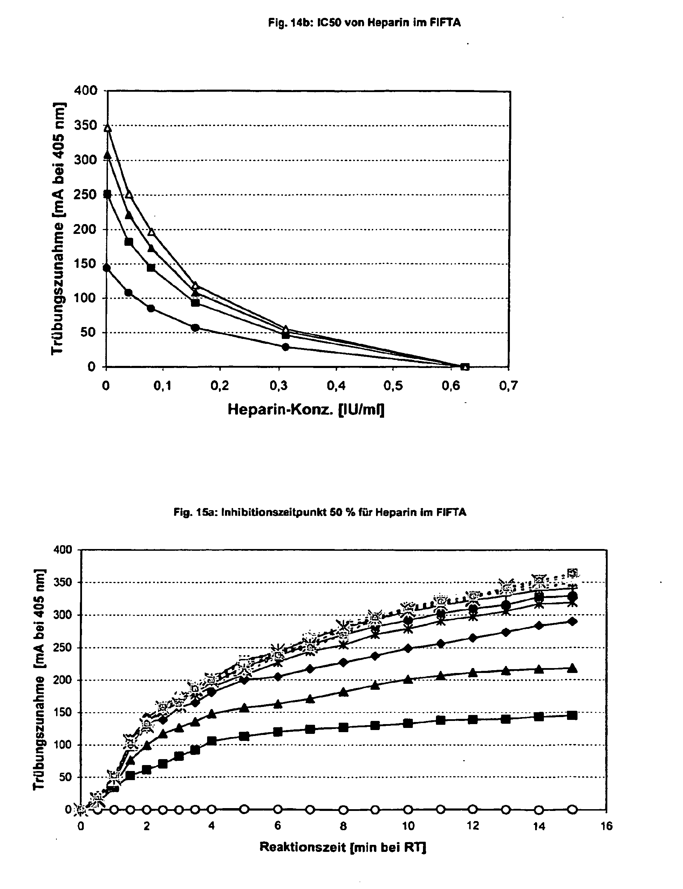

Heparin inhibiert dosisabhängig den Trübungs-Anstieg (Abb. 14a). Heparin-Konzentrationen ≥ 0.63 IU/ml inhibieren Thrombin im beschriebenen Testsystem vollständig. Die Inhibitory Concentration 50 % (IC50) ist ca. 0.1 IU/ml Heparin (Abb. 14b).

### 1.8.2. Inhibitorischer Zeitpunkt 50% der 10 IU/ml Heparin/Thrombin-Wechselwirkung

50 µl P-Pool wurden mit 100 µl 200 mlU/ml Thrombin in 6 % BSA-PBS (ohne Polybrene®) inkubiert. Bei 0 min (vor Thrombin-Zusatz) und 0.5-11 min Reaktionszeit (bei RT) wurden 10 µl 160 IU/ml Heparin in 6% BSA-PBS zugesetzt (Endkonzentration an Heparin in Plasma + Thrombin-Reagenz = 10 IU/ml; diese Heparin-Konzentration erhöhte die Absorption um 20 mA; alle Testergebnisse wurden korrigiert um diese Heparin-induzierte Trübungs-Zunahme). Die Trübungs-Zunahme bei 405 nm wurde sofort nach jedem einzelnen Heparinzusatz-Schritt und als eine Funktion der Inkubationszeit bestimmt.
Zur Bestimmung des Heparinzusatz-Zeitpunktes (bei RT), der nötig ist, um den Trübungsanstieg nach 5 min, 10 min, 15 min oder 20 min um 50 % (Inhibitions-Zeitpunkt 50 %) zu reduzieren, wurden 50 µl P-Pool inkubiert mit 100 µl 200 mlU/ml Thrombin in 6 % BSA-PBS (ohne Polybrene®). Nach 0 Sekunden (vor Thrombin-Zusatz) und 10-140 Sekunden (in 10 Sekunden-Intervallen) wurden 10 µl 160 IU/ml Heparin in 6 % BSA-PBS zugefügt. Die Trübungs-Zunahme bei 405 nm wurde als eine Funktion der Inkubationszeit (3-20 min) bestimmt.

Heparin kann die Fibrinbildung nur innerhalb der ersten 2 min (RT) inhibieren (Figs.15a-c). Trotz des Zusatzes von 10 IU/ml finaler Heparin-Konzentration bei 30 s Inkubationszeit (RT) wird noch ca. 50 % des Fibrinogens in Fibrin umgewandelt; d.h. 133 mIU/ml finales Thrombin wandeln ca. 50 % des plasmatischen Fibrinogens innerhalb ca. 30 Sekunden um. Die 15 min Fibrin Trübung ist > 80 % der Maximaltrübung wie zuvor gesehen. Jedoch ist der Trübungs-Anstieg in Anwesenheit von 10 lU/ml Heparin innerhalb der ersten 5 min Reaktionszeit höher als der in Abwesenheit von Heparin. Der Zeitpunkt des Heparinzusatzes, der zu einem 50 %igen Abfall von ΔA führt (ITP50) bei 5-20 min Inkubationszeit (RT) beträgt ca. 30 s.

### 1.9. Inhibition der Fibrin-Bildung durch Arginin

a) 10 ml FIFTA-Reagenz wurde zu 5 ml P-Pool zugesetzt, die Mischung wurde in eine Multipette aufgesogen, und 100 µl dieser Mischung wurden sofort in eine Mikrotiterplatte pipettiert, die mit 200 µl 0-1500 mM Arginin, pH 8.7, je Vertiefung vorbereitet war, woraus finale Arginin-Konzentrationen von 0, 16, 31, 62.5, 125, 250, 500, 1000 mM resultierten. Die Trübungszunahme bei 405 nm wurde als Funktion der Inkubationszeit gemessen.
b) 10 ml FIFTA-Reagenz wurden zu 5 ml P-Pool gegeben, die Mischung wurde in eine Multipette aufgesogen, und nach 0 - 4 min (bei RT) wurden 100 µl davon in eine Mikrotiterplatte pipettiert, die mit 200 µl 0-1500 mM Arginin, pH 8.7, je Vertiefung vorbereitet worden war. Die Trübung bei 405 nm wurde sofort bestimmt nach jedem Zusatz-Schritt der Reaktionsmischung mit dem Arginin der Platte. Die basale Kontroll-Absorption wurde bestimmt durch Zusatz von FIFTA-Reagenz ohne Thrombin zu Plasma und anschließendes Pipettieren der Mischung zu 0-1500 mM Arginin. Die Absorptions-Zunahme wurde berechnet durch Subtraktion der Basal-Absorption von der Absorption, die durch Thrombin verursacht wird (Zusatz von 100 µl FIFTA-Reagenz ohne Thrombin ergibt etwa einen Absorptions-Anstieg von 100 mA verglichen mit der Absorption von 50 µl Probe).
c) 50 µl P-Pool wurden mit 100 µl FIFTA-Reagenz inkubiert. Nach 0-30 min (bei RT) wurden 0-70 µl 1.5 M Arginin, pH 8.7, zugesetzt und nach dem letzten Zeitpunkt (30 min) wurde die finale Trübungs-Zunahme bei 405 nm bestimmt.

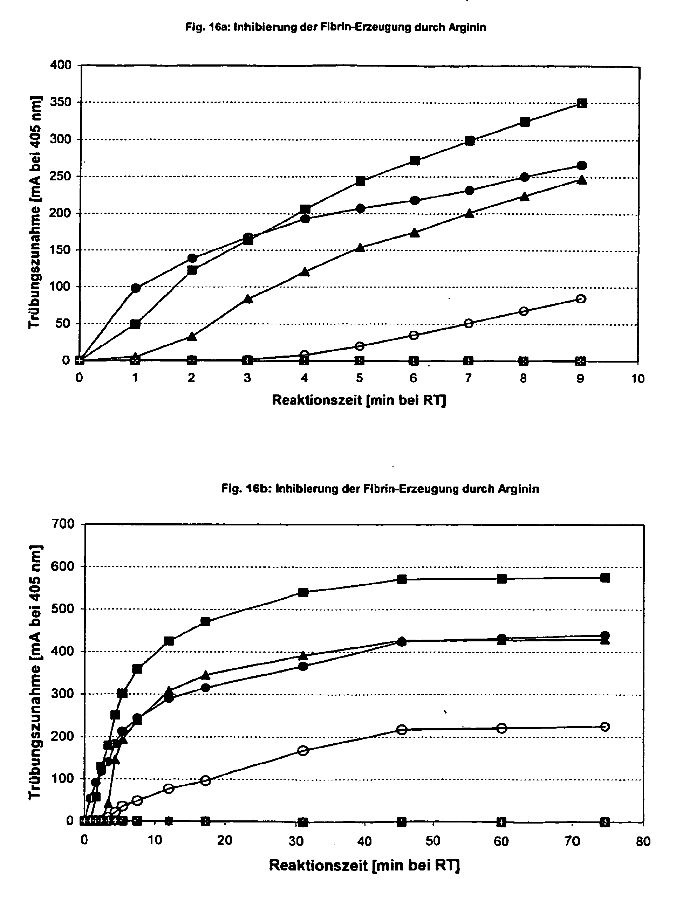

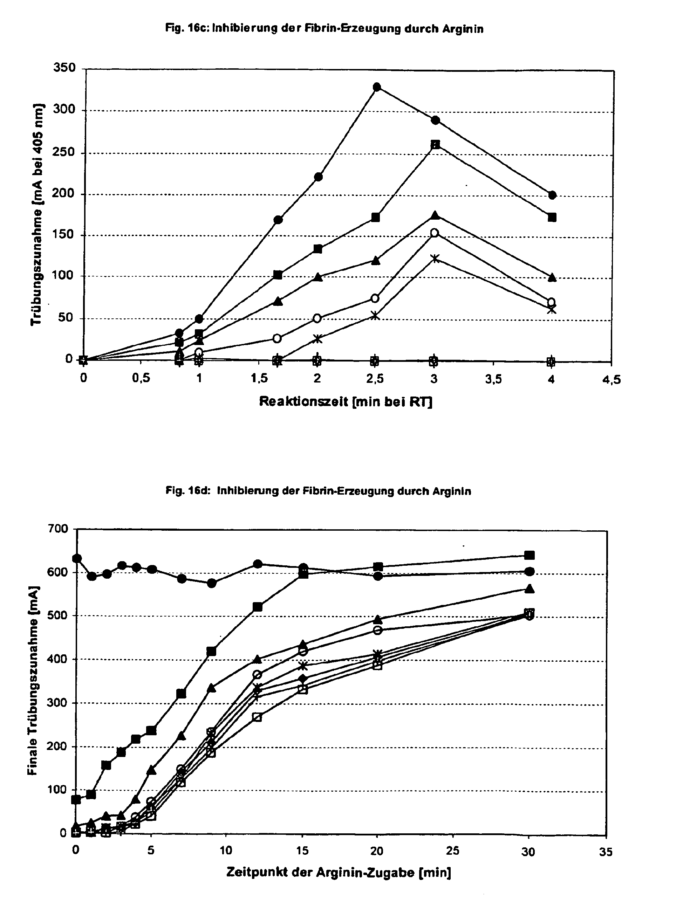

Eine finale Arginin-Konzentration ≥ 125 mM inhibiert vollständig den Trübungs-Anstieg, wenn das Arginin seit Beginn der Thrombinwirkung agiert (Figs. 16a-c). Halbmaximaler Trübungs-Anstieg tritt auf bei 62.5 mM Arginin. 16 mM Arginin erhöhen den Trübungs-Anstieg um ca. 20 %. Finale Arginin-Konzentrationen ≥ 250 mM inhibieren vollständig den Trübungs-Anstieg, wenn Arginin innerhalb der ersten 4 min (RT) der Thrombin/Fibrinogen Interaktion zugesetzt wird. 125 mM Arginin können ΔA nur dann inhibieren, wenn es innerhalb der ersten 100 s (RT) der Thrombin/Fibrinogen Interaktion zugegeben wird. Zusatz von Arginin bei 2.5 min der Thrombin/Fibrinogen Reaktionszeit resultiert in einen Abfall von ΔA, der von der Arginin-Konzentration abhängt. Da in der Multipipette ein großes Gerinnsel entsteht, sind die Messwerte bei > 3 min (RT) nicht verlässlich. Abb. 16d zeigt, daß finale Arginin-Konzentrationen ≥ 250 mM ΔA nur dann inhibieren können, wenn sie innerhalb der ersten 3 min (RT) der Thrombin/Fibrinogen Interaktion zugesetzt werden. Halbmaximale Trübungs-Anstiege bei dem 7 min oder dem 15 min Reaktionszeitpunkt resultieren durch 94 mM oder 477 mM Arginin.

### 1.10. Fibrin-Auflösung durch Arginin

50 µl P-Pool wurden mit 100 µl FIFTA-Reagenz inkubiert. Nach 30 min (bei RT) wurden 10 µl oder 70 µl 1.5 M Arginin, pH 8.7 (finale Arginin-Konz. 94 mM oder 477 mM) zugesetzt und der finale Trübungs-Anstieg bei 405 nm wurde nach 0-132 min (bei RT) bestimmt.

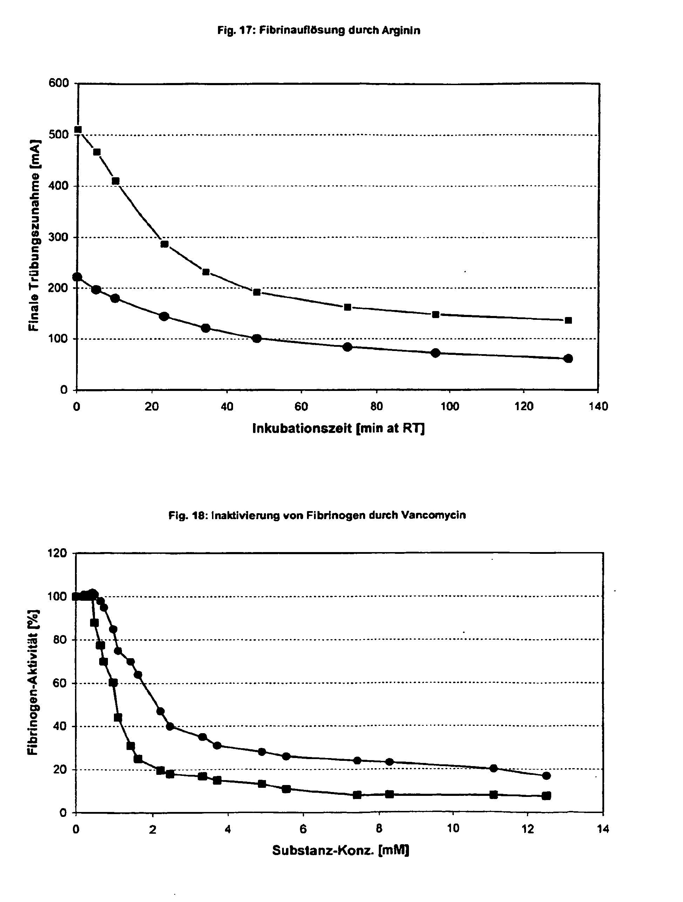

Finale Arginin-Konzentrationen von 477 mM zum Zeitpunkt 12 min oder 30 min der Thrombin/Fibrinogen Interaktion erniedrigt ΔA in Abhängigkeit von der Inkubationszeit: halbmaximaler Abfall tritt auf nach weiteren 30 min (RT) (Abb. 17). Verlängerung der Arginin-Inkubation auf > 100 min zeigt, daß ca. 30 % der einmal generierten maximalen ΔA nicht mehr aufgehellt werden können.

### 2. FIATA

### 2.1. Inaktivierung von Fibrinogen durch Vancomycin

200 µl des 256 % der Norm aktives Fibrinogen in P-Pool wurden inkubiert mit 25 µl 0 - 100 mM Vancomycin oder 0 - 100 mM Chloramin-T® in aqua dest. für 15 min (37°C). 50 µl Proben dieser Reaktionsgemische wurden entnommen und mit 100 µl 200 mlU/ml bovinem Thrombin, 400 µg/ml Polybrene®, 6 % BSA-PBS inkubiert. Die Ergebnisse wurden standardisiert durch gleichzeitige Analyse von 143 % der Norm P-Pool.
Vancomycin inaktiviert konzentrationsabhängig plasmatisches Fibrinogen: ca. 1 mM Vancomycin inaktiviert 50 % des Fibrinogens in menschlichem Plasma (Inhibitory Concentration 50% (IC50) = 1 mM). Zum Vergleich, die IC50 von Chloramin-T® gegen plasmatisches Fibrinogen ist ca. 2 mM (Abb. 18).

### 2.2. Unterdrücken Antioxidantien die Reaktion zwischen Vancomycin und plasmatischem Fibrinogen ?

750 µl 256 % der Norm aktives Fibrinogen in P-Pool wurde supplementiert mit 250 µl a) ¹O₂ Inhibitoren Methionin (200 mM) oder Natriumascorbat (200 mM), b) ·OH Radikal-Inhibitor Mannit (200 mM), oder c) H₂O. 25 µl Proben davon wurden mit 25 µl 0 - 2.5 mM Vancomycin oder 0 - 5 mM Chloramin-T® in PBS für 15 min bei 37°C inkubiert. Dann wurden 100 µl 200 mIU/ml Thrombin, 400 µg/ml Polybren® in 6 % BSA-PBS zugesetzt und die Mischung wurde für 3, 5, 9, and 17 min (RT) inkubiert. Die entsprechenden Fibrinogen-Aktivitäten am 5 min Inkubations-Zeitpunkt wurden berechnet gegen 100 % normalen Kontroll-Pool (0 mM Vancomycin bzw. Chloramin-T®).

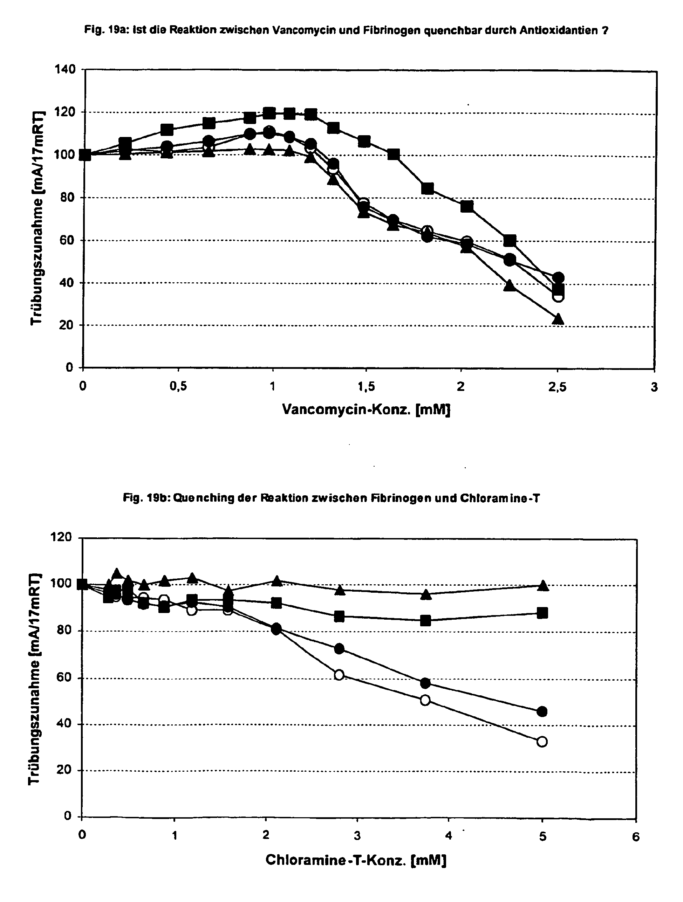

Die Vancomycin-Interaktion mit Fibrinogen in Plasma kann nicht inhibiert werden durch Methionin, Askorbat, oder Mannit (Abb. 19a). Die 100 % Kontrolle von Methionin oder H₂O hatte einen Trübungs-Anstieg/5 min (RT) von 386 ± 11 mA, die von Ascorbat war 360 ± 14 mA und die von Mannit war 435 ± 12 mA. Das Kontroll-Experiment mit Chloramin-T® anstelle von Vancomycin zeigte, daß die Chloramin-Reaktion mit plasmatischem Fibrinogen inhibiert werden kann durch Methionin oder Ascorbat (Abb. 19b). Daher scheinen beim Reaktionsmechanismus der Vancomycin-Interaktion mit Fibrinogen - im Gegensatz zu der zwischen Chloraminen und Fibrinogen - nicht reaktive Sauerstoff-Spezies vom Typ des Singulett-Sauerstoff beteiligt zu sein.

### 2.3. FIATA Optimierung

### Vancomycin-Konzentration

50 µl P-Pool oder 1:2 oder 1:4 verdünnter (in 6 % BSA-PBS) P-Pool wurden mit 50 µl 0 - 6.8 mM Vancomycin in PBS inkubiert. ΔA bei 405 nm wurde nach 1 min (RT) bestimmt. 25 µl Fbgen-Pool (300 % der Norm; 8.4 g/l Fibrinogen-Antigen) wurden mit 125 µl 0.55 mM, 1.1 mM, oder 2.2 mM Vancomycin in PBS für 1 min (RT) inkubiert. Dann wurde ΔA bei 405 nm bestimmt.

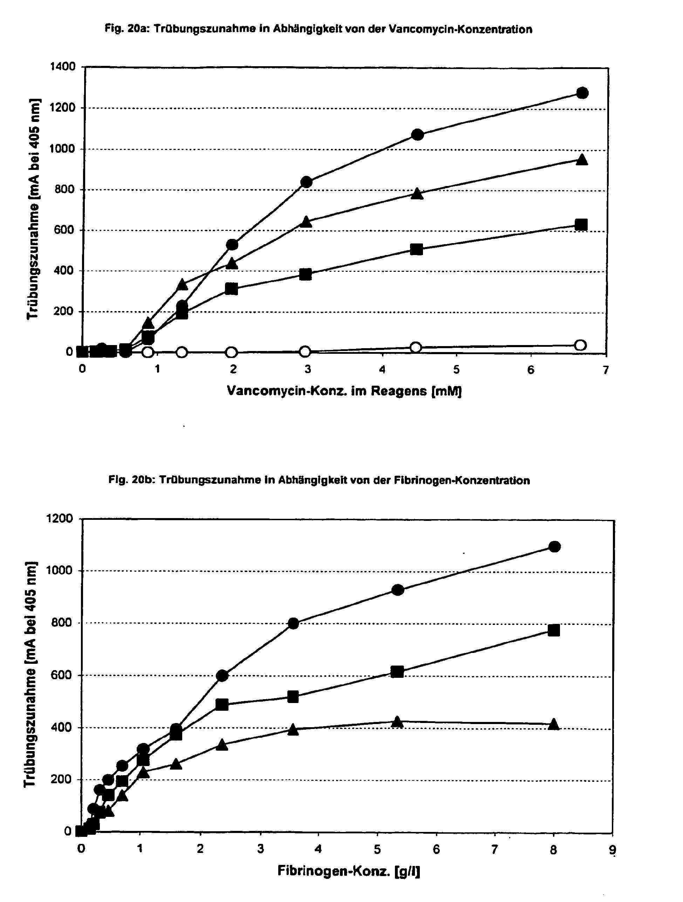

Steigende Konzentrationen an Vancomycin resultieren in zunehmender Trübung der Reaktionsmischung. Dieser proportionale Anstieg tritt auf bei P-Pool und bei 1:2 oder 1:4 verdünntem P-Pool. Die Eigentrübung von 4.4 mM Vancomycin in PBS (0 % Fibrinogen-Standard in BSA) ist < 50 mA bei 405 nm (Abb. 20a). Dieser Trübungs-Anstieg tritt innerhalb von Sekunden ein und ist für mindestens 10 min stabil.

Abb. 20b zeigt, daß 68.8 µmol Vancomycin zugesetzt zu 25 µl 300 % Fbgen-Pool resultieren in eine Sättigung von ΔA bei ca. 4 g/l Fibrinogen. Bei 137.5-27.5 µmol Vancomycin tritt diese Sättigung bei 6-8 g/l Fibrinogen ein. Die optimale Vancomycin-Konzentration im Vancomycin-Reagenz des FIATA ist 3 - 6 mM.

### 2.4. FIATA Eichung mit gereinigtem Fibrinogen

250 mg lyophilisiertes gereinigtes Fibrinogen wurden rekonstituiert mit 12.5 ml 6 % BSA-PBS oder P-Pool. Die so erhaltenen supplementierten Fibrinogen-Proben wurden 1:2, 1:4, 1:8, 1:16, 1:32, 1:64, 1:128 verdünnt entweder mit BSA-PBS oder P-Pool, wodurch Proben erhalten werden mit 0 - 20 g/l Fibrinogen-Antigen in BSA-PBS oder solche mit 4 - 24 g/l Fibrinogen-Antigen in P-Pool.

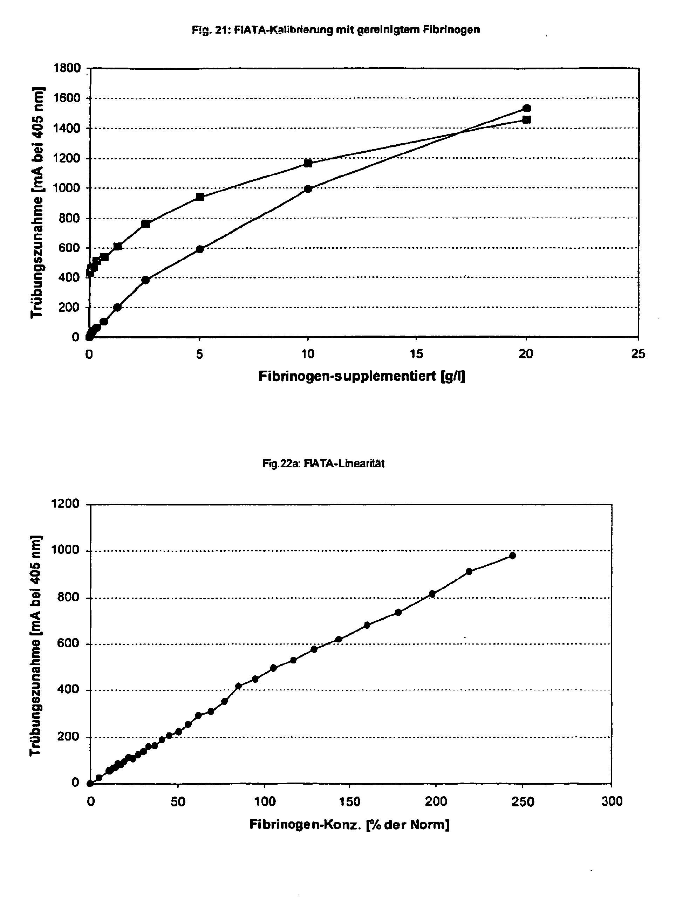

Abb. 21 zeigt die Reaktion von gereinigtem Fibrinogen im FIATA. Eine Fibrinogen-Konzentration von 10 g/l resultiert in ein ΔA von ca. 1000 mA, sowohl für Fibrinogen in 6 % Albumin als auch für Fibrinogen in P-Pool. Der FIATA ist linear bis ca. 8 g/l (286 % der Norm) Fibrinogen in der Probe, was eine ΔA von ca. 800 mA ergibt. 2.5. FIATA-Linearität
0-55 µl 300 % der Norm (8.4 g/l) Fibrinogen P-Pool wurde inkubiert mit a) 50 µl PBS und 50 µl 4.4 mM Vancomycin in PBS oder b) 100 µl PBS und 100 µl 4.4 mM Vancomycin in PBS. 0-35 µl 300 % Fbgen-Pool wurden inkubiert mit 35-0 µl PBS (zum Volumenausgleich) und a) 50 µl PBS und 50 µl 4.4 mM Vancomycin in PBS oder b) 100 µl PBS und 100 µl 4.4 mM Vancomycin in PBS.

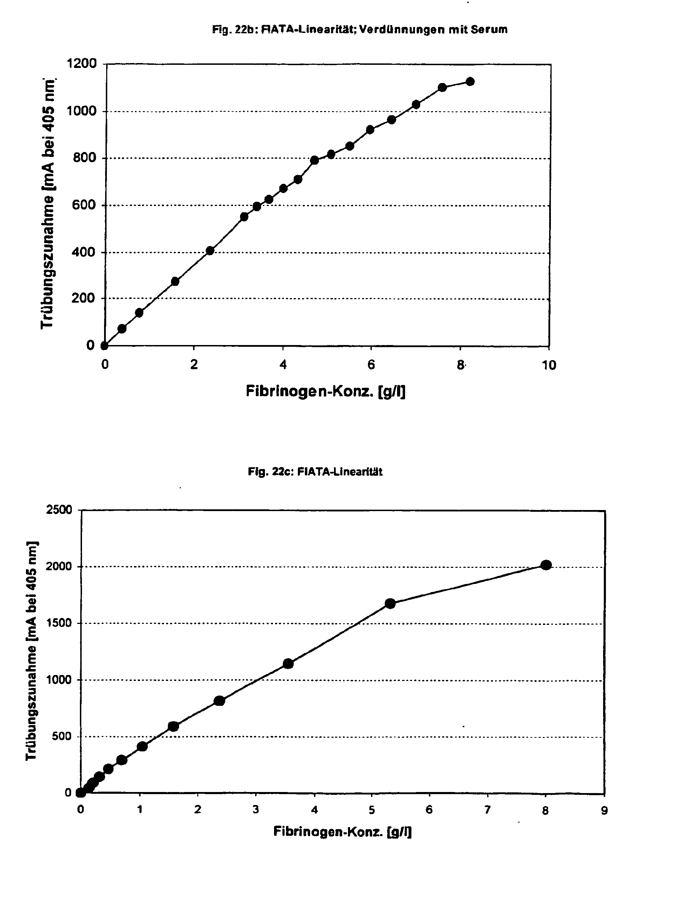

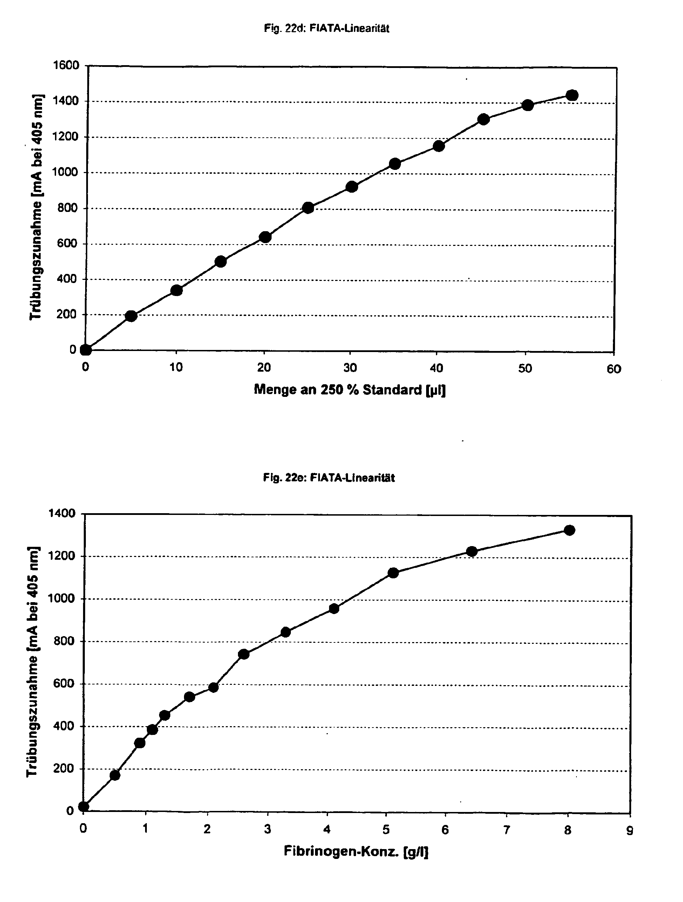

Der FIATA ist nahezu linear bis zu einer Fibrinogen-Konzentration von ca. 150 % der Norm (4.2 g/l) (Abb. 22a,b). Eine Verdopplung der Test-Volumina ergibt eine Verdopplung der Absorption bei 405 nm (Abb. 22c). Da die Eigentrübung von 50 µl Plasma > 500 mA sein könnte (hypertryglyceridämische oder hyperbilirubinämische Patienten), ist die 25 µl/50 µl/ 50 µl FIATA-Version die bevorzugte Version (Abb. 22d). Ein modifizierter FIATA mit 50 µl Probe und 50 µl Reagenz ist nur bis ca. 100 % der Norm Fibrinogen linear (Abb. 22e).

### 2.6. FIATA-Sensitivität

Zur Bestimmung der unteren Nachweisgrenze des FIATA wurde gepooltes normales Serum (0 % der Norm antigenisches Fibrinogen) 10fach analysiert, und die Standardabweichung (SD) wurde bestimmt. Die untere Nachweisgrenze wurde definiert als die 3fache SD des 0-Wertes.

Die untere Nachweisgrenze des FIATA - definiert als die 3fache SD des 0 % Wertes - ist 4 % der Norm (0.1 g/l).

### 2.7. FIATA-Präzision

Der P-Pool (143 % der Norm = 4.0 g/l Fibrinogen) wurde 10fach intra-assay und inter-assay analysiert. Die Mittelwerte wurden bestimmt und die Variationskoeffizienten (VK) wurden berechnet.
Für den 143 % Standard (P-Pool) wurde ein intra-assay VK von 1.9 % und ein inter-assay VK von 3.5 % bestimmt.

### 2.8. FIATA-Normal-Bereich

n=39 normale Citratplasma-Proben wurden im FIATA analysiert, um Mittelwert und Standardabweichung (SD) zu ermitteln, woraus der Normalbereich antigenischen Fibrinogens (0 ± 1 SD) in Plasma resultiert. Der FIATA wurde kalibriert mit gepoolten normalen Studentenplasmen und mit kommerziellem 100 % normalem gepoolten Plasma mit 2.8 g/l Fibrinogen, tiefgefroren ohne Lyophilisationszusätze (Haemochrom, Essen, Germany).
Der Normalbereich des FIATA ist 100 % ± 20% (MV ± 1 SD), 100 % der Norm = 2.8 g/l.

### 2.9. FIATA Korrelation mit funktionellen Fibrinogen-Bestimmungen

Bei n=321 unselektionierten Patienten-Plasmen (≤ 6 h alt; zum Zeitpunkt des üblichen Verwerfens der Proben) wurde der FIATA durchgeführt und verglichen mit den routinemäßig gemessenen Fibrinogen-Konzentrationen (modifizierte Clauss-Methode (Multifibren U®) auf einem Behring Coagulation Timer, DadeBehring, Marburg, Germany). Bei n=344 unselektionierten Patienten-Plasmen wurde der FIATA verglichen mit einem neuen turbidimetrischen funktionellen Fibrinogen-Test, dem FIFTA: 50 µl Plasma + 100 µl 300 mlU/ml Thrombin, 6 % Albumin-PBS, 400 µg/ml Polybrene®; Bestimmung von ΔA/5 min (RT).

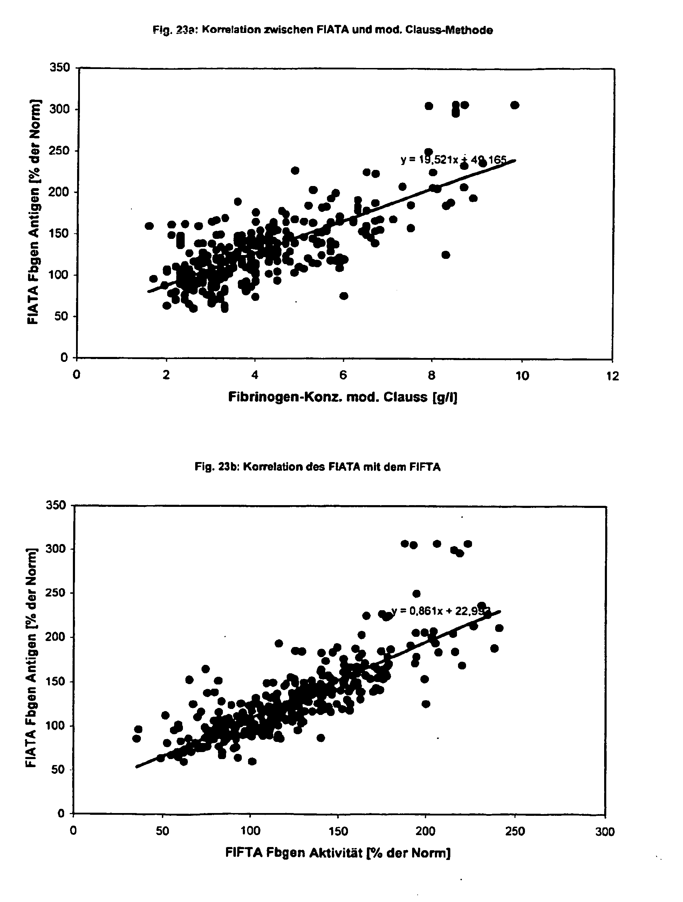

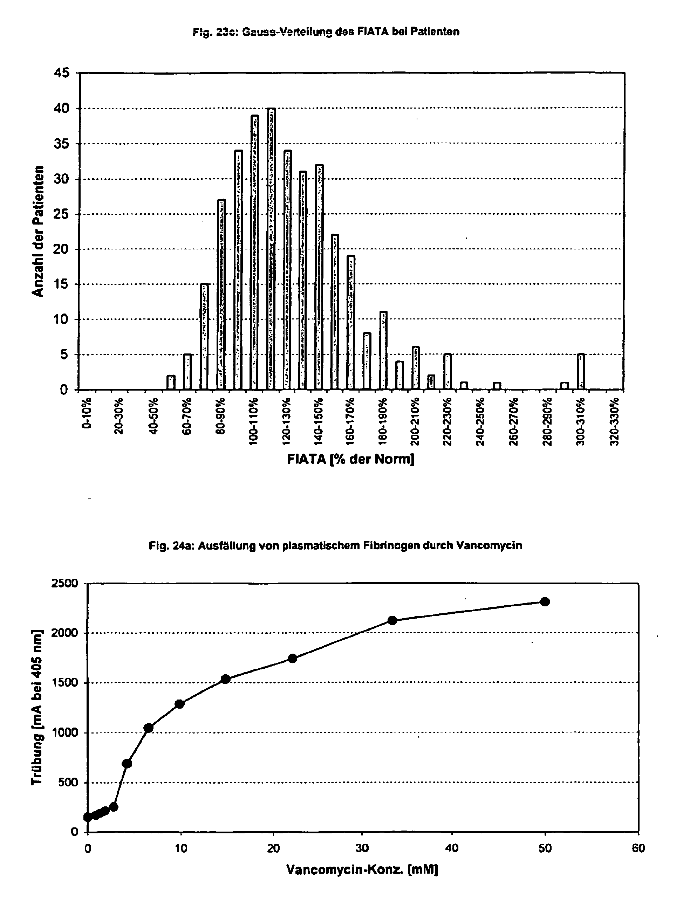

Bei n=321 unselektionierten Patientenplasmen korrelierte der FIATA (MV = 130±52%) mit einer modifizierten Clauss-Methode (MV = 4.1±1.7g/l) mit r=0.755 ; y=19.521x + 49.165 (Abb. 23a). Bei n=344 unselektionierten Patienten-Plasmen korrelierte der FIATA (MV = 130±43%) mit einer funktionellen Fibrinogen Bestimmung im FIFTA (MV = 124±40%) mit r=0.813 ; y=0.861x + 22.993 (Abb. 23b). Die FIATA-Ergebnisse der Patienten sind normalverteilt mit einer Gauss-Verteilung (Abb. 23c).

### 2.10. Ausfällung von plasmatischem Fibrinogen durch Vancomycin

25 µl des 143 % (4.0 g/l) P-Pools wurden inkubiert mit 25 µl 0-50 mM Vancomycin in PBS, 50 µl PBS, 0.1 % Triton X 100® und 50 µl 850 mM KCI in H₂O für 1 min (RT). Die Trübung bei 405 nm wurde bestimmt. Um die Ausfällung wieder rückgängig zu machen, wurden 0-110 µl 1 M NaHCO₃, pH 8.6, oder H₂O zugesetzt zu 50 µl P-Pool, der mit 50 µl 4.4 mM Vancomycin in PBS für 1 min (RT) reagiert hatte. Zu pH-Studien wurden 5 ml P-Pool gemischt mit 5 ml PBS und 0-1750 µl 1 M NaHCO₃, pH 8.6.
5 ml P-Pool wurden inkubiert mit 5 ml 4.4 mM Vancomycin in PBS für 3 min (RT). Dann wurde die Reaktionsmischung zentrifugiert bei 2500 g (4000 rpm) für 10 min.

Der Überstand wurde getrennt vom Präzipitat. Das Präzipitat wurde nicht gewaschen oder wurde 3x mit H₂O gewaschen und nach der Resuspension jeweils zentrifugiert. Das ungewaschene Präzipitat wurde gelöst durch Zusatz von 500 µl 1 M NaHCO₃, pH 8.6, und 4500 µl PBS; das gewaschene Präzipitat wurde gelöst durch Zusatz von 300 µl 1 M NaHCO₃ zu 1700 µl in dest. H₂O resuspendiertem Präzipitat. 3 Ansätze gewaschenen Präzipitates wurden bei Raumtemperatur getrocknet und gewogen. Das gelöste Präzipität und der Überstand wurden funktionell auf Fibrinogen untersucht. 100 µl 1 M NaHCO₃, pH 8.6 wurden zugesetzt zu 400 µl des gelösten ungewaschenen Präzipitates und diese Lösung, das gelöste gewaschene Präzipitat und der Überstand wurden analysiert auf Gesamtprotein-Konzentration, Albumin-Konzentration, und Pseudocholinesterase-Aktivität an einem Hitachi 917 Analyzer, Roche, Basel, Schweiz und auf Vancomycin an einem Integra 800 (Fluorescence Polarisation Immuno Assay); Elektrophoresen wurden durchgeführt an einem auf Agarosegel basierenden Hydrasis LC-Electrophoresis System (Sebia, Fulda, Germany). Als Kontrolle wurde 1 ml gepooltes Serum inkubiert mit 1 ml 4.4 mM Vancomycin in PBS, und die Vancomycin-Konzentration wurde bestimmt.

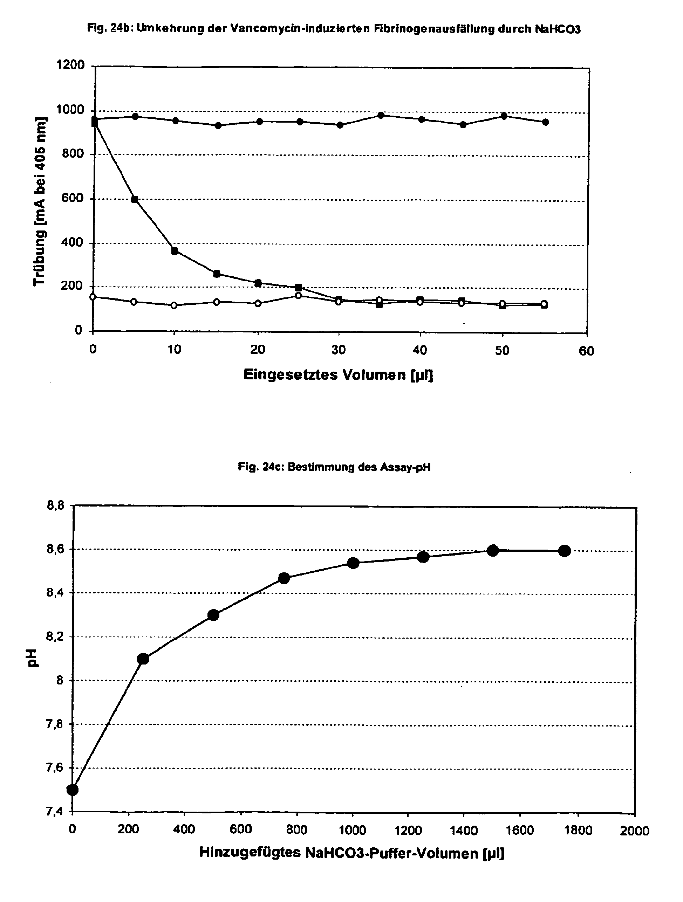

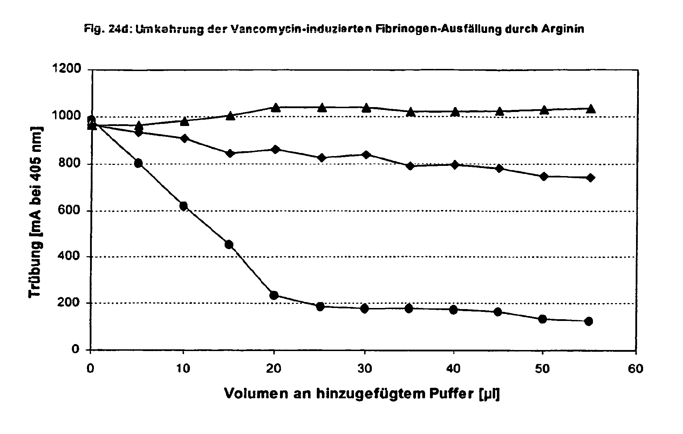

Vancomycin erhöht dosisabhängig die Trübung von Citratplasma. Die Trübung steigt bis zu ca. 20fach (Abb. 24a). Diese Ausfällung kann rückgängig gemacht werden durch NaHCO₃; 30 µl 1 M NaHCO₃, pH 8.6 erniedrigen die resultierende Trübung auf den PBS-Kontrollwert (ohne Vancomycin). Zusatz von H₂O anstelle von NaHCO₃ verändert nicht die Trübung, die durch die Vancomycin/Plasma Reaktion erzeugt worden war (Abb. 24b). Die generierte Trübung vermindert sich auch durch Zusatz von Arginin, pH 7.5, aber nicht durch PBS und nur leicht durch 20fach konzentriertes PBS (Abb. 24c). Zusatz von ≥ 10 µl 1 M NaHCO₃, pH 8.6 zu 50 µl Plasma und 50 µl PBS resultiert in einen pH-Wert von ≥ 8.5 (Abb. 24d).

### 2.11. Elektrophorese von Plasma, das mit Vancomycin inkubiert worden war

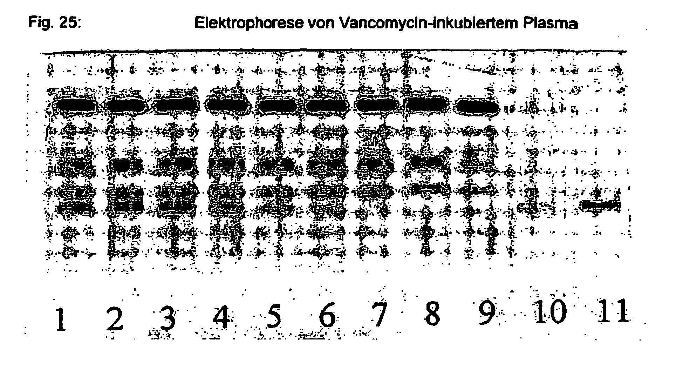

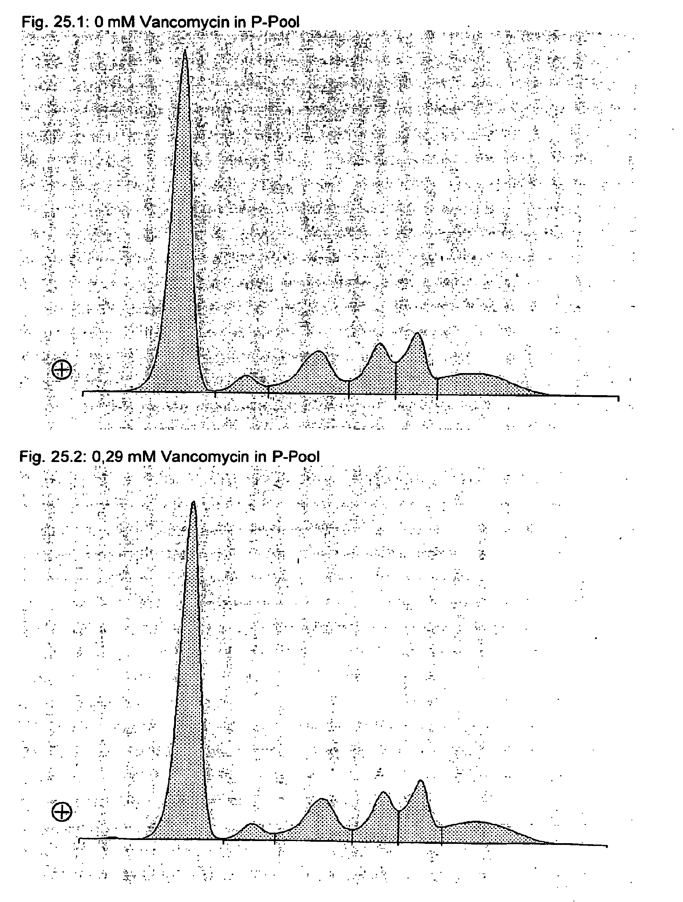

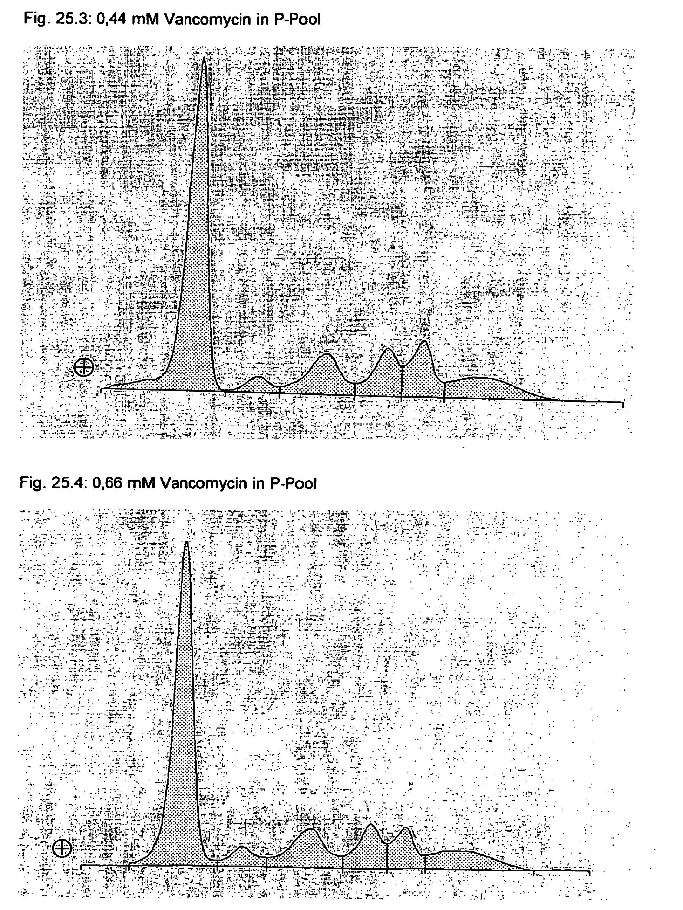

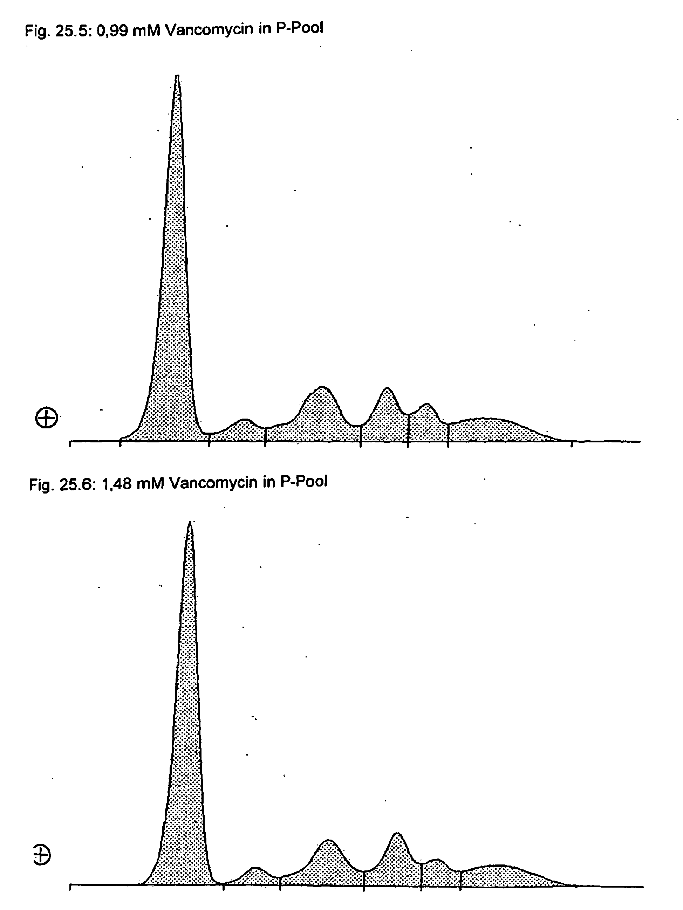

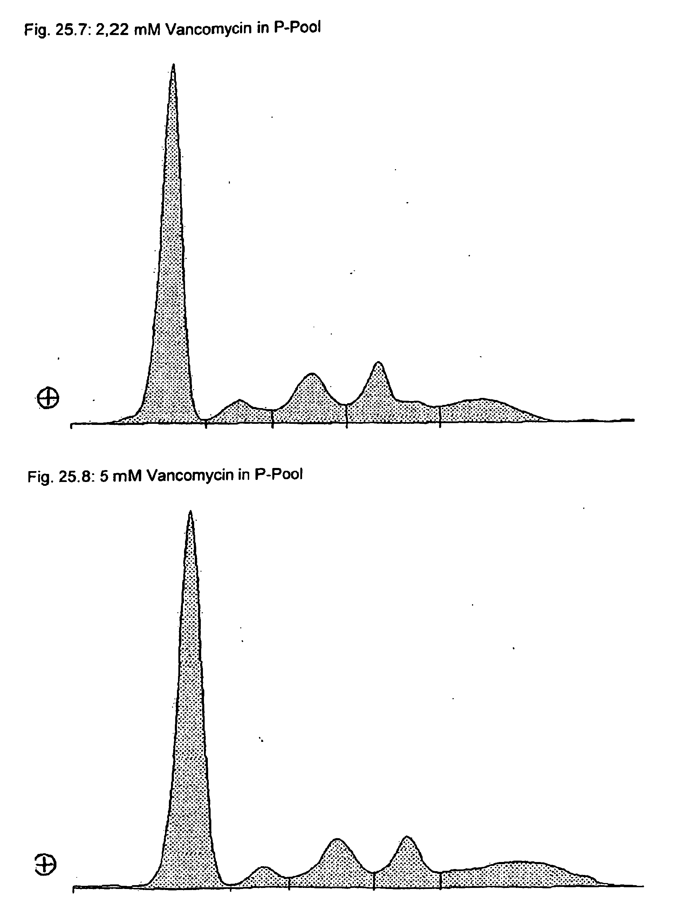

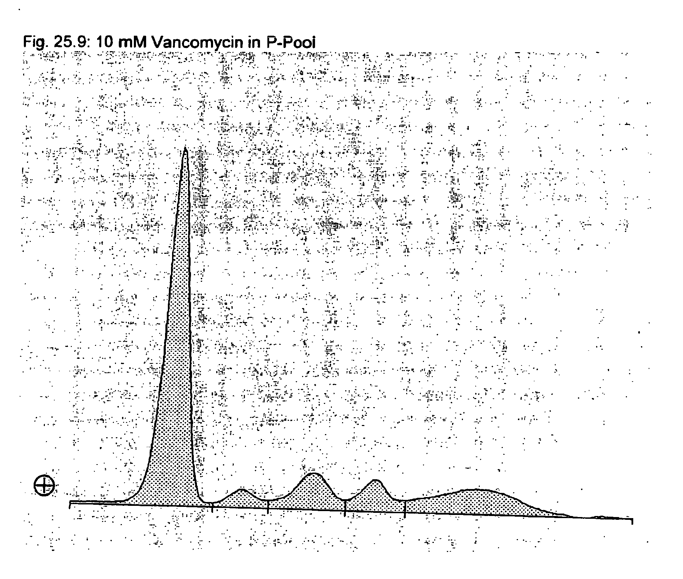

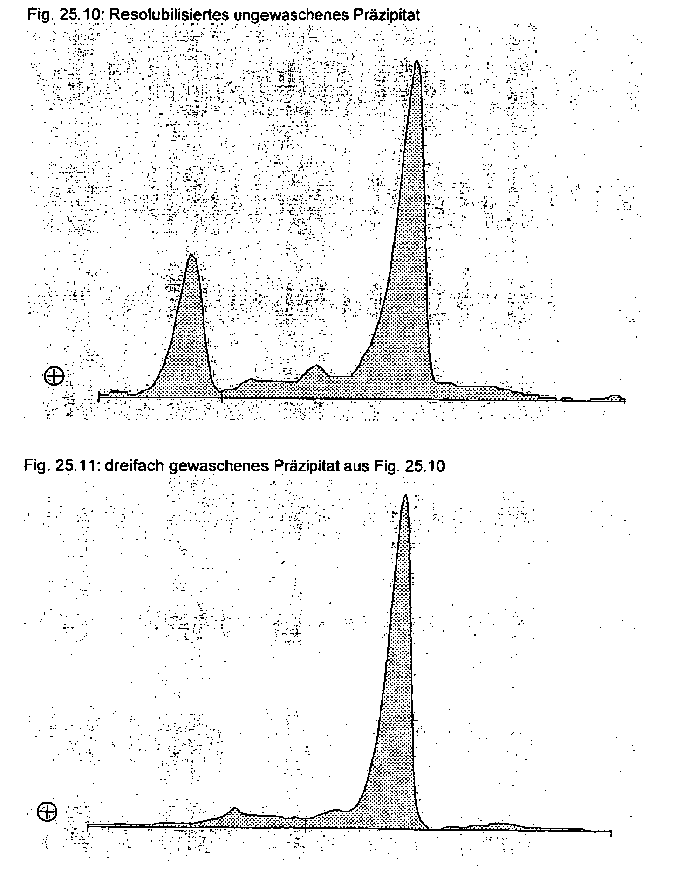

950 µl P-Pool wurde inkubiert mit 50 µl 0 - 200 mM (0 - 10 mM finale Konz.) Vancomycin für 5 min (RT). Dann wurden die Elektrophoresen der Proben und der gelösten Präzipitäte von 2.10. durchgeführt und die Gesamtprotein-Konzentrationen in den behandelten Proben wurden bestimmt.
Fibrinogen läuft in der β2 Fraktion der Plasma-Elektrophorese. Ein Anstieg der Vancomycin-Konzentration im Plasma führt zu einer proportionalen Verminderung der β2 Fraktion (Abb. 25 1-9). Bei 5 mM Vancomycin in Plasma verschwindet vollständig selektiv der β2 Peak. Die Gesamtprotein-Konzentrationen waren: 59.0, 59.1, 58.6, 59.1, 58.4, 57.0, 57.8, 56.1, 55.7 bzw. 49.4 g/l für 0, 0.29, 0.44, 0.66, 0.99, 1.48, 2.22, 5 bzw. 10 mM Vancomycin. Die Kontroll-Elektrophorese (0 mM Vancomycin) hatte die folgenden Protein Peaks: 52.8 % Albumin, 3.4 % α1, 12.0 % α2, 10.2 % β1, 10.9 % β2, and 10.7 % γ. Die β2 Fraktionen der Vancomycinbehandelten Plasmen waren 10.9 %, 10.6 %, 10.4 %, 8.0 %, 6.4 %, 5.2 %, 5.5 %, 0 % bzw. 0 % für 0 mM, 0.29 mM, 0.44 mM, 0.66 mM, 0.99 mM, 1.48 mM, 2.22 mM, 5 mM bzw. 10 mM Vancomycin.

Die Fibrinogen-Konzentration im Überstand von 1 Teil Plasma und 1 Teil 4.4 mM Vancomycin in PBS, war 0.32 g/l, d.h. 0.64 g/l Fibrinogen verblieb bei anfänglichen 4.0 g/l. Folglich verblieben 16 % des Fibrinogen im Plasma, wohingegen 84 % des Fibrinogens durch 4.4 mM Vancomycin präzipitiert wurden. Abb. 25.10. zeigt die Elektrophorese von gelöstem ungewaschenen präzipitat. 77 % des Proteins wird in der β Fraktion der Electrophorese gefunden, ca. 23 % ist Albumin. Die Gesamtprotein-Konzentration war 6.1 g/l, die Albumin-Konzentration 0.82 g/l, und die Pseudocholinesterase-Aktivität (PCHE) 136 U/l. Der P-Pool hatte eine Albumin-Konzentration von 27 g/l und eine PCHE of 3340 U/l.

Nach 3x Waschen des Präzipitates mit aqua dest. > 95 % wandert das Protein des Präzipitats in der β Fraktion der Elektrophorese als ein einziger Peak (Abb. 25.11). Die Protein-Konzentration in dieser 2 ml- Fraktion war 7 g/l (= 2.8 g/l in 5 ml), Albumin war nicht nachweisbar, PCHE war 13 U/L und die Vancomycin-Konzentration war 583 mg/l (0.4 mM), d.h. von den 2.2 mM Vancomycin in dem PlasmaNancomycin Reaktionsgemisch banden 80 µmole Vancomycin an 5 µmole präzipitierendes Fibrinogen (16 Vancomycin Moleküle/Fibrinogen Molekül). Gepooltes Serum enthielt nach 1+1 Inkubation mit 4.4 mM Vancomycin in PBS 2.1 mM Vancomycin, der Überstand von gepooltem Plasma enthielt 0.17 mM Vancomycin. Das getrocknete Präzipitat wog 13±2 mg, d.h. ca. 2.6 g/l (76 %) des 3.4 g/l ausgefallenen Plasma-Fibrinogens wurde gereinigt, ca. 24 % des Fibrinogens ging während des Waschprozesses verloren.

### 2.12. Fibrinogen-Reaktivität bei Vancomycin-präzipitiertem und wiedergelöstem Fibrinogen

50 µl P-Pool oder 50 µl 300 % Fbgen-Pool wurden inkubiert mit 50 µl 4.4 mM Vancomycin in PBS für 1 min (RT). Dann wurden 20 µl or 40 µl 1 M NaHCO₃, pH 8.6 und 200 µl 300 mlU/ml Thrombin, 400 µg/ml Polybrene®, 6 % BSA-PBS (FIFTA-Reagenz) zugesetzt und ΔA bei 405 nm wurde bestimmt. Kontrolle 1 bestand im Zusatz von Vancomycin nach (anstatt vor) dem NaHCO₃. Kontrolle 2 bestand im Ersatz von Vancomycin von Kontrolle 1 durch PBS. Kontrolle 3 bestand im Ersatz von Vancomycin und NaHCO₃ von Kontrolle 1 durch PBS. Ein Kontroll-Experiment bestand in der Inkubation von 50 µl P-Pool mit 0-50 µl 1 M NaHCO₃, pH 8.6, 50 µl PBS und 200 µl FIFTA-Reagenz.

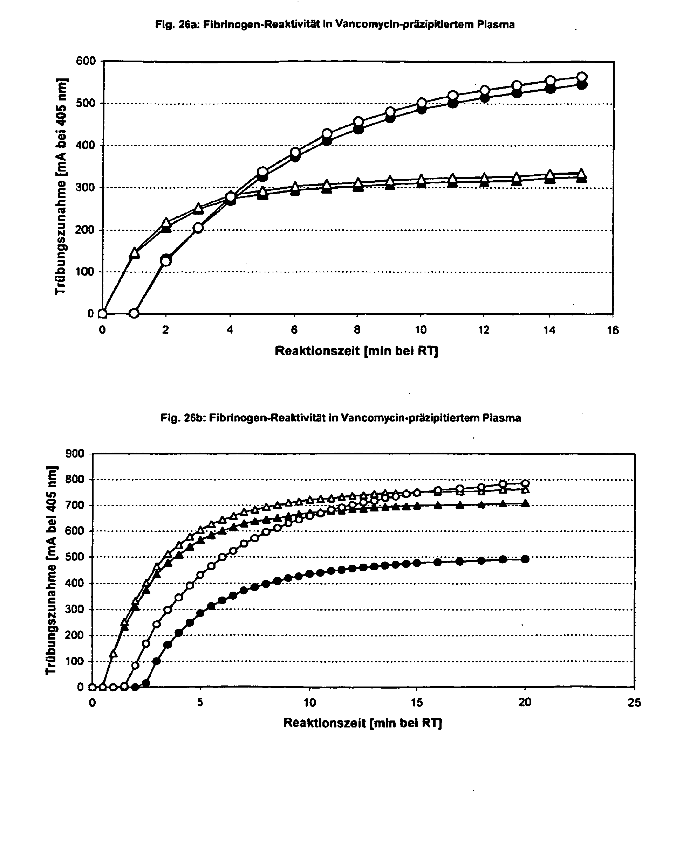

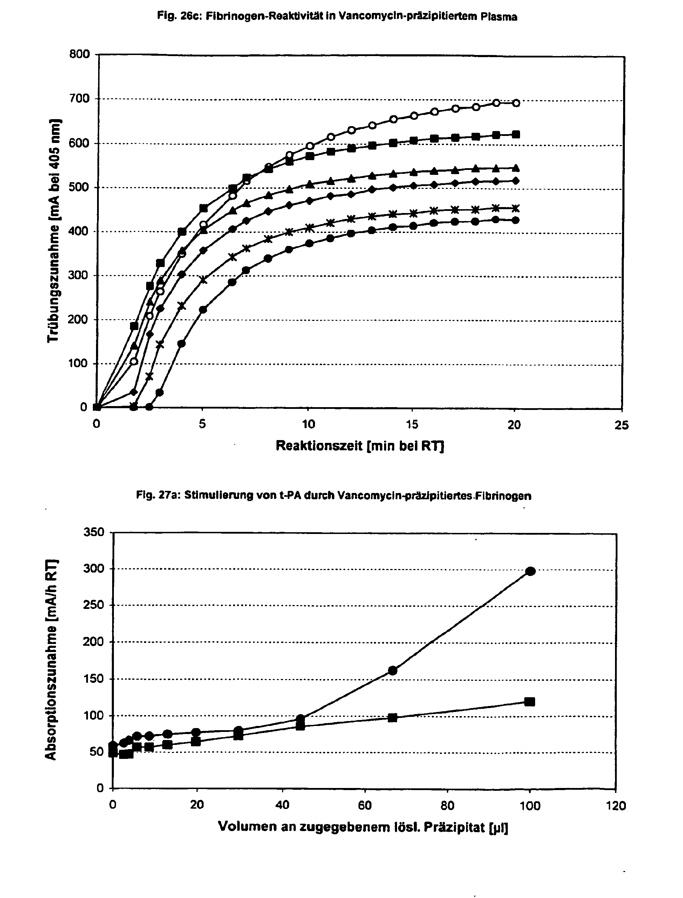

Das Vancomycin-präzipitierte und wiedergelöste Fibrinogen erhält die Aktivität zurück (Abb. 26a,b). Es scheint sogar reaktiver als normales Fibrinogen: der halbmaximale ΔA von Vancomycin-reagiertem Fibrinogen wird nach 2 min bei RT erreicht, wogegen der von normalem Fibrinogen etwa 4 min bei RT benötigt. Der maximale ΔA bei Kontrolle 1 und Kontrolle 3 ist leicht höher. Kontrolle 2 zeigt, daß der FIFTA bei physiologischem pH zu einem nahezu 2fach höheren ΔA führt als bei pH 8.6. Das NaHCO₃- Kontrollexperiment zeigt eine Verminderung des maximalen ΔA durch Erhöhung der NaHCO₃-Puffer Menge. Zusatz von 10-20 µl NaHCO₃-Puffer resultiert anscheinend in einer Beschleunigung der Fibrinbildung innerhalb der ersten 5 min RT (Abb. 26c).

### 2.13. Stimulation von t-PA durch Vancomycin-präzipitiertes Fibrinogen Reinsystem

0-100 µl des gelösten (in 500 µl 1 M NaHCO₃, pH 8.6, + 4500 µl PBS, pH 7.4) Präzipitates aus 2.4. und 100-0 µl PBS (Volumenausgleich) wurden inkubiert mit 50 µl 0.1 mg/ml Glu-Plasminogen in PBS, 1 % BSA, 50 µl 0.6 M Natriumphosphat Puffer, pH 7.4, oder 50 µl 1 M NaHCO₃, pH 8.6, und 50 µl 1000 IU/ml t-PA in PBS, 0.1 % Triton X 100® für 30 min (RT). Dann wurden 25 µl 2.7 mM Val-Leu-Lys-pNA, 1.7 M KCI zugesetzt und Δ A bei 405 nm/h RT an einem Mikrotiterplatten-Leser gemessen.

### Plasmasystem

25 µl P-Pool wurden inkubiert mit 25 µl 0-50 mM Vancomycin in PBS und 50 µl 1000 IU/ml t-PA in PBS, 0.1 % Triton X 100® und 25 µl 0 mM oder 15 mM Chloramin-T® für 30 min (RT). Dann wurden 25 µl 2.7 mM Val-Leu-Lys-pNA, 1.7 M KCI zugesetzt und die resultierende Plasminaktivität wurde bestimmt durch Messung des linearen ΔA/min bei RT an einem Mikrotiterplatten-Leser.

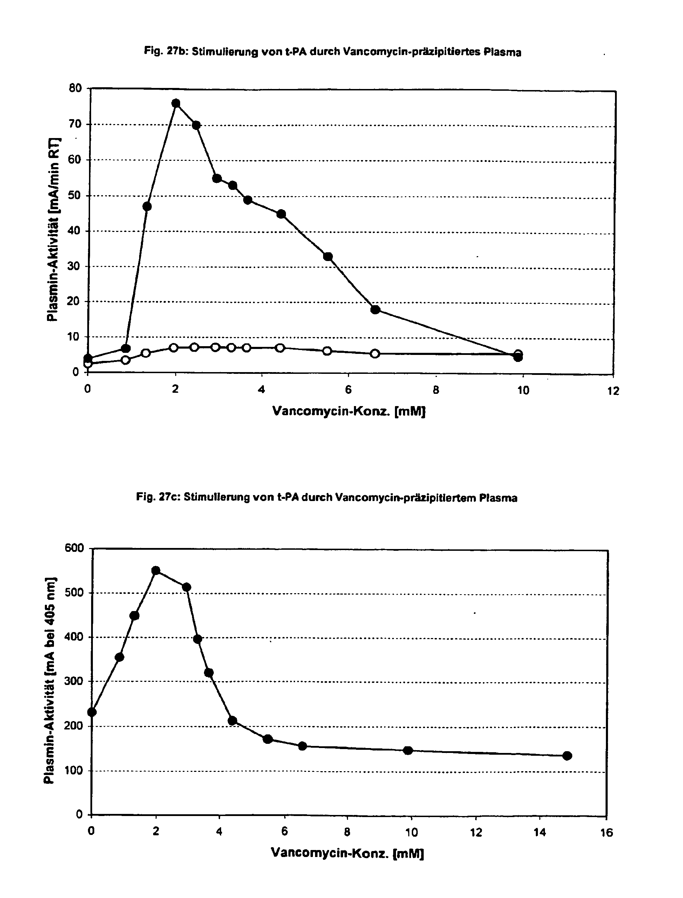

Bei pH 7.4 stimuliert das wiedergelöste Präzipitat dosisabhängig t-PA. 100 µl gelöstes Präzipitat stimulieren t-PA ca. 6fach sogar in Anwesenheit einer abnormal hohen Ionenstärke (> 600 mM Na⁺); bei pH 8.6 ist diese Stimulation nur 2.5fach (Abb. 27a). Im Plasmasystem werden maximale Plasmin-Aktivitäten nach Inkubation von 1 Teil Plasma mit 1 Teil 1.5 - 5 mM Vancomycin in PBS gemessen, wenn diese Mischungen mit 1 Teil 15 mM Chloramin-T® oxidiert werden; in dieser Testversion wird t-PA bis zu 20fach stimuliert (Abb. 27b). Ohne Oxidation steigt die t-PA Aktivität 2.5fach, wenn die finale Inkubationszeit auf 158 min (RT) verlängert und die Probe mit 2 mM Vancomycin inkubiert wird (Abb. 27c).

### 2.14. Vancomycin-Reaktivität mit plasmatischem löslichen Fibrin oder Serum

1 ml Normalplasma-Pool wurde supplementiert mit 50 µl 100 mg/ml gereinigtem Fibrinogen (Haemocomplettan®, Aventis, Frankfurt, Germany), woraus 260 % lösliches Fibrin-Polymer im Plasma entstand. 50 µl Proben der Fibrinsupplementierten und unsupplementierten Plasmen sowie ein pathologisches Standardplasma mit Fibrin-Monomeren (Berichrom FM, DadeBehring, Marburg, Germany) wurden im FIFTA und im FIATA analysiert. Gepooltes Serum wurde auch im FIFTA und im FIATA analysiert.

Normales Plasma, welches auf 260 % der Norm lösliches Fibrin aufgestockt wurde, ergibt ein FIATA-Ergebnis von 271 % der Norm und ein FIFTA-Ergebnis von 171 % der Norm. Gepooltes abzentrifugiertes Serum ergibt 0 % FIFTA und FIATA.

### 2.15. Vancomycin-Reaktivität mit oxidiertem Plasma

25 µl P-Pool oder P-Pool supplementiert mit 8.6 mM (3.2 g/l) EDTA wurden mit 25 µl 0-89 mM Chloramin-T® in PBS für 60 min bei RT inkubiert. Dann wurde der FIATA und der FIFTA durchgeführt. Beim FIATA wurden 25 µl PBS zugesetzt und die Absorption bei 405 nm wurde bestimmt (Basiswert). 50 µl 4.4 mM Vancomycin in PBS wurden zugesetzt und nach 1 min (RT) wurde die Absorption bei 405 nm erneut bestimmt. Beim FIFTA wurden 100 µl FIFTA-Reagenz zugesetzt und die ΔA bei 405 nm wurde bestimmt. 100 % Kontrolle = P-Pool ohne Oxidans.

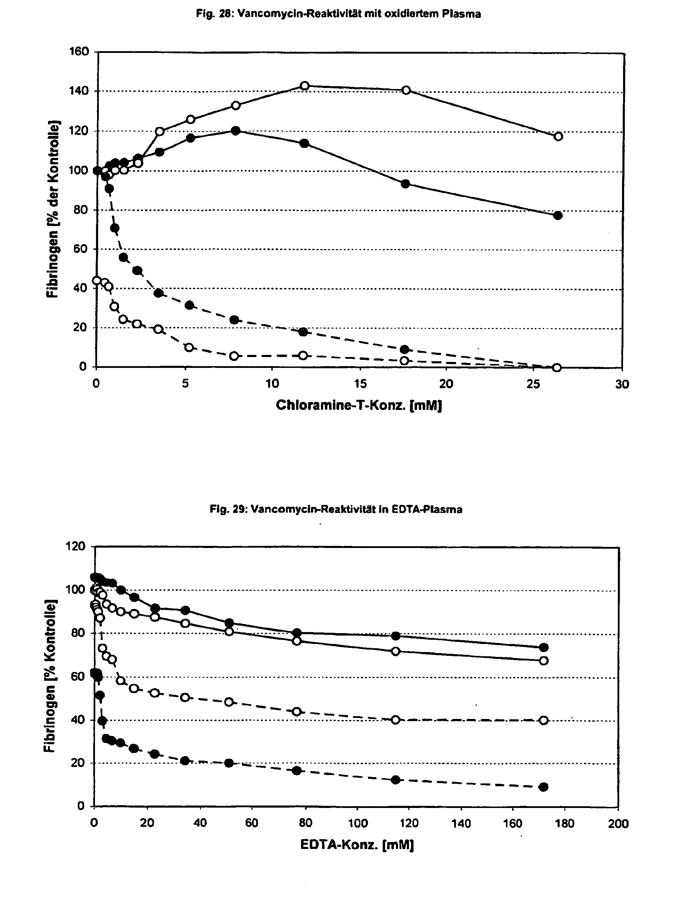

Sowohl Citrat- als auch EDTA-supplementiertes Citratplasma reagiert mit Vancomycin. Die Reaktivität steigt um 40 % des Citrat-Kontrollwertes, wenn die EDTA-Plasmen zuvor bei 10-20 mM Chloramin-T® oxidiert wurden (Abb. 28). Dagegen inaktiviert Chloramin-T® (CT) Fibrinogen im FIFTA: 2 mM Chloramin-T® inaktivieren 50 % des Fibrinogens. Diese Inaktivierung tritt ein sowohl bei P-Pool als auch bei EDTA-supplementiertem P-Pool. Zusatz von 8.6 mM EDTA führt zu einem ca. 50 % FIFTA-Abfall, unabhängig von der Oxidans-Konzentration.

### 2.16. Vancomycin-Reaktivität mit EDTA-supplementiertem Plasma

25 µl P-Pool wurde inkubiert mit 25 µl PBS oder mit 25 µl 2 mM Chloramin-T® in PBS. Nach 60 min bei RT, wurden 25 µl 0-172 mM EDTA in PBS zugesetzt. Nach 5 min bei RT wurden FIATA und FIFTA durchgeführt wie in 2.7. aufgeführt.

Zusatz von EDTA in Konzentrationen von 50-100 mM erniedrigt das FIATA-Ergebnis um nur 10-20 %. Dagegen resultieren EDTA-Konzentrationen > 5 mmol/l in eine Verminderung der FIFTA-Aktivität von ca. 50 %, sowohl bei 0 mM als auch bei 2 mM oxidierten Proben (Abb. 29).

### 2.17. Vancomycin-Reaktivität mit Fibrinogen-Spaltprodukten oder BrCNgespaltenem Fibrinogen

25 µl P-Pool wurden inkubiert mit 25 µl 0 mM oder 15 mM Chloramin-T® in PBS für 60 min (RT). Dann wurden 50 µl 1000 IU/ml t-PA, 0.1 % Triton X 100® in PBS zugefügt. Nach 0-8 h bei RT wurden 25 µl 4.4 mM Vancomycin in PBS zugesetzt und ΔA bei 405 nm wurde bestimmt. Weiterhin wurden 10 mg/ml BrCN-gespaltenes Fibrinogen in 6 % BSA-PBS oder in gepooltem Serum im FIATA getestet.

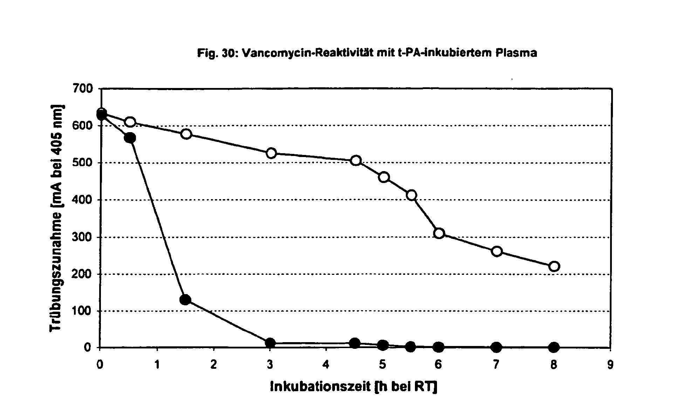

Der Zusatz von t-PA vermindert die Reaktivität von Citratplasma mit Vancomycin. Eine 3 h Inkubation von 25 µl 15 mM CT-oxidiertem Citratplasma mit 50 µl 1000 IU/ml t-PA resultiert in einen vollständigen Verlust der Vancomycin-Reaktivität, ein 50 %iger Verlust tritt auf nach ca. 1 h, wogegen bei nichtoxidiertem Plasma 6 h gebraucht werden für einen 50 %igen Verlust der Vancomycin-Reaktivität; dies ist bedingt durch die Abwesenheit von funktionellem α2-Antiplasmin in 15 mM CT-oxidiertem Plasma (Abb. 30). BrCN-gespaltenes Fibrinogen reagiert nicht im FIATA.

### Abbildungen

### Abb. 1a-d: Optimierung der Thrombin-Konzentration

50 µl P-Pool (Abb. 1a) oder P-Pool verdünnt 1:2 mit 6 % BSA-PBS (Abb. 1b) wurden mit 100 µl 0 - 1519 mlU/ml bovinem Thrombin für 0 - 15 min (RT) inkubiert und die Trübungs-Zunahme (ΔA) bei 405 nm wurde bestimmt; Thrombin-Konzentrationen im Thrombin-Reagenz: 0 mlU/ml (O), 26 mlU/ml (■), 39 mlU/ml (-), 59 mlU/ml (X), 89 mlU/ml (*), 133 mlU/ml (□), 200 mlU/ml (+), 300 mlU/ml (●), 450 mlU/ml (◊), 675 mIU/ml (◆), 1013 mIU/ml (Δ), 1519 mIU/ml (▲); ΔA-Quotienten verdünnter P-Pool / P-Pool (Abb. 1c). Inkubations-Zeiten (RT) für P-Pool: 2.5 min (◆), 5 min (●), 10 min(▲), 15 min (■); Inkubations-Zeiten (RT) für 1:2 verdünntem P-Pool: 2.5 min (◇), 5 min (○), 10 min (Δ), 15 min (□) (Abb. 1d).

### Abb. 2a-d: Optimierung der Polybrene® (PB)-Konzentration

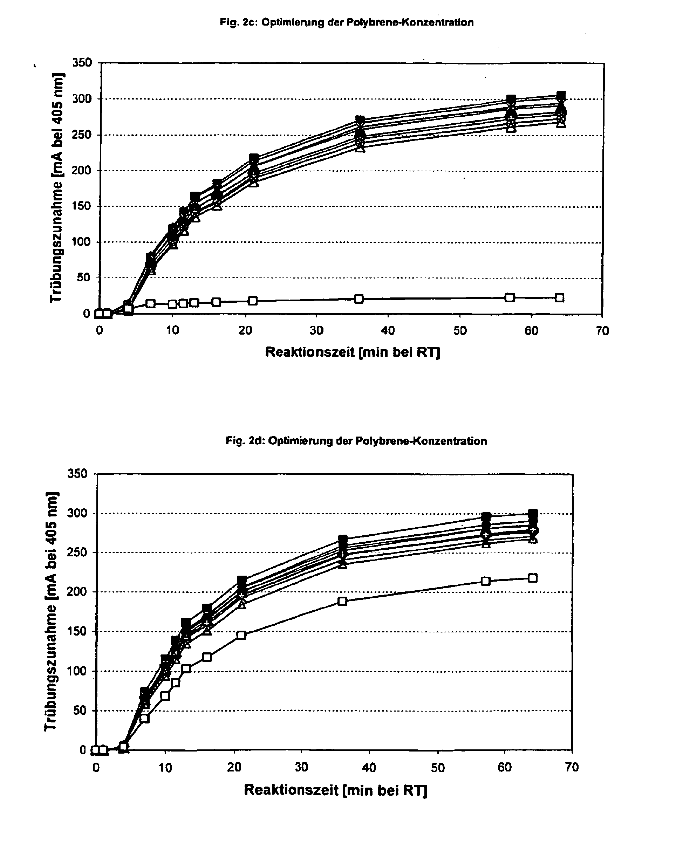

P-Pool supplementiert mit 0-35 IU/ml Heparin wurde im FIFTA analysiert. Das FIFTA-Reagenz enthielt 200 mlU/ml Thrombin, 6% BSA-PBS und 0 µg/ml Polybrene® (PB; Abb. 2a), 50 µg/ml PB (Abb. 2b), 100 µg/ml PB (Abb. 2c), 200 µg/ml PB (Abb. 2d); Heparin-Konzentrationen in der Probe: 0 IU/ml (◆), 0.14 IU/ml (■), 0.27 IU/ml (▲), 0.55 IU/ml (X), 1.09 IU/ml (*), 2.19 IU/ml (●), 4.38 IU/ml (+), 8.75 IU/ml (△), 17.5 IU/ml (O), 35 IU/ml (□).

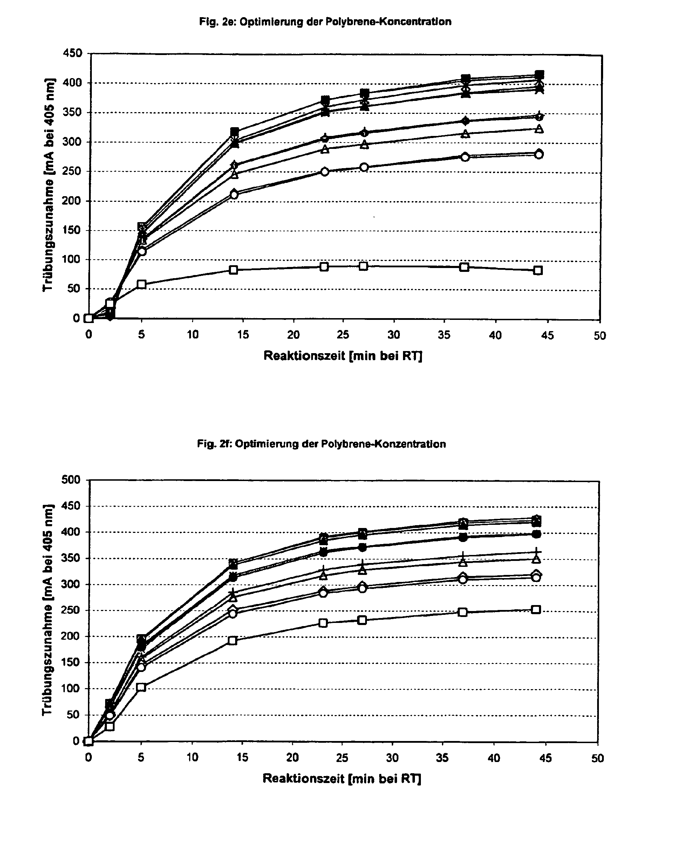

### Abb. 2e-g: Optimierung der Polybrene®-Konzentration

P-Pool supplementiert mit 0-100 IU/ml Heparin wurde im FIFTA analysiert. Das FIFTA-Reagenz enthielt 200 mlU/ml Thrombin, 6% BSA-PBS und 400 µg/ml PB (Abb. 2e), 800 µg/ml PB (Abb. 2f), 1600 µg/ml PB (Abb. 2g); Heparin-Konzentrationen in der Probe: 0 IU/ml (◆), 11 IU/ml (■), 13 IU/ml (▲), 17 IU/ml (X), 21 IU/ml (*), 26 IU/ml (●), 33 IU/ml (+), 41 IU/ml (Δ), 51 IU/ml (◊), 64 IU/ml (O), 80 IU/ml (□), 100 IU/ml (-).

### Abb. 3: Neutralisierung von 100 IU/ml Heparin im Plasma durch Polybrene®

25 µl 0-75 mg/ml Polybrene® in aqua dest. wurden zugesetzt zu 50 µl P-Pool (■) oder zu 50 µl P-Pool, der mit 100 IU/ml Heparin supplementiert war (**●**). Dann wurden 100 µl 200 mlU/ml Thrombin in 6 % BSA-PBS, 400 µg/ml PB zugesetzt und ΔA at 405 nm wurde bestimmt.

### Abb. 4: FIFTA bei Polybrene®-neutralisiertem heparinisiertem Plasma

25 µl 0-75 mg/ml Polybrene® in aqua dest. wurden zugesetzt zu 50 µl P-Pool (●) oder zu 50 µl P-Pool supplementiert mit 100 IU/ml Heparin (■). Dann wurden 100 µl 200 mlU/ml Thrombin, 400 µg/ml PB, 6 % BSA-PBS zugesetzt und ΔA bei 405 nm wurde bestimmt.

### Abb. 5: Trübungs-Zunahme durch Heparin/Polybrene® Komplexe

50 µl Plasma mit 0-80 IU/ml Heparin wurden inkubiert mit 100 µl FIFTA-Reagenz ohne Thrombin, enthaltend 0 µg/ml (O), 400 µg/ml (■), 800 µg/ml (▲), or 1600 µg/ml (●) PB. ΔA bei 405 nm wurde bestimmt.

### Abb. 6a,b: Albumin-Abhängigkeit der Trübungs-Zunahme

P-Pool (●), P-Pool verdünnt 1:2 mit PBS (■) oder mit 6 % BSA-PBS (O) wurden im FIFTA analysiert unter Verwendung des 200 mlU/ml Thrombin-Reagenzes ohne Albumin (Abb. 6a). Die ΔA-Quotienten verdünnt/unverdünnt wurden berechnet (Abb. 6b).

### Abb. 7a,b: Reaktions-Kinetik des FIFTA

Der FIFTA wurde durchgeführt mit P-Pool und mit 6% Albumin-PBS Verdünnungen von P-Pool. ΔA wurde nach 0-15 min und nach 25 min, 75 min, 17 h (RT) bestimmt; Fibrinogen-Aktivität [% der Norm] in der Probe: 0 % (O), 2.5 % ( ), 3.9 % (-), 5.7 % (◇), 8.6 % (●), 19 % (+), 29 % (Δ), 43 % (*), 65 % (◆), 98 % (■), 146 % (▲) (Abb. 7a). ΔA nach 2 min (◆), 5 min (■), 10 min (▲), 15 min (□), 25 min (*), 75 min (●), 17 h (O) (Abb. 7b).

### Figg. 7c-e: Reaktions-Kinetik des FIFTA

Der FIFTA wurde durchgeführt mit 100 % Normal-Pool (●), 200 % Pool-Plasma (■) (Abb. 7c), 256 % Plasma-Pool (•), 120 % Plasma-Pool (■), und 63 % Plasma-Pool (▲) (Abb. 7d); ΔA-Quotienten 120% / 256% (●) and 63% / 256% (▲) (Abb. 7e).

### Abb. 7f,g: Reaktions-Kinetik des FIFTA

Der FIFTA wurde durchgeführt mit 300 % Fbgen-Pool (■), mit 1:2 mit 6 % BSA-PBS verdünntem 300 % Fbgen Pool (●) und mit 120 % Standard-Pool-Plasma (Δ) (Abb. 7f); ΔA - Quotienten 150% /300% (Abb. 7g).

### Abb. 8a,b: Reaktions-Kinetik bei 37°C

Der FIFTA wurde durchgeführt mit 256 % Fbgen-Pool (■) und mit 256 % Fbgen-Pool, der 1:2 mit 6 % BSA-PBS verdünnt worden war (●). Nach Reaktions-Zeiten von 30 - 370 Sekunden in 10 Sekunden-Intervallen und nach 20 und 60 min in einem 37°C Wasserbad wurde die Trübungs-Zunahme bei at 405 nm bestimmt (Abb. 8a). Die 125% / 250% ΔA - Quotienten wurden berechnet (Abb. 8b).

### Abb. 9a: Test-Linearität

Der FIFTA wurde mit 300 % Fbgen-Pool und Verdünnungen daraus (mit 6% BSA-PBS) durchgeführt; 50 µl + 100 µl Test-Version (●), 25 µl + 50 µl Test-Version (O).

### Abb. 9b,c: Test-Linearität

Der FIFTA wurde mit 300 % Fbgen-Pool und Verdünnugen (physiol. NaCl) daraus durchgeführt; Fibrinogen-Aktivität in den Proben: 0 % (-), 17 % (□), 23 % (Δ), 30 % (◇), 40 % (*), 53 % (●), 71 % (+), 95 % (X), 127 % (○), 169 % (■), 225 % (▲), 300 % (◆) Trübungs-Zunahme in Abhängigkeit vo der Reaktions-Zeit (Abb. 9b); ΔA in Abhängigkeit von der Fibrinogen-Aktivität (Abb. 9c): Inkubations-Zeiten bei RT: 2.5 min (O), 5 min (●), 10 min (■), 15 min (□).

### Abb. 10a,b: Variation der Test-Volumina

250 % Fbgen-Pool (●) und ein 250 % Fbgen-Pool, der 1:2 mit 6 % BSA-PBS (O) verdünnt worden war, wurde im FIFTA (50 µl Probe + 100 µl Thrombin-Reagenz) untersucht. 67 µl 250 % Fbgen-Pool (■) oder 1:2 verdünnter Fbgen-Pool (□) wurden mit 83 µl Thrombin-Reagenz inkubiert. 100 µl 250 % Fbgen-Pool (▲) oder 1:2 verdünnter Fbgen-Pool (Δ) wurde inkubiert mit 50 µl Thrombin-Reagenz, enthaltend 600 mIU/ml Thrombin (Abb. 10a); ΔA - Quotienten 125% / 250% (Abb. 10b).

### Abb. 11: Absorptions-Wellenlänge für Trübung

Der FIFTA wurde mit P-Pool und mit 256 % Fbgen-Pool durchgeführt. Reaktions-Zeit = 30 min RT. Die End-Absorption wurde bei 405 nm, 450 nm, 595 nm, und 650 nm durch einen Mikrotiterplatten-Leser bestimmt.

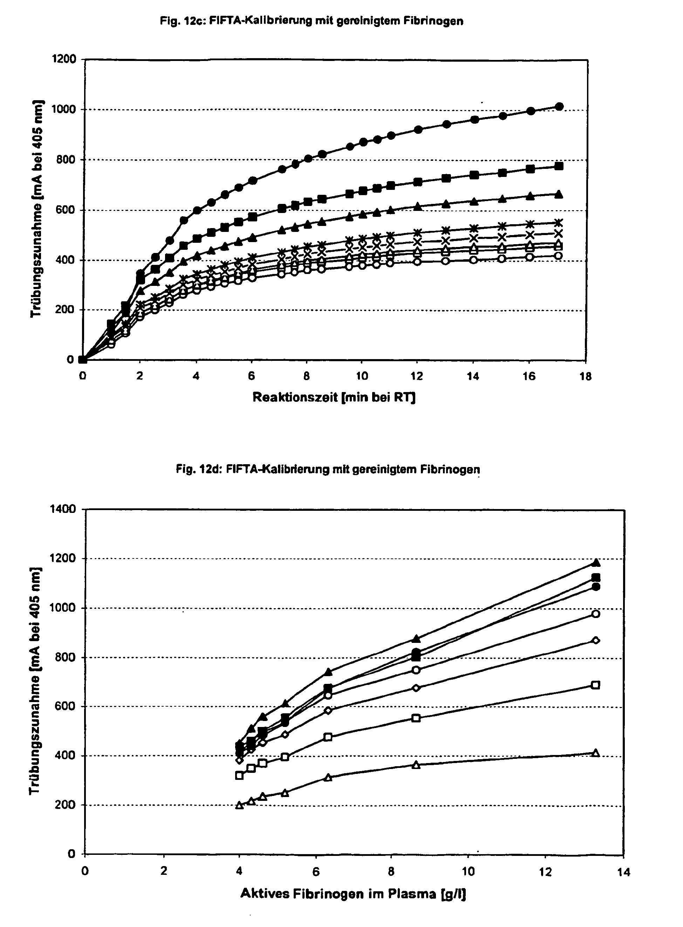

### Abb. 12 a-e: Additionskalibration des FIFTA mit gereinigtem Fibrinogen

6 % BSA-PBS (Abb. 12a,b) oder P-Pool (enthaltend 144 % der Norm Fibrinogen Aktivität) (Abb. 12c) wurden supplementiert mit gereinigtem Fibrinogen um 20 g/l (Δ), 10 g/l ( ), 5 g/l (◇), 2.5 g/l (*), 1.25 g/l (X), 0.63 g/l (▲), 0.31 g/l (■), 0 g/l (◆). Der FIFTA wurde durchgeführt; Trübungs-Zunahme nach Inkubationen (RT) von 2.5 min (Δ), 5 min (□), 10 min (*), 15 min (O), 48 min (▲), 158 min (■), 18 h (●) (Abb. 12b,d). Aus Abb. 12c resultierte, daß 46 % des gereinigten Fibrinogens gerinnbar war; 6 % BSA-PBS (●) oder P-Pool (■) (Abb. 12e).

### Abb. 13a,b: Korrelation mit der Clauss-Methode

Bei n=334 unselektionierten Patientenplasmen (≤ 6 h alt; zum Zeitpunkt des üblichen Verwerfens der Proben) wurde der FIFTA (MV = 132%, SD=41%) durchgeführt und verglichen mit der modifizierten Clauss-Methode (MV = 4.18 g/l, SD=1.65 g/l) (Abb. 13a). Die FIFTA-Ergebnisse der Patienten verteilen sich mit einer normalen Gauss-Verteilung (Abb. 13b).

### Abb. 14a,b: IC50 von Heparin im FIFTA

50 µl P-Pool wurden zu 10 µl 0-160 IU/ml Heparin zugesetzt. 100 µl 200 mIU/ml Thrombin in 6 % BSA-PBS (ohne Polybrene®) wurden zugefügt und ΔA bei 405 nm wurde gemessen; Heparin-Konzentrationen: 0 IU/ml (●), 0.04 IU/ml (■), 0.08 IU/ml (▲), 0.16 IU/ml (Δ), 0.31 IU/ml ( ), 0.63 IU/ml (○) (Abb. 14a). ΔA nach Inkubationen von 5 min (●), 10 min (■), 15 min (▲), 20 min (Δ) (RT) (Abb. 14b).

### Abb. 15a: Inhibitorischer Zeitpunkt 50 % von 10 IU/ml Heparin im FIFTA

50 µl P-Pool wurden inkubiert mit 100 µl 200 mIU/ml Thrombin in 6 % BSA-PBS (ohne Polybrene®). Zu den Inkubations-Zeitpunkten 0 min (vor Thrombinzusatz) (O), 0.5 min (■), 1 min (▲), 1.5 min (◆), 2 min (*), 2.5 min (●), 3 min (+), 3.5 min (Δ), 4 min (X), 5 min (-Δ-), 6 min (-■-), 7 min (-), 8 min (-*-), 9 min (-X-), 10 min (◊), 11 min (-□-) wurden 10 µl 160 IU/ml Heparin in 6% BSA-PBS zugesetzt (finale Heparin-Konzentration in Plasma + Thrombin-Rreagenz =10 IU/ml) und ΔA bei 405 nm wurde bestimmt.

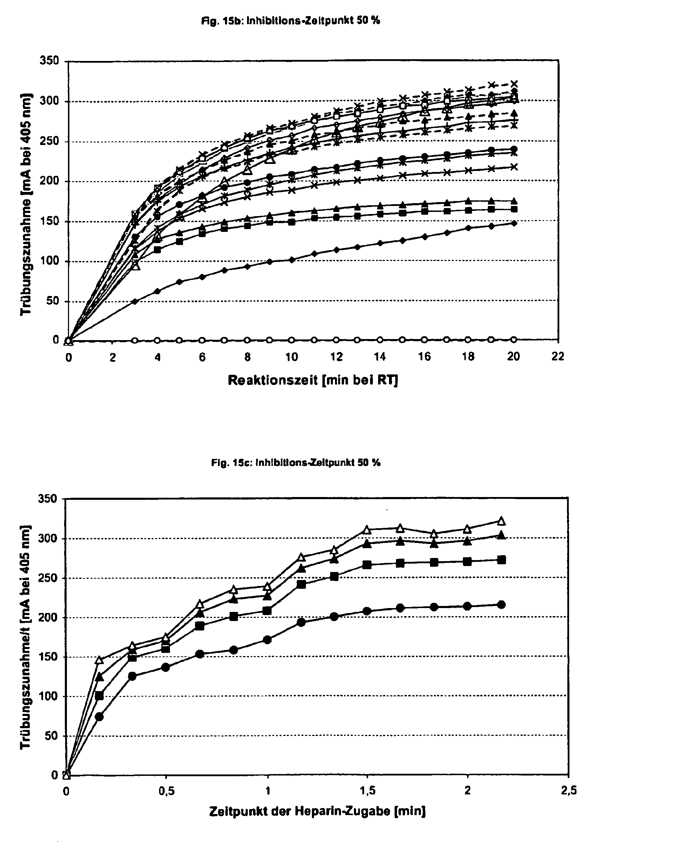

### Abb. 15b,c: Inhibitorischer Zeitpunkt 50 % von 10 IU/ml Heparin im FIFTA

50 µl P-Pool wurden inkubiert mit 100 µl 200 mlU/ml Thrombin in 6 % BSA-PBS (ohne Polybrene®). Nach Inkubationszeiten von 10 s (◆), 20 s (■), 30 s (▲), 40 s (X), 50 s (*), 60 s (●), 70 s (+), 80 s (◊), 90 s (-), 100 s (-◆-). 110 s (□), 120 s (-▲-), 130 s (-x-), 140 s (-*-) bei RT wurden 10 µl 160 IU/ml Heparin in 6 % BSA-PBS zugesetzt; Kontrolle: Zusatz von 10 µl 0 IU/ml Heparin (Δ). ΔA bei 405 nm wurde bestimmt. (Abb.15b). ΔA nach Inkubationen von 5 min (•), 10 min (■), 15 min (▲), 20 min (Δ) (RT) (Abb. 15c).

### Abb. 16a,b: Inhibition der Fibrin-Bildung durch Arginin

10 ml FIFTA-Reagenz wurden zu 5 ml P-Pool zugesetzt, und 100 µl dieser Mischung wurden sofort in eine Mikrotiterplatte pipettiert, die vorgefült worden war mit 200 µl 0-1500 mM Arginin, pH 8.7, je Vertiefung, woraus finale Arginin- Konz. von 0 mM (•), 16 mM (■), 31 mM (A), 62.5 mM (O), 125 mM (*), 250 mM (◇), 500 mM (+), 1000 mM (□) resultierten. ΔA während der ersten 9 min (RT) (Abb. 16a) oder 75 min (RT) (Abb. 16b) wurden gemessen.

### Abb. 16c: Inhibition der Fibrin-Bildung durch Arginin

10 ml FIFTA-Reagenz wurden zu 5 ml P-Pool zugesetzt, und nach 0 - 4 min (RT) wurden 100 µl dieser Mischung in eine Mikrotiterplatte pipettiert, die mit 200 µl 0-1500 mM Arginin, pH 8.7, je Vertiefung vorgefüllt worden war. ΔA bei 405 nm wurde bestimmt; die finalen Arginin-Konz. waren: 0 mM (●), 16 mM (■), 31 mM (▲), 62.5 mM (○), 125 mM (*), 250 mM (◇), 500 mM (+), 1000 mM (□).

### Abb. 16d: Inhibition der Fibrin-Bildung durch Arginin

50 µl P-Pool wurden inkubiert mit 100 µl FIFTA-Reagenz. Nach 0-30 min (RT) wurden 0-70 µl 1.5 M Arginin, pH 8.7, zugesetzt und nach dem letzten Zeitpunkt (30 min) wurde die finale ΔA bei 405 nm bestimmt; die finalen Arginin-Konz. waren: 0 mM (●), 94 mM (■), 176 mM (▲), 250 mM (O), 316 mM (*), 375 mM (◇), 429 mM (+), 477 mM (□).

### Abb. 17: Fibrin-Auflösung durch Arginin

50 µl P-Pool wurden inkubiert mit 100 µl FIFTA-Reagenz. Nach 30 min (RT) wurden 10 µl oder 70 µl 1.5 M Arginin, pH 8.7 (finale Arginin-Konz. 94 mM (●) oder 477 mM (■)) zugesetzt and der finale ΔA wurde bei 405 nm nach 0-132 min (RT) bestimmt.

### Abb. 18: Inaktierung von Fibrinogen durch Vancomycin

200 µl 300 % (8.4 g/l) Fbgen-Pool wurden mit 25 µl 0 - 100 mM Vancomycin (■) oder 0-100 mM Chloramin-T® (●) für 15 min (37°C) inkubiert. 50 µl Proben dieser Reaktionsgemische wurden entnommen und mit 100 µl 200 mlU/ml Thrombin, 400 µg/ml Polybrene®, 6 % BSA-PBS inkubiert. ΔA bei 405 nm wurde bestimmt.

Abb. 19a,b: Ist die Reaktion zwischen Vancomycin und plasmatischem Fibrinogen durch Antioxidantien inhibierbar ? 750 µl 300 % (8.4 g/l) Fbgen-Plasma-Pool wurden supplementiert mit 250 µl 200 mM von a) den ¹O₂ Inhibitoren Methionin (▲) oder Natriumaskorbat (■), b) dem OH Radikal-Inhibitor Mannit (●), oder c) H₂O (O). 25 µl Proben davon wurden mit 25 µl 0-2.5 mM Vancomycin (Abb. 19a) oder 0 - 5 mM Chloramin-T® (Abb. 19b) in PBS für 15 min bei 37°C inkubiert. Dann wurden 100 µl 200 mlU/ml Thrombin, 400 µg/ml Polybrene® in 6 % BSA-PBS zugesetzt und ΔA/5 min (RT) verglichen mit der 100 % Kontrolle (0 mM Vancomycin oder Chloramin-T®).

### Abb. 20a: ΔA in Abhängigkeit von der Vancomycin-Konzentration

50 µl Patienten-Pool (●) oder 1:2 (▲) oder 1:4 (■) verdünnter P-Pool, oder 6 % BSA-PBS (O) wurden mit 50 µl 0 - 6.8 mM Vancomycin in PBS inkubiert. ΔA bei 405 nm wurde nach 1 min (RT) bestimmt.

### Abb. 20b: ΔA in Abhängigkeit der Fibrinogen-Konzentration

25 µl 300 % Fbgen-Pool wurden inkubiert mit 125 µl 0.55 mM (▲), 1.1 mM (■), oder 2.2 mM (●) Vancomycin in PBS für 1 min (RT). Dann wurde ΔA bei 405 nm bestimmt.

### Abb. 21: FIATA-Eichung mit gereinigtem Fibrinogen

250 mg lyophilisiertes gereinigtes Fibrinogen wurden mit 12.5 ml von entweder 6 % BSA-PBS (●) oder P-Pool (■) rekonstituiert. Die erhaltenen supplementierten Fibrinogen-Proben wurden 1:2, 1:4, 1:8, 1:16, 1:32, 1:64, 1:128 verdünnt entweder mit BSA-PBS oder P-Pool, woraus Proben resultierten, die entweder 0 - 20 g/l Fibrinogen in BSA-PBS oder Proben, die 4 - 24 g/l Fibrinogen in P-Pool enthielten. Der FIATA wurde durchgeführt.

### Abb. 22a-e: FIATA-Linearität

250 % Fbgen-Pool wurde mit 6% BSA-PBS verdünnt und im FIATA getestet (Abb. 22a). 300 % Fbgen-Pool wurde mit normlem Serum verdünnt und im FIATA getestet (Abb. 22b). Die Test-Volumina von Abb. 22b wurden verdoppelt und der FIATA wurde durchgeführt (Abb. 22c). 0-55 µl 250 % Fbgen-Pool wurden mit a) 50 µl PBS und 50 µl 4.4 mM Vancomycin in PBS inkubiert. ΔA bei 405 nm wurde bestimmt (Abb. 22d). Der FIATA wurde durchgeführt mit 50 µl Probe (0-300 % der Norm) und 50 µl 4.4 mM Vancomycin in PBS (Abb. 22e).

### Abb. 23a-c: FIATA-Korrelation mit funktioneller Fibrinogen-Bestimmungen

Bei n=321 unselektionierten Patientenplasmen wurde der FIATA durchgeführt und verglichen mit der modifizierten Clauss-Methode (Multifibren U®; Behring Coagulation Timer). r=0.755 ; y=19.521x + 49.165 (Abb. 23a). Bei n=344 unselektionierten Patientenplasmen wurde der FIATA durchgeführt und mit der funktionellen Fibrinogen-Bestimmung im FIFTA verglichen; r=0.813 ; y=0.861x + 22.993 (Abb. 23b), die FIATA Ergebnisse verteilen sich mit einer normalen Gauss-Verteilung (Abb. 23c).

### Abb. 24a: Ausfällung von plasmatischem Fibrinogen durch Vancomycin

25 µl von 143 % (4.0 g/l) P-Pool wurden inkubiert mit 25 µl 0-50 mM Vancomycin in PBS, 50 µl PBS, 0.1 % Triton X 100® und 50 µl 850 mM KCI in H₂O für 1 min (RT). ΔA bei 405 nm wurde bestimmt.

### Abb. 24b: Aufhebung der Vancomycin-induzierten Fibrinogen-Ausfällung NaHCO₃

50 µl P-Pool reagierten mit 50 µl 4.4 mM Vancomycin in PBS für 1 min (RT). Zur Aufhebung der Vancomycin-induzierten Fibrinogen-Ausfällung wurden 0 - 55 µl 1 M NaHCO₃, pH 8.6 (■), oder H₂O (●) zugesetzt. Die Absorption bei 405 nm wurde nach 1 min (RT) bestimmt. Absorptions-Kontrolle (O) ohne Vancomycin, mit NaHCO₃.

### Abb. 24c: Bestimmung des Test-pH von Abb. 26b

5 ml P-Plasma wurden mit 5 ml PBS und 0-1750 µl 1 M NaHCO₃, pH 8.6 gemischt und der pH wurde bestimmt.

### Abb. 24d: Aufhebung der Vancomycin-induzierten Fibrinogen-Ausfällung durch Arginin

50 µl P-Pool reagierten mit 50 µl 4.4 mM Vancomycin in PBS für 1 min (RT). Zur Aufhebebung der Vancomycin-induzierten Fibrinogen-Ausfällung wurden 0 - 55 µl PBS (▲) zugesetzt, 20fach konzentriertes PBS (◆), oder 1 M Arginin, pH 7.5 (●). Die Absorption bei 405 nm wurde nach 1 min (RT) bestimmt.

### Abb. 25: Electrophorese von Plasma, das mit Vancomycin inkubiert worden war

P-Pool wurde mit 0 - 10 mM (finale Konz.) Vancomycin für 5 min (RT) inkubiert. Dann wurden Elektrophoresen der Proben durchgeführt (⊕ = Anode zur Linken der Abbildung).
Spur 1: 0 mM Vancomycin in 143 % der Fibrinogen-Antigen-Norm P-Pool (Abb.25.1)
Spur 2: 0.29 mM Vancomycin in P-Pool (Abb. 25.2)
Spur 3: 0.44 mM Vancomycin in P-Pool (Abb. 25.3)
Spur 4: 0.66 mM Vancomycin in P-Pool (Abb. 25.4)
Spur 5: 0.99 mM Vancomycin in P-Pool (Abb. 25.5)
Spur 6: 1.48 mM Vancomycin in P-Pool (Abb. 25.6)
Spur 7: 2.22 mM Vancomycin in P-Pool (Abb. 25.7)
Spur 8: 5 mM Vancomycin in P-Pool (Abb. 25.8)
Spur 9: 10 mM Vancomycin in P-Pool (Abb. 25.9)
Spur 10: Wiedergelöstes ungewaschenes Präzipitat aus 5 ml Plasma und 5 ml 4.4 mM Vancomycin in PBS (Abb. 25.10)
Spur 11: 3x gewaschenes Präzipitat von Abb. 25.10 (Abb. 25.11)

### Abb. 26a-c: Fibrinogen-Reaktivität bei Vancomycin-präzipitiertem Plasma

50 µl P-Pool oder 300 % Fbgen-Pool wurden mit 50 µl 4.4 mM Vancomycin in PBS für 1 min (RT) inkubiert. Dann wurden 20 µl 1 M NaHCO₃ , pH 8.6, und 200 µl 300 mlU/ml Thrombin, 400 µg/ml Polybrene®, 6 % BSA-PBS (FIFTA-Reagenz) zugesetzt und ΔA bei 405 nm wurde bestimmt (Plasma/Vanco/NaHCO₃ = ▲). Kontrolle 1 bestand in dem Zusatz von Vancomycin nach dem NaHCO₃ (Plasma/NaHCO₃/Vanco = Δ). Kontrolle 2 bestand in dem Ersatz des Vancomycins von Kontrolle 3 durch PBS (Plasma/PBS/ NaHCO₃ = ●). Kontrolle 3 bestand in dem Ersatz von Vancomycin und NaHCO₃ von Kontrolle 1 durch PBS (Plasma/PBS/PBS = O) (Abb. 26a).
Anstatt von 20 µl NaHCO₃ wurde 40 µl NaHCO₃ verwendet (Abb. 26b).
50 µl P-Pool wurden mit 0 µl (O), 10 µl (■), 20 µl (▲), 30 µl (◆), 40 µl (*), 50 µl (●) 1 M NaHCO₃, pH 8.6, 50 µl PBS und 200 µl FIFTA-Reagenz inkubiert, ΔA bei 405 nm wurde bestimmt (Abb. 26c).

### Abb. 27a: Stimulation von t-PA durch Vancomycin-präzipitiertes Fibrinogen

0-100 µl des gelösten Präzipitates von Abb. 25 und 100-0 µl PBS (zum VolumenAusgleich) wurden inkubiert mit 50 µl 0.1 mg/ml Glu-Plasminogen in PBS, 1 % BSA, 50 µl 0.6 M PO₄³⁻ Puffer, pH 7.4, (●), oder 50 µl 1 M NaHCO₃, pH 8.6 (■), und 50 µl 1000 IU/ml t-PA in PBS, 0.1 % Triton X 100® für 30 min (RT). Dann wurden 25 µl 2.7 mM Val-Leu-Lys-pNA, 1.7 M KCl zugesetzt und ΔA bei 405 nm/h RT wurde bestimmt.

### Abb. 27b,c: Stimulation von t-PA durch Vancomycin-präzipitiertes Plasma

25 µl P-Pool wurde inkubiert mit 25 µl 0-22 mM Vancomycin in PBS und 50 µl 1000 IU/ml t-PA in PBS, 0.1 % Triton X 100® und 25 µl 0 mM (O) or 15 mM Chloramin-T® (●) für 30 min bei RT. Dann wurden 25 µl 2.7 mM Val-Leu-Lys-pNA, 1.7 M KCI zugesetzt und die resultierende Plasmin-Aktivität wurde gemessen durch Bestimmung von ΔA/min RT (Abb. 27b). Verlängerung der finalen Inkubationszeit auf 158 min (RT) im nichtoxidierten Ansatz (Abb. 27c).

### Abb. 28: Vancomycin-Reaktivität mit oxidiertem Plasma

25 µl P-Pool, unsupplementiert (●) oder supplementiert mit 8.6 mM (3.2 g/l) EDTA (O), wurden inkubiert mit 25 µl 0-89 mM Chloramin-T® in PBS für 60 min bei RT. Dann wurde der FIATA (-) oder der FIFTA (- - -) durchgeführt und gegen 100 % der Norm Plasmapool kalibriert.

### Abb. 29: Vancomycin-Reaktivität mit EDTA-supplementiertem Plasma

25 µl P-Pool wurden mit 25 µl PBS (O) oder mit 25 µl 2 mM Chloramin-T® PBS (●) inkubiert. Nach 60 min (RT) wurden 25 µl 0-172 mM EDTA in PBS zugesetzt. Nach 5 min (RT) wurden FIATA (-) und FIFTA (- - -) durchgeführt.

### Abb. 30: Vancomycin-Reaktivität mit t-PA inkubiertem Plasma

25 µl 300 % Fbgen-Pool wurden mit 25 µl PBS (O) oder 15 mM chloramine-T®, PBS (●) für 60 min (RT) inkubiert. Dann wurden 50 µl 1000 IU/ml t-PA, 0.1 % Triton X 100®, PBS zugesetzt. Nach 0-8 h (RT) wurden 25 µl 4.4 mM Vancomycin, PBS zugefügt und ΔA bei 405 nm wurde bestimmt.

## Patentansprüche

1. Verfahren zum Nachweis von Fibrinogen und/oder Fibrinogen-Derivaten, **dadurch gekennzeichnet, dass** es sich bei dem Verfahren um ein turbidimetrisches matrixunabhängiges und/oder gerinnungszeitunabhängiges Verfahren handelt, wobei
a) zu einer auf Fibrinogen und/oder Fibrinogen-Derivate zu untersuchende Lösung Thrombin in gelöster Form hinzugegeben wird, so dass sich finale Konzentrationen von weniger als 2 IU/ml Thrombin einstellen,
b) der Trübungsanstieg des so erhaltenen Ansatzes wird bestimmt.

2. Verfahren nach Anspruch 1, folgende Schritte umfassend:
a) zu einer auf Fibrinogen und/oder Fibrinogen-Derivate zu untersuchende Lösung wird Thrombin und Albumin in gelöster Form hinzugegeben, so dass sich finale Konzentrationen von weniger als 2 IU/ml Thrombin, und 1,7-10 % (w/v) Albumin einstellen,
b) der Trübungsanstieg des so erhaltenen Ansatzes wird bestimmt.

3. Verfahren nach Anspruch 1 oder 2, **dadurch gekennzeichnet, dass** eine Vergleichsmessung mit einem bekannten Standard, insbesondere einem Normalstandard, enthaltend Fibrinogen und/oder Fibrinogen-Derivate, durchgeführt wird.

4. Verfahren nach Anspruch 1 oder 2, **dadurch gekennzeichnet, dass** es sich bei der zu untersuchenden Lösung um Blut oder Blutplasma handelt.

5. Verfahren nach Anspruch 4, **dadurch gekennzeichnet, dass** es sich bei dem Blutplasma um Citrat-Plasma, EDTA-Plasma, Heparin-Plasma und/oder Guanidinoverbindungs-Plasma handelt.

6. Verfahren nach Anspruch 1 oder 2, **dadurch gekennzeichnet, dass** die Thrombin-Konzentration in der Thrombin- oder Thrombin- und Albumin-haltigen Lösung zwischen 50 und 1000 mlU/ml beträgt und/oder die zuzusetzende Thrombinmenge 5 - 100 mlU pro 50 µl Plasma ist.

7. Verfahren nach Anspruch 2, **dadurch gekennzeichnet, dass** die Albumin-Konzentration in der Thrombin- und Albumin-haltigen Lösung zwischen 2 und 20 % (w/v) beträgt.

8. Verfahren nach Anspruch 1 oder 2, **dadurch gekennzeichnet, dass** Thrombin oder Thrombin und Albumin in einer Puffer-haltigen Lösung gelöst sind.

9. Verfahren nach Anspruch 8, **dadurch gekennzeichnet, dass** es sich bei dem Puffer um Phosphat-gepuffertes Natriumchlorid (PBS) handelt.

10. Verfahren nach Anspruch 1 oder 2, **dadurch gekennzeichnet, dass** die Thrombin- oder Thrombin- und Albumin-haltige Lösung zusätzlich Polybren enthält.

11. Verfahren nach Anspruch 10, **dadurch gekennzeichnet, dass** die Polybren-Konzentration bis zu 1000 µg/ml beträgt und/oder die zuzusetzende Polybrenmenge bis zu 100 µg pro 50 µl Plasma ist.

12. Verfahren nach Anspruch 10, **dadurch gekennzeichnet, dass** bei Untersuchung von Heparin-Plasmen die Polybren-Konzentration 1000-30000 µg/ml beträgt und/oder die zuzusetzende Polybren-Menge 100-3000 µg pro 50 µl Plasma ist.

13. Verfahren nach Anspruch 1 oder 2, **dadurch gekennzeichnet, dass** das Verfahren bei einer Temperatur zwischen 0 und 42 °C durchgeführt wird.

14. Verfahren nach Anspruch 1 oder 2, **dadurch gekennzeichnet, dass** die Reaktionszeit zwischen 20 Sekunden und 60 Minuten beträgt.

15. Verfahren nach einem der Ansprüche 1 bis 14, **dadurch gekennzeichnet, dass** der Trübungsanstieg durch Zusatz von Thrombin in einer Probe auf dysfunktionelles Fibrinogen zu untersuchenden Lösung gemessen wird (FIFTA) und dass der Trübungsanstieg durch Zusatz von Vancomycin in einer weiteren Probe der zu untersuchenden Lösung gemessen wird (FIATA) und der Quotient aus beiden Messungen zur Bestimmung der Konzentration an dysfunktionellem Fibrinogen in der Lösung bestimmt wird.

16. Verwendung eines Testsystems zum Nachweis von Fibrinogen und/oder Fibrinogen-Derivatenund/oder von dysfunktionellem Fibrinogen mit einem Verfahren nach einem der Ansprüche 1 bis 15, **dadurch gekennzeichnet, dass** es als Komponente eine Lösung enthaltend Thrombin oder eine Lösung enthaltend Thrombin und Albumin, zur Erzeugung einer finalen Konzentration an Thrombin in der Testlösung von weniger als 2 IU/ml Thrombin, umfasst.

17. Verwendung eines Testsystems nach Anspruch 16, **dadurch gekennzeichnet, dass** es als Komponente eine Lösung enthaltend Polybren umfasst, wobei Thrombin und/oder Albumin und Polybren in derselben Lösung enthalten sein können.

18. Verwendung eines Testsystems nach einem der Ansprüche 16 oder 17 zum Nachweis der pathologischen disseminierten intravaskulären Gerinnung und/oder zur Beurteilung des Patienten-Risikos für atherothrombische Erkrankungen wie myokardiale oder cerebrale Ischämie/Infarkt und/oder zur Diagnose einer Dysfibrinogenämie.

19. Verwendung eines Diagnostikum für Fibrinogen enthaltend bis zu 2 IU/ml Thrombin, zur Diagnose von Fibrinogen mittels eines Verfahrens gemäß einem der Ansprüche 1 bis 14 und/oder zur Diagnose dysfunktionellem Fibrinogen mittels einem Verfahren gemäß Anspruch 15.

20. Verwendung eines Diagnostikums nach Anspruch 19, **dadurch gekennzeichnet, dass** es zusätzlich 2 bis 20 % Albumin enthält.

21. Verwendung eines Diagnostikums nach Anspruch 19 oder 20, **dadurch gekennzeichnet, dass** es zusätzlich Polybren enthält.

22. Verwendung eines Diagnostikums nach einem der Ansprüche 19 bis 21, **dadurch gekennzeichnet, dass** es sich um ein Diagnostikum zum Nachweis der pathologischen disseminierten intravaskulären Gerinnung und/oder um ein Diagnostikum zur Beurteilung des Patienten-Risikos für atherothrombische Erkrankungen wie myokardiale oder cerebrale Ischämie/Infarkt und/oder um ein Diagnostikum zum Nachweis einer Dysfibrinogenämie handelt.

23. Verwendung eines Diagnostikums nach einem der Ansprüche 19 bis 21 zum Nachweis der pathologischen disseminierten intravaskulären Gerinnung und/oder zur Beurteilung des Patienten-Risikos für atherothrombische Erkrankungen wie myokardiale oder cerebrale Ischämie/Infarkt und/oder zur Diagnose einer Dysfibrinogenämie.

## Claims

1. A method to determine fibrinogen and/or derivatives of fibrinogen, with the characteristics, that it is a procedure that works with increase in turbidity, that is independent of matrix and/or independent of a time of coagulation, in which
a) thrombin in dissolved form is added to a solution that shall be analyzed, giving final concentrations of less than 2 IU/ml thrombin,
b) the increase of turbidity of the so obtained mixture is determined.

2. A method according to claim 1, comprising the following steps:
a) thrombin and albumin in dissolved form are added to a solution that shall be analyzed for fibrinogen and/or for derivatives of fibrinogen, giving final concentrations of less than 2 IU/ml thrombin, and more than 1.7-10 % (weight / volume) albumin.
b) the increase of turbidity of this mixture is determined.

3. A method according to claim 1 or 2, whereby a determination of comparison is performed with a known standard, particularly a normal standard, that contains fibrinogen and/or derivatives of fibrinogen.

4. A method according to claim 1 or 2, whereby the solution that is analyzed is blood or plasma of blood.

5. A method according to claim 4, whereby the plasma of blood is plasma with citrate, plasma with EDTA, plasma with heparin and/or plasma with a compound that has a group of guanidine.

6. A method according to claim 1 or 2, whereby the concentration of thrombin in the solution that contains thrombin, or that contains thrombin and albumin is between 50 and 1000 mlU/ml, and/or the amount of thrombin, that is added, is 5-100 mlU per 50 µl of plasma.

7. A method according to claim 2, whereby the concentration of albumin in the solution that contains thrombin and albumin is 2 - 20 % (weight/volume).

8. A method according to claim 1 or 2, whereby thrombin or albumin is dissolved in a solution that contains a buffer.

9. A method according to claim 8, whereby the buffer is a buffer of phosphate with sodium chloride (PBS).

10. A method according to claim 1 or 2, whereby the solution that contains thrombin and albumin also contains Polybrene.

11. A method according to claim 10, whereby the concentration of Polybrene is up to 1000 µg/ml and/or the amount of Polybrene is up to 100 µg per 50 µl of plasma.

12. A method according to claim 10, whereby for the analysis of plasmas with heparin the concentration of Polybrene is 1000-30000 µg/ml and/or the amount of Polybrene that will be added is 100-3000 µg per 50 µl of plasma.

13. A method according to claim 1 or 2, whereby the method is performed at a temperature between 0 and 42°C.

14. A method according to claim 1 or 2, whereby the time of reaction is between 20 seconds and 60 minutes.

15. A method according to one of the claims 1 to 14, with the characteristics, that the increase of turbidity is determined by means of thrombin that is added to a sample, that shall be analyzed for fibrinogen that is dysfunctional(FIFTA), and that the increase of turbidity is determined by means of vancomycin that is added to a further sample (FIATA), and that the values of both determinations are divided to determine the concentration of fibrinogen which is dysfunctional.

16. Usage of a test system for the determination of fibrinogen and/or of derivatives of fibrinogen and/or of fibrinogen that is dysfunctional by means of a procedure according to one of the claims 1 to 15, with the characteristics, that it contains a component that is a solution that contains thrombin or a solution that contains thrombin and albumin, to generate a final concentration of thrombin in the mixture of reaction of the test of less than 2 IU/ml thrombin.

17. Usage of a test system according to claim 16, with the characteristics, that it contains a component that is a solution that contains Polybrene, whereby thrombin and/or albumin and Polybrene can be parts of the same solution.

18. Usage of a test system according to one of the claims 16 or 17 to determine the pathologic disseminated intravascular coagulation and/or for the diagnosis of the risk of patients to develop atherothrombotic diseases, as ischemia or infarction of heart or of brain and/or for the diagnosis of the presence of fibrinogen, that is dysfunctional, in blood.

19. Usage of a diagnostic for fibrinogen, that contains up to 2 IU/ml thrombin, for the diagnosis of fibrinogen by means of a procedure according to one of the claims 1 to 14 and/or for the diagnosis of fibrinogen, that is dysfunctional, by means of a procedure according to claim 15.

20. Usage of a diagnostic according to claim 19, with the characteristics, that it contains also 2 to 20 % albumin.

21. Usage of a diagnostic according to claim 19 or 20, with the characteristics, that is contains also Polybrene.

22. Usage of a diagnostic according to one of the claims19 to 21, with the characteristics, that it is a diagnostic to determine the pathologic disseminated intravascular coagulation and/or a diagnostic to determine the risk of a patient to develop atherothrombotic diseases, such as ischemia / infarction of heart or of brain and/or a diagnostic to determine fibrinogen, that is dysfunctional, in blood.

23. Usage of a diagnostic according to one of the claims 19 to 21 to determine the pathologic disseminated intravascular coagulation and/or to determine the risk of a patient to develop atherothrombotic diseases, such as ischemia /infarction of heart or of brain and/or for the diagnosis of fibrinogen, that is dysfunctional, in blood.

## Revendications

1. Procédé de détection du fibrinogène et/ou de dérivés du fibrinogène, **caractérisé en ce qu'**il s'agit pour le procédé d'un procédé turbidimétrique indépendant de la matrice et/ou indépendant du temps de coagulation, dans lequel
a) d'une part la thrombine est rendue sous forme dissoute sur le fibrinogène et/ou les dérivés du fibrinogène dans la solution à analyser, de sorte que les concentrations finales s'ajustent à moins de 2 UI/ml de thrombine,
b) on détermine la montée du trouble du lot ainsi obtenu.

2. Procédé selon la revendication 1, comprenant les étapes suivantes :
a) d'une part la thrombine et l'albumine sont rendues sous forme dissoute sur le fibrinogène et/ou les dérivés du fibrinogène dans la solution à étudier, de sorte que les concentrations finales s'ajustent à moins de 2 UI/ml de thrombine, et 1,7-10 % (w/v) d'albumine,
b) on détermine la montée du trouble du lot ainsi obtenu.

3. Procédé selon la revendication 1 ou 2, **caractérisé en ce qu'**on réalise une mesure comparative avec un étalon connu, en particulier un étalon normal, contenant du fibrinogène et/ou des dérivés de fibrinogène.

4. Procédé selon la revendication 1 ou 2, **caractérisé en ce qu'**il s'agit pour la solution à étudier de sang ou de plasma sanguin.

5. Procédé selon la revendication 4, **caractérisé en ce qu'**il s'agit pour le plasma sanguin de plasma-citrate, EDTA-plasma, héparine-plasma, et/ou composé guanidine-plasma.

6. Procédé selon la revendication 1 ou 2, **caractérisé en ce que**, la concentration de thrombine dans la solution contenant de la thrombine ou de la thrombine et de l'albumine s'élève à entre 50 et 1000 mUI/ml et/ou la quantité de thrombine à ajouter est de 5-100 mUI pour 50 µl de plasma.

7. Procédé selon la revendication 2, **caractérisé en ce que** la concentration d'albumine dans la solution contenant de la thrombine et de l'albumine s'élève à entre 2 et 20 % (w/v).

8. Procédé selon la revendication 1 ou 2, **caractérisé en ce que** la thrombine ou la thrombine et l'albumine sont dissoutes dans une solution contenant un tampon.

9. Procédé selon la revendication 8, **caractérisé en ce qu'**il s'agit pour le tampon de chlorure de sodium tamponné au phosphate (PBS).

10. Procédé selon la revendication 1 ou 2, **caractérisé en ce que** la solution contenant de la thrombine ou de la thrombine et de l'albumine contient en plus du Polybren.

11. Procédé selon la revendication 10, **caractérisé en ce que** la concentration de Polybren s'élève à jusqu'à 1000 µg/ml et/ou la quantité de Polybren à ajouter est de jusqu'à 100 µg pour 50 µl de plasma.

12. Procédé selon la revendication 10, **caractérisé en ce que** dans l'analyse d'héparine-plasmas la concentration de Polybren s'élève à 1000-30000 µg/ml et/ou la quantité de Polybren à ajouter est de 100-3000 µg pour 50 µl de plasma.

13. Procédé selon la revendication 1 ou 2, **caractérisé en ce que** le procédé est réalisé à une température entre 0 et 42°C.

14. Procédé selon la revendication 1 ou 2, **caractérisé en ce que** le temps de réaction s'élève à entre 20 secondes et 60 minutes.

15. Procédé selon l'une des revendications 1 à 14, **caractérisé en ce que** l'augmentation du trouble est mesurée par ajout de thrombine dans un échantillon de fibrinogène en dysfonctionnement à la solution à analyser (FIFTA) et **en ce que** l'augmentation du trouble est mesurée par ajout de Vancomycine dans un autre échantillon de la solution à analyser (FIATA) et on détermine le quotient des deux mesures pour déterminer la concentration de fibrinogène en dysfonctionnement dans la solution.

16. Utilisation d'un système d'essai pour la détection de fibrinogène et/ou de dérivés de fibrinogène et/ou de fibrinogène en dysfonctionnement avec un procédé selon l'une des revendications 1 à 15, **caractérisée en ce qu'**il comprend comme composant une solution contenant de la thrombine ou une solution contenant de la thrombine et de l'albumine, pour produire une concentration finale de thrombine dans la solution d'essai de moins de 2 UI/ml de thrombine.

17. Utilisation d'un système d'essai selon la revendication 16, **caractérisé en ce qu'**il comprend comme composant une solution contenant du Polybren, la thrombine et/ou l'albumine et le Polybren pouvant être contenu dans la même solution.

18. Utilisation d'un système d'essai selon l'une des revendications 16 ou 17 pour la détection de la coagulation intravasculaire disséminée pathologique et/ou pour l'évaluation du risque du patient pour les maladies athérothrombotiques comme l'ischémie/infarctus myocardique ou cérébral et/ou pour le diagnostic d'une dysfibrinogènémie.

19. Utilisation d'un diagnostic pour le fibrinogène contenant jusqu'à 2 UI/ml de thrombine, pour le diagnostic de fibrinogène au moyen d'un procédé selon l'une des revendications 1 à 14 et/ou pour le diagnostic de fibrinogène en dysfonctionnement au moyen d'un procédé selon la revendication 15.

20. Utilisation d'un diagnostic selon la revendication 19, **caractérisée en ce qu'**il contient en plus 2 à 20 % d'albumine.

21. Utilisation d'un diagnostic selon la revendication 19 ou 20, **caractérisée en ce qu'**il contient en plus du Polybren.

22. Utilisation d'un diagnostic selon l'une des revendications 19 à 21, **caractérisée en ce qu'**il s'agit d'un diagnostic pour la détection de la coagulation intravasculaire disséminée pathologique et/ou d'un diagnostic pour la détermination du risque du patient pour des maladies athérothrombotiques comme l'ischémie/infarctus myocardique ou cérébral et/ou un diagnostic pour la détection d'une dysfibrinogènémie.

23. Utilisation d'un diagnostic selon l'une des revendications 19 à 21 pour la détection de la coagulation intravasculaire disséminée pathologique et/ou pour la détermination du risque du patient pour les maladies athérothrombotiques comme l'ischémie/infarctus myocardique ou cérébral et/ou un diagnostic pour la détection d'une dysfibrinogènémie.
